(19)

Europäisches Patentamt

European Patent Office

Office européen des brevets

(11) **EP 4 357 449 A1**

(12) **EUROPEAN PATENT APPLICATION**
published in accordance with Art. 153(4) EPC

(43) Date of publication:
**24.04.2024 Bulletin 2024/17**

(21) Application number: **22825074.2**

(22) Date of filing: **16.06.2022**

(51) International Patent Classification (IPC):
**C12N 5/0735** (2010.01)     **C12N 5/074** (2010.01)

(52) Cooperative Patent Classification (CPC):
**C12N 5/06**

(86) International application number:
**PCT/JP2022/024226**

(87) International publication number:
**WO 2022/265086 (22.12.2022 Gazette 2022/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.06.2021 US 202163211622 P**

(71) Applicants:
• **Kyoto University**
  **Kyoto-shi, Kyoto 606-8501 (JP)**
• **Sumitomo Pharma Co., Ltd.**
  **Osaka 541-0045 (JP)**

(72) Inventors:
• **TAKAHASHI, Jun**
  **Kyoto-shi, Kyoto 606-8501 (JP)**
• **DOI, Daisuke**
  **Kyoto-shi, Kyoto 606-8501 (JP)**
• **IKEDA, Megumi**
  **Kobe-shi, Hyogo 650-0047 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **METHOD FOR PRODUCING CEREBRAL CORTICAL CELL PREPARATION DERIVED FROM HUMAN PLURIPOTENT STEM CELLS**

(57)    An object of the present invention is to provide a cerebral organoid derived from a human pluripotent stem cell, a cell aggregate including a cerebral cortical cell, and a method for producing any of them, each being useful for regenerative therapy. The method of the present invention for producing a cerebral organoid is a method for producing a cerebral organoid from a pluripotent stem cell in the absence of a sustentacular cell, and comprises: (1) a step of culturing the pluripotent stem cell in a culture solution, wherein the culture solution is substantially free of bFGF and provokes substantially no TGFβ signal; and (2) a step of inducing the cell obtained in step (1) to differentiate into a neural cell.

**EP 4 357 449 A1**

**Description**

**Technical Field**

The present invention relates to a cerebral organoid or cerebral cortical cell aggregate derived from pluripotent stem cells, and a method for producing any of them.

**Background Art**

Cell transplantation therapy, in which neural cells formed by inducing differentiation of human pluripotent stem cells, in particular, of human induced pluripotent stem cells (iPS cells) are transplanted, is expected to be promising for the purpose of ameliorating symptoms of cerebrovascular disorder such as motor paralysis.
A method of inducing differentiation of human embryonic stem cells (ES cells) maintained in the presence of sustentacular cells (occasionally referred to as feeder cells) such as mouse embryonic fibroblasts (MEF) into a cerebral organoid (Serum-free Floating culture of Embryoid Bodies-like aggregates with quick reaggregation: SFEBq method) has been established, and it has become possible to obtain neural cells of cerebral cortical layer V or VI (deep layer neurons) including motor neurons.
On the other hand, cells for transplantation that are to be used in clinical situations are desired to be produced in the absence of xenogeneic cells (feeder-free). If human pluripotent stem cells maintained in the absence of sustentacular cells are induced to differentiate by the SFEBq method, however, low efficiency of generation of cerebral organoids results, which has been considered as a problem (Non Patent Literature 3).

**Citation List**

**Patent Literature**

> Patent Literature 1: WO2015/076388
> Patent Literature 2: WO2016/167372
> Patent Literature 3: WO2016/063985

**Non Patent Literature**

> Non Patent Literature 1: Kitahara, et al., Stem Cell Reports 2020 Vol. 15, 467-481
> Non Patent Literature 2: Kuwahara, et al., Scientific Reports 2019, 9:18936
> Non Patent Literature 3: Eiraku, M et al., Cell Stem Cell, 3, 519-532 (2008)

**Summary of Invention**

**Technical Problem**

An object of the present invention is to provide a cerebral organoid derived from human pluripotent stem cells, a cell aggregate including cerebral cortical cells, and a method for producing any of them, each being useful for regenerative therapy. More specifically, an object of the present invention is to provide a method for producing a cerebral organoid from pluripotent stem cells in the absence of sustentacular cells, and a product obtained by the method.

**Solution to Problem**

In an attempt to solve the above problem, the present inventors have found that cerebral organoids can be efficiently produced by culturing pluripotent stem cells in a culture solution in the absence of sustentacular cells, wherein the culture solution is substantially free of bFGF and provokes substantially no TGFβ signal, and then inducing differentiation into neural cells, and further found that cerebral cortical cell aggregates having quality suitable for transplantation can be efficiently produced by culturing cerebral organoids in a culture solution containing a Notch signaling inhibitor.
Specifically, the present invention provides the followings.

[1] A method for producing a cerebral organoid from a pluripotent stem cell in the absence of a sustentacular cell, comprising:

(1) a step of culturing the pluripotent stem cell in a culture solution, wherein the culture solution is substantially free of bFGF and provokes substantially no TGFβ signal; and
(2) a step of inducing the cell obtained in step (1) to differentiate into a neural cell.

[2] The method according to [1], wherein step (2) comprises:

(2a) a step of subjecting the cell obtained in step (1) to suspension culture in a culture solution containing a TGFβ signaling inhibitor and a Wnt signaling inhibitor to obtain a cell aggregate; and
(2b) a step of subjecting the cell aggregate obtained in step (2a) to suspension culture in a culture solution substantially free of a TGFβ signaling inhibitor and a Wnt signaling inhibitor to obtain a cerebral organoid.

[3] The method according to [2], wherein the suspension culture in step (2a) is static culture.
[4] The method according to [2] or [3], wherein the suspension culture in step (2b) is shaking culture.
[5] The method according to any of [1] to [4], wherein culture period in step (1) is less than 3 days.
[6] The method according to any of [1] to [5], wherein culture period in step (1) is 12 hours or more and 2 days or less.
[7] The method according to any of [1] to [6], wherein the culture solution in step (1) contains a TGFβ signaling inhibitor selected from the group consisting of SB431542, A-83-01, and XAV-939.
[8] The method according to any of [2] to [7], wherein the culture solutions in step (1), step (2a), and step (2b) are each a serum-free culture solution.
[9] The method according to any of [1] to [8], wherein the pluripotent stem cell is a human induced pluripotent stem cell or a human embryonic stem cell.
[10] The method according to any of [1] to [9], further comprising:
(3) a step of screening a cerebral organoid from a plurality of cell aggregates obtained in step (2) on the basis of, as indices, one or more selected from the group consisting of shape, internal structure, size, surface coloring or patterning, and gene expression of a cell aggregate.
[11] A cell culture produced by using the method according to any of [1] to [9], wherein

the cell culture comprises a plurality of spherical cell aggregates, and
a proportion of cerebral organoids in the plurality of spherical cell aggregates is 40% or more.

[12] The cell culture according to [11], wherein a proportion of a cerebral cortex-like structural body occupying each of the cerebral organoids is 40% or more.
[13] The cell culture according to [12], wherein each of the cerebral organoids is a cell aggregate further having one or more characteristics selected from the following (1) to (5):

(1) being a spherical cell aggregate;
(2) having a cerebral cortex-like structural body inside of the cell aggregate;
(3) having no pigmentation in a surface;
(4) having none of cystoid shape, protruding shape, and balloon-like shape in a part of the cell aggregate; and
(5) expressing at least one marker selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

[14] A cerebral cortical cell aggregate, wherein

(a) number of cells positive for a proliferation marker is 10% or less of total number of cells,
(b) number of cells positive for one or more markers selected from the group consisting of a neuronal marker, a cortical layer V/VI marker, and a forebrain marker is 70% or more of total number of cells, and (c) the cerebral cortical cell aggregate includes substantially no neuroepithelium or cerebral cortex-like structure.

[15] The cerebral cortical cell aggregate according to [14], wherein the proliferation marker in (a) is Ki67, and the neuronal marker, the cortical layer V/VI marker, and the forebrain marker in (b) are βIII-tubulin, Ctip2, and FOXG1, respectively.

[16] The cerebral cortical cell aggregate according to [14] or [15], further expressing at least one marker selected from the group consisting of:

(d) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

[17] The cerebral cortical cell aggregate according to [16], expressing SLC17A7.

[18] The cerebral cortical cell aggregate according to any of [15] to [17], substantially unexpressing one or more genes selected from the group consisting of GAD2, COL1A1, TYR, TTR, and HOXA2.

[19] A method for producing a cerebral cortical cell aggregate from a pluripotent stem cell in the absence of a sustentacular cell, comprising:

(i) a step of obtaining a cerebral organoid from the pluripotent stem cell; and
(ii) a step of culturing the cerebral organoid obtained in step (i) in a culture solution containing a Notch signaling inhibitor to obtain a cerebral cortical cell aggregate.

[20] The method according to [19], wherein, in step (i), a cerebral organoid is obtained from a pluripotent stem cell by the method according to any of [1] to [10].

[21] A high-purity cerebral cortical cell aggregate, wherein

(A) number of cells positive for a proliferation marker is 5% or less of total number of cells,
(B) number of cells positive for one or more markers selected from a neuronal marker, a cortical layer V/VI marker, and a forebrain marker is 70% or more of total number of cells, and
(C) the high-purity cerebral cortical cell aggregate includes substantially no neuroepithelium or cerebral cortex-like structure.

[22] The high-purity cerebral cortical cell aggregate according to [21], wherein the proliferation marker in (A) is Ki67, and
the neuronal marker, the cortical layer V/VI marker, and the forebrain marker in (B) are βIII-tubulin, Ctip2, and FOXG1, respectively.

[23] The high-purity cerebral cortical cell aggregate according to [21] or [22], expressing at least one gene selected from the group consisting of:

(D) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

[24] The high-purity cerebral cortical cell aggregate according to [23], expressing one or more genes selected from the group consisting of SLC17A7, NEUROD6, and EMX1.

[25] The high-purity cerebral cortical cell aggregate according to any of [21] to [24], substantially unexpressing one or more genes selected from the group consisting of GAD2, COL1A1, TYR, TTR, and HOXA2.

[26] A method for producing a high-purity cerebral cortical cell aggregate from a pluripotent stem cell in the absence of a sustentacular cell, comprising:

(i) a step of obtaining a cerebral organoid from the pluripotent stem cell;
(ii) a step of culturing the cerebral organoid obtained in step (i) in a culture solution;
(iii) a step of dispersing the cell culture obtained in step (ii) into single cells or two- to five-membered cell clumps; and
(iv) a step of culturing the cell culture obtained in step (ii) or the cell population obtained in step (iii) in a culture solution containing one or more neurotrophic factors, ascorbic acid, and a cAMP activator to obtain a cell aggregate, wherein
the culture solution in step (ii) and/or the culture solution in step (iv) contain or contains a Notch signaling inhibitor.

[27] The method according to [26], wherein, in step (i), a cerebral organoid is obtained from the pluripotent stem cell by the method according to any of [1] to [10].

[28] The method according to any of [19], [20], [26], and [27], wherein the cerebral organoid to be subjected to step (ii) is a cerebral organoid 28 to 44 days after initiation of induction of differentiation into a neural cell.

[29] The method according to any of [19], [20], and [26] to [28], wherein culture period in step (ii) is 2 to 6 days.

[30] The method according to any of [19], [20], and [26] to [29], wherein culture period in step (iv) is 2 to 14 days.

[31] The method according to any of [19], [20], and [26] to [30], wherein the Notch signaling inhibitor is a γ-secretase inhibitor.

[32] The method according to [31], wherein the γ-secretase inhibitor is N-[N-(3,5-difluorophenacetyl)-L-alanyl]-S-phenylglycine t-butyl ester (DAPT) or Compound E.

[33] A cell population comprising the high-purity cerebral cortical cell aggregate according to any of [12] to [25], wherein size, shape, or constituent cell composition of the high-purity cerebral cortical cell aggregate is homogeneous.

[34] A pharmaceutical composition comprising the cerebral cortical cell aggregate according to any of [14] to [18], the high-purity cerebral cortical cell aggregate according to any of [21] to [25], or the cell population according to [33], or a cell population obtained by dispersing any of them into a constituent cell, as an active ingredient.

[35] A tissue for transplantation, the tissue comprising:

the cerebral cortical cell aggregate according to any of [14] to [18], the high-purity cerebral cortical cell aggregate according to any of [21] to [25], or the cell population according to [33], or a cell population obtained by dispersing any of them into a constituent cell.

[36] A therapeutic drug for cerebrovascular disorder, the therapeutic drug comprising the cerebral cortical cell aggregate according to any of [14] to [18], the high-purity cerebral cortical cell aggregate according to any of [21] to [25], or the cell population according to [33], or a cell population obtained by dispersing any of them into a constituent cell, as an active ingredient.

[37] A therapeutic method for cerebrovascular disorder, comprising administering or transplanting the cerebral cortical cell aggregate according to any of [14] to [18], the high-purity cerebral cortical cell aggregate according to any of [21] to [25], or the cell population according to [33], or a cell population obtained by dispersing any of them into a constituent cell to a cerebral cortex or basal ganglion of a subject in need thereof.

[38] A quality assessment method for a cerebral organoid or a cerebral cortical cell aggregate, comprising:

(aa) a step of measuring an expression level of at least one gene selected from the group consisting of GAD2, COL1A1, TYR, TTR, and HOXA2, or a protein encoded by the gene or a fragment thereof in a cerebral organoid or a cerebral cortical cell aggregate; and

(bb) a step of determining with reference to a measurement result in step (aa) that an amount of non-target cells included in the cerebral organoid or the cerebral cortical cell aggregate is equal to or less than a reference value if the expression level of the gene is equal to or less than a reference value.

[39] A quality assessment method for a cerebral organoid or a cerebral cortical cell aggregate, comprising:

(AA) a step of measuring an expression level of at least one gene selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3 in a cerebral organoid or a cerebral cortical cell aggregate; and

(BB) a step of determining with reference to a measurement result in step (AA) that an amount of target cells included in the cerebral organoid or the cerebral cortical cell aggregate is equal to or more than a reference value if the expression level of the gene is equal to or more than a reference value.

**Advantageous Effects of Invention**

The present invention enables production of cerebral organoids applicable as a material of cerebral cortical cell preparations from human pluripotent stem cells with high efficiency. The cerebral cortical cell aggregate and the like of the present invention that are derived from a cerebral organoid are useful as a therapeutic drug or a material for transplantation to treat cerebrovascular disorder or the like.

**Brief Description of Drawings**

[Figure 1] Figure 1 shows images of the morphologies of cell aggregates after induction of differentiation into neural cells in Preliminary Test 1.

[Figure 2] Figure 2 shows results of expression analysis for FGF2 and TGFβ pathway-related genes with microarrays in Preliminary Test 2.

[Figure 3] Figure 3 shows an exemplary differentiation induction scheme in Example 1.

[Figure 4] Figure 4 shows (A) bright field images and (B) confocal fluorescence microscopy images of cell aggregates after induction of differentiation in 1-1 of Example 1.

[Figure 5] Figure 5 shows an exemplary differentiation induction scheme in Example 1.

[Figure 6] Figure 6 shows confocal fluorescence microscopy images of cerebral cortical cell aggregates after induction of differentiation in 1-1 of Example 1.

[Figure 7] Figure 7 shows confocal fluorescence microscopy images of cerebral organoids on Day 35 after induction of differentiation in 1-2 of Example 1.

[Figure 8] Figure 8 shows representative bright field images of cultures on Day 18, Day 27, and Day 34 after induction of differentiation in Example 2.

[Figure 9] Figure 9 shows efficiencies (%) of cerebral organoid formation under different conditions after induction of differentiation in Example 2.

[Figure 10] Figure 10 shows results of expression analysis for different marker genes after step (1) (Day 0) in Example 3.

[Figure 11] Figure 11 shows (A) a differentiation induction scheme in 4-1 of Example 4, and (B) bright field images of cerebral organoids (DAPT-, Day 36), cerebral cortical cell aggregates (DAPT+, Day 36), and a high-purity cerebral cortical cell aggregate (Day 40).

[Figure 12] Figure 12 shows representative confocal fluorescence microscopy images of immunostaining in 4-1 of Example 4.

[Figure 13] Figure 13 shows a differentiation induction scheme in 4-2 of Example 4.

[Figure 14] Figure 14 shows results of analysis of marker gene expression by flow cytometry in 4-2 of Example 4.

[Figure 15] Figure 15 shows results of analysis of marker gene expression by flow cytometry in 4-2 of Example 4.

[Figure 16] Figure 16 shows (A) a scheme of a DAPT method in 4-3 of Example 4, and (B) results of analysis of gene expression variations by RT-qPCR for cell aggregates obtained.

[Figure 17] Figure 17 shows (A) a differentiation induction scheme in Example 5, and (B) results of immunostaining of cerebral organoids on Day 28 (4 wk), Day 42 (6 wk), and Day 75 (10 wk).

[Figure 18] Figure 18 shows results of analysis of relative expression levels of different markers in cerebral organoids on Day 28 (4 wk), Day 35 (5 wk), Day 42 (6 wk), and Day 75 (10 wk) in Example 5.

[Figure 19] Figure 19 shows representative confocal fluorescence microscopy images of immunostained transplants in Example 6.

[Figure 20] Figure 20 shows results of analysis of volumes of immunostained transplants in Example 6.

[Figure 21] Figure 21 shows a scheme of a method of a preferred embodiment.

[Figure 22] Figure 22 shows representative confocal fluorescence microscopy images of immunostaining in Example 7.

[Figure 23] Figure 23 shows results of analysis by flow cytometry in Example 7.

[Figure 24] Figure 24 shows schemes of two methods in 8-1 of Example 8.

[Figure 25] Figure 25 shows results of RT-qPCR analysis of gene expression levels of different markers over time in cell aggregates obtained by a single-cell DAPT method in 8-1 of Example 8.

[Figure 26] Figure 26 shows results of RT-qPCR analysis of gene expression levels of different markers over time in cell aggregates obtained by a single-cell DAPT method and those obtained by an organoid method in 8-1 of Example 8.

[Figure 27] Figure 27 shows results of flow cytometry analysis of gene expression levels of different markers on Day 10 in cell aggregates obtained by a single-cell DAPT method and those obtained by an organoid method in 8-1 of Example 8.

[Figure 28] Figure 28 shows a scheme of a single-cell DAPT method in 8-2 of Example 8.

[Figure 29] Figure 29 shows results of analysis of gene expression by flow cytometry for cell aggregates obtained by a single-cell DAPT method in 8-2 of Example 8.

[Figure 30] Figure 30 shows representative bright field images of the morphologies of organoids in 9-1 of Example 9.

[Figure 31] Figure 31 shows representative bright field images of seven morphological groups in 9-1 of Example 9.

[Figure 32] Figure 32 shows bar graphs showing proportions of organoids of different morphologies in 9-1 of Example 9.

[Figure 33] Figure 33 shows bright field images of nine organoids obtained through three operations of induction of differentiation in 9-2 of Example 9.

[Figure 34] Figure 34 shows results of UMAP representation of data of single-cell RNA-seq analysis for nine organoids in 9-2 of Example 9.

[Figure 35] Figure 35 shows results of UMAP representation of data of single-cell RNA-seq analysis for nine organoids in 9-2 of Example 9 as represented by UMAP.

[Figure 36] Figure 36 shows results of identification of the cell types of different clusters in 9-2 of Example 9 on the basis of gene expression in the different clusters.

[Figure 37] Figure 37 shows expression profiles of characteristic genes in different clusters and known marker genes from single-cell gene expression data in 9-2 of Example 9 as represented by dot plots.

[Figure 38] Figure 38 shows expression profiles of characteristic genes in different clusters and known marker genes from single-cell gene expression data in 9-2 of Example 9 as represented by dot plots.

[Figure 39] Figure 39 shows (A) proportions of different cell types and (B) proportions of neural crest cells at different differentiation stages in nine organoids in 9-2 of Example 9.

[Figure 40] Figure 40 shows results of immunostaining of representative marker proteins for organoids in different groups in 9-3 of Example 9.

[Figure 41] Figure 41 shows bright field images showing three organoids per group in 9-4 of Example 9.

[Figure 42] Figure 42 shows results of analysis of expression of marker genes by an RT-qPCR method for different organoids in 9-4 in Example 9.

[Figure 43] Figure 43 shows results of analysis of expression of marker genes by an RT-qPCR method for different organoids in 9-4 in Example 9.

[Figure 44] Figure 44 shows (A) bright field images of Rosettes organoids in Lot 1, Lot 2, and Lot 3 and (B) UMAP plots of single-cell gene expression analysis in 9-5 of Example 9.

[Figure 45] Figure 45 shows results of UMAP representation of analysis of expression of marker genes for organoids in 9-5 of Example 9.

## Description of Embodiments

### 1. Definitions

[Stem cells]

Herein, the term "stem cell" refers to an undifferentiated cell having differentiation potential and proliferative capacity (in particular, replication competence) retaining differentiation potential. Stem cells include subpopulations with different differentiation abilities, such as pluripotent stem cells, multipotent stem cells, and unipotent stem cells.

A pluripotent stem cell is a stem cell that can be cultured in vitro and has an ability to differentiate into all cell lineages belonging to triploblastic (ectodermal, mesodermal, endodermal) and/or extraembryonic tissues (pluripotency in terms of differentiation). The term multipotent stem cell refers to a stem cell having an ability to differentiate into multiple types, but not all types, of tissues or cells. The term unipotent stem cell refers to a stem cell having an ability to differentiate into a specific tissue or cell.

Pluripotent stem cells can be induced from fertilized ova, cloned embryos, germline stem cells, stem cells in tissue, somatic cells, and so on. Examples of pluripotent stem cells include embryonic stem cells (ES cells), EG cells (embryonic germ cells), and induced pluripotent stem cells (iPS cells). Muse cells (multi-lineage differentiating stress enduring cells), which are obtained from mesenchymal stem cells (MSC), and mGS cells prepared from germ cells (e.g., testis) are also included in pluripotent stem cells.

Human embryonic stem cells, which were established in 1998, are increasingly used even for regenerative medicine. Embryonic stem cells can be produced by culturing an inner cell mass in the blastocyst stage, specifically, within 14 days after fertilization on sustentacular cells or in a culture medium containing FGF2. Methods for producing embryonic stem cells are described, for example, in WO96/22362, WO02/101057, US5,843,780, US6,200,806, and US6,280,718. Embryonic stem cells are available from specific institutions, and commercially available products thereof can be purchased. For example, KhES-1, KhES-2, and KhES-3, which are human embryonic stem cells, are available from Institute for Frontier Life and Medical Sciences, Kyoto University.

Herein, an "induced pluripotent stem cell" is a cell obtained by inducing pluripotency for a somatic cell through reprogramming, for example, with a known method.

Induced pluripotent stem cells were established with mouse cells by Yamanaka et al. in 2006 (Cell, 2006, 126(4), pp. 663-676). Induced pluripotent stem cells were established also with human fibroblasts in 2007, and have pluripotency and replication competence like embryonic stem cells (Cell, 2007, 131(5), pp. 861-872; Science, 2007, 318(5858), pp. 1917-1920; Nat. Biotechnol., 2008, 26(1), pp. 101-106).

Specific examples of induced pluripotent stem cells include cells obtained by inducing pluripotency for differentiated somatic cells such as fibroblasts and peripheral blood mononuclear cells through reprogramming by forced expression of any combination of a plurality of genes selected from a group of reprogramming genes including OCT3/4, SOX2, KLF4, MYC (c-MYC, N-MYC, L-MYC), GLIS1, NANOG, SALL4, LIN28, and ESRRB. Examples of preferred combinations of reprogramming factors include (1) OCT3/4, SOX2, KLF4, and MYC (c-MYC or L-MYC) and (2) OCT3/4, SOX2, KLF4, LIN28, and L-MYC (Stem Cells, 2013; 31:458-466).

In addition to the method of producing induced pluripotent stem cells through induction by reprogramming by gene expression, induced pluripotent stem cells can be induced, for example, by addition of a compound to somatic cells (Science, 2013, 341, pp. 651-654; Nature, 2022, 605, pp. 325-331).

In addition, established induced pluripotent stem cells can be obtained, and, for example, human induced pluripotent cell lines established by Kyoto University, such as 201B7 cells, 201B7-Ff cells, 253G1 cells, 253G4 cells, 1201C1 cells, 1205D1 cells, 1210B2 cells, and 1231A3 cells, are available from Kyoto University and iPS Academia Japan, Inc. As established iPS cells for clinical applications, for example, Ff-101, Ff-I14, QHJI01, and QHJI14 established by Kyoto University are available from Kyoto University. Moreover, iPS cells can be produced by reprogramming somatic cells such as hematopoietic progenitor cells derived from peripheral blood or cord blood and fibroblasts with use of a reprogramming factor. S2WCB 1 and S2WCB3 used herein have been established from adult peripheral blood mononuclear cells by using CytoTune (TM)-2.0 (ID Pharma Co., Ltd.).

Herein, pluripotent stem cells are preferably embryonic stem cells or induced pluripotent stem cells, and more preferably induced pluripotent stem cells.

Herein, pluripotent stem cells are mammalian pluripotent stem cells, preferably rodent (e.g., mouse, rat) or primate (e.g., human, simian) pluripotent stem cells, more preferably human pluripotent stem cells, and even more preferably human induced pluripotent stem cells (iPS cells) or human embryonic stem cells (ES cells).

Pluripotent stem cells such as human iPS cells can be subjected to maintenance culture and expansion culture with methods well known to those skilled in the art.

[Marker]

Herein, the term "marker" refers to a substance that is present in a cell and allows identification or determination of the type or character or the like of the cell on the basis of the presence or abundance of the substance. Specific examples of markers include mRNA, proteins encoded by such mRNA, and sugar chains, and fragments of them.

[Neural cell]

Herein, a neural cell is a neural unit composed of a cell body, a dendrite, and an axon, and is also called a neuron. Neural cells have a function to transmit stimuli from another neural cell or a stimulus receptor cell to still another neural cell or a muscle or glandular cell, and classified by the difference in neurotransmitter that neural cells produce into dopaminergic neurons, serotonergic neurons, GABAergic neurons, and glutamatergic neurons; however, limitation is not set on the type of neurotransmitter herein. Neural cells can be identified with a marker that is significantly expressed, and examples of the marker include βIII-tubulin and MAP2.

[Neural stem cell]

Herein, the term "neural stem cell" refers to a stem cell destined to differentiate into a nervous system cell, but having a capacity to differentiate into any of a plurality of types of nervous system cells and retaining proliferation potential, being a cell having differentiation potential into a neural progenitor cell and differentiation potential into a cerebral cortical cell in combination. Neural stem cells can be identified, for example, with markers for primitive neuroectoderms and neural stem cells such as intermediate filament proteins (e.g., nestin, vimentin) and the transcription factors SOX1, SOX2 and PAX6. Herein, neural stem cells include radial glia.

A cell that is generated from a neural stem cell, capable of differentiating into any of a plurality of types of neural cells, and immature cells is occasionally referred to as a neural progenitor cell.

[Cerebral organoid]

Herein, the term "cell culture" is a term referring to any product obtained through culture of cells, wherein the product is not limited to one having a specific composition. A cell culture can be a clump of a plurality of cell aggregates, and may further include single cells and two-to five-membered cell clumps described later. A cell culture may contain a culture medium or a suspending medium, or not, and the term refers to a cell culture containing neither a culture medium nor a suspending medium, unless otherwise stated.

Herein, the term "cerebral organoid" refers to a spherical cell aggregate including one or more, preferably a plurality of cerebral cortex-like structural bodies each including a neural cell layer external to a neuroepithelium. Here, a cerebral cortex-like structural body is a rosette-like structural body including a neural cell layer external to a neuroepithelium. Accordingly, a cerebral cortex-like structural body can include a neuroepithelium-like structure, and the cerebral organoid of the present application may include a neuroepithelium-like structure.

Here, a "neuroepithelium" is a layered structural body including neural stem cells and/or neural progenitor cells as primary constituent cells, and can also be regarded as a region in which neural stem cells and/or neural progenitor cells are localized.

Here, a "neural cell layer" is a layered structural body including neural cells. The neural cells are not limited as long as they are neural cells that can be generated at a differentiation stage of a cerebral organoid, and examples thereof include cells positive for at least one of a neuronal marker, a forebrain marker, and a cerebral cortical nerve cell marker. The neural cell layer preferably includes two or more or all of cells positive for a neuronal marker, cells positive for a forebrain marker, and cells positive for a marker for cerebral cortical nerve cells or progenitor cells thereof. A neural cell layer can also be regarded as a region in which neural cells generated from a neuroepithelium (cerebral cortical nerve cells) are localized.

In one embodiment, the cerebral organoid herein includes a cerebral organoid obtained by inducing differentiation of pluripotent stem cells.

Here, the term "spherical" means not being bar-like (rod-like) or sheet-like (plate-like), and preferably refers to a "three-dimensional structural body similar to a sphere". Examples thereof include a three-dimensional structural body that presents a circle, an ellipse, or the like when being projected onto a two-dimensional surface. However, the three-dimensional structural body does not need to present a smooth curve, and even if unevenness is found in some parts, the case meets the requirement of "spherical" as long as the cell aggregate can be recognized to have a shape similar to a sphere as a whole. High sphericity is not necessarily needed for "spherical" herein, and an example is a structural body having a sphericity of 0.7 or more or 0.8 or more, or preferably of 0.9 or more.

Examples of marker genes for neural stem cells or neural progenitor cells herein include SOX1, SOX2, and PAX6.

Examples of neuronal marker genes include βIII-tubulin and MAP2.

Examples of marker genes for the forebrain include FOXG1 (also referred to as BF1), SIX3, and EMX1.

Examples of cerebral cortical nerve cell marker genes include layer I to layer VI markers described later.

As described later, examples of marker genes for various cells are genes shown in Table 6 below.

[Cerebral cortical cell]

Herein, a "cerebral cortical cell" is also referred to as a cerebral cortical neuron or a cerebral cortical nerve cell, and the term refers to a neural cell constituting the cerebral cortex.

In the cerebral organoid, the neural cell layer may be a monolayer, or even include a plurality of separate cell layers. For example, layers in which, from the side near the neuroepithelium to the outer side of the organoid, neural cells characteristic to respective layers of the cerebral cortex layer VI (Tbr1, Tbr2), layer V (Ctip2, Er81, Fezf2), layer IV (Rorb), layer III/II (Foxp1, Mef2c, Satb2), and layer I (Reelin) are localized may be included. In each pair of parentheses, marker genes that are frequently used for identifying the corresponding layer are shown.

Herein, cerebral cortical cells are preferably cells positive for the forebrain marker FOXG1. In the present invention, examples of FOXG1 include a polynucleotide specified by NCBI Accession No. NM_005249, and a protein encoded by it.

Herein, cerebral cortical cells may include neural cells or upper motor neurons from the motor area of the cerebral cortex, in other words, neural cells from the anterior part of the cerebral cortex, more specifically, may include neural cells from the layer V and/or layer VI of the motor area of the cerebral cortex.

Herein, neural cells from the layer V or layer VI (also referred to as the layer V/VI, collectively) are a cell population characterized by being positive for Ctip2. In the present invention, examples of Ctip2 include a polynucleotide specified by NCBI Accession No. NM_001282237, NM_001282238, NM_022898, or NM_138576, and a protein encoded by it. Cerebral cortical cells may include neural cells from the layer I, layer II/III, or layer IV

Cerebral cortical cells herein may be produced as a cell population including other cell types, and a cell population including cerebral cortical cells may include cerebral cortical cells, for example, in a proportion of 15% or more, 20% or more, 30% or more, 40% or more, or 50% or more in the cell population produced.

Herein, the term "cerebral cortical cells" is meant to include "cerebral cortical progenitor cells", and cerebral cortical progenitor cells are included in target cells for the "cerebral organoid" in the present invention. On the other hand, almost no or only a minimal number of cerebral cortical progenitor cells can be included in target cells for the "cerebral cortical cell aggregate (including the high-purity cerebral cortical cell aggregate)" of the present invention, which is in a more advanced differentiation stage.

[Cell aggregate and cell population]

Herein, each "cell aggregate " is not limited as long as the cell aggregate is one in which a plurality of cells is adhering to each other to form a three-dimensional structure, and is, for example, a mass formed in such a manner that cells that have been dispersed in a medium such as a culture medium assemble together, or a mass of cells formed through cell division. Cell aggregates forming a particular tissue are also included in the definition. Embryoid bodies, spheres, and spheroids are also included in the definition of a cell aggregate. Each cell aggregate may have any shape, and examples include spherical cell aggregates and layered cell aggregates.

Herein, each "cell population" is a population including a plurality of cells, and may be a cell aggregate (a spherical cell aggregate, a layered cell aggregates) or a two- to five-membered cell clumps. Each "cell population" may also be a population of a plurality of cell aggregates, a population of a plurality of dispersed single cells, or a population of a plurality of two- to five-membered cell clumps, or a population of any combination of them.

Herein, the statement that a cell aggregate has high purity means that the content of target cells included in the cell aggregate is high. The specific content depends on cell type; if the content of target cells is 70% or more, preferably 80% or more or 90% or more to the total number of cells in a cell aggregate, for example, the cell aggregate can be said to have high purity.

Herein, if the statement that a cell aggregate has high purity is made, it can be said that the content of non-target cells is 30% or less, preferably 20% or less or 10% or less to the total number of cells in the cell aggregate. More preferably, it can be said that the content of proliferative cells in the cell aggregate is 10% or less or 5% or less, preferably 3% or less, more preferably 2% or less to the total number of cells in the cell aggregate.

Herein, the term "regathered cell aggregate" refers to a cell aggregate formed in such a manner that a cell aggregate is dispersed into single cells or two- to five-membered cell clumps and the dispersed single cells or cell clumps then reaggregate (e.g., a regathered cerebral cortical cell aggregate). Regathered cerebral cortical cell aggregates at least include high-purity cerebral cortical cell aggregates.

[Culture solution]

Herein, each "culture solution (also referred to as a culture medium)" is not limited and may be any culture solution (culture medium) commonly used for animal cell culture, as long as the culture solution can maintain the lives of animal cells, but is preferably a culture solution that provides an environment that allows target cells to proliferate. Each culture solution (culture medium) may be prepared in-house, and commercially available culture media may be purchased for use.

Examples of minimal essential media include culture media that can be used for culture of animal cells such as BME culture medium, BGJb culture medium, CMRL 1066 culture medium, Glasgow MEM (GMEM) culture medium, Improved MEM Zinc Option culture medium, IMDM culture medium, Medium 199 culture medium, Eagle MEM culture medium, aMEM culture medium, DMEM culture medium, F-12 culture medium, DMEM/F-12 culture medium, IMDM/F12 culture medium, Ham's culture medium, RPMI 1640 culture medium, Fischer's culture medium, and mixed culture media of them. Carbon sources such as carbohydrates including glucose and amino acids, vitamins, inorganic salts, and so on are contained in those minimal essential media.

Any component commonly used for culture of animal cells may be appropriately added to minimal essential media unless induction of differentiation of interest is adversely affected.

It is preferable from the viewpoint of using for producing a cell aggregate suitable for transplantation that each culture medium to be used in the present invention be a serum-free culture solution.

The term "serum-free culture solution" in the present invention refers to a culture medium substantially free of raw or unpurified serum. Herein, even a culture medium contaminated with a purified component derived from blood or a component derived from animal tissue (e.g., a growth factor) is included in the definition of a serum-free culture solution, as long as the culture medium contains no raw or unpurified serum. The serum-free culture solution may contain, as appropriate, a fatty acid or lipid, an amino acid (e.g., a non-essential amino acid), a vitamin, a growth factor, a cytokine, an antioxidant, 2-mercaptoethanol, pyruvic acid, a buffer, an inorganic salt, and so on.

Each culture medium to be used in the present invention is preferably a xeno-free culture medium. Here, the term "xeno-free" refers to conditions in which components derived from a biological species differing from the biological species of cells to be cultured (xenogeneic components, also referred to as xenogeneic factors) are excluded. Some of the serum-free culture solutions may be xeno-free culture media.

[Serum substitute]

A serum substitute may be contained in each culture medium to be used in the present invention. Examples of the serum substitute include a serum substitute appropriately containing albumin, transferrin, a fatty acid, a collagen precursor, a trace element, 2-mercaptoethanol, or 3'-thiol glycerol, or an equivalent of any of them. Such a serum substitute can be prepared, for example, with a method described in WO98/30679. Commercially available products of serum substitutes may be used. Examples of such commercially available serum substitutes include Knockout Serum Replacement (manufactured by Thermo Fisher Scientific Inc.; hereinafter, occasionally written as KSR), "StemSure (R) Serum Replacement (SSR)" chemically-defined lipid concentrate (manufactured by Thermo Fisher Scientific Inc.), B27 supplement (manufactured by Thermo Fisher Scientific Inc.), N2 supplement (manufactured by Thermo Fisher Scientific Inc.), and ITS supplement (manufactured by Thermo Fisher Scientific Inc.), and preferred examples thereof include N2 supplement or B27 supplement.

[Sustentacular cell]

Herein, sustentacular cells, which are also referred to as feeder cells, are cells that are allowed to coexist in culturing stem cells such as pluripotent stem cells and are different from the stem cells. Examples of sustentacular cells include mouse fibroblasts (e.g., MEF), human fibroblasts, SNL cells, and STO cells. The sustentacular cells may be sustentacular cells subjected to growth inhibition treatment. Examples of the growth inhibition treatment include treatment with a growth inhibitor (e.g., mitomycin C) and treatment with gamma-ray irradiation or UV irradiation.

Herein, the phrase "in the absence of sustentacular cells (also referred to as feeder-free)" means culturing in the absence of sustentacular cells. Examples of the situation in the absence of sustentacular cells include conditions without addition of any sustentacular cell and conditions substantially free of any sustentacular cell (e.g., the proportion of the number of sustentacular cells to the total number of cells is 3% or less, preferably 1% or less).

[Suspension culture]

In the present invention, the term "suspension culture" refers to allowing cells to survive in a state of being suspended in a culture medium, or culturing to allow cells to form an aggregate (also called a sphere) without the cells adhering to a culture vessel. Herein, cells in a state of single cells or an assembled mass of a plurality of cells (a cell aggregate or a cell population) are subjected to suspension culture.

Examples of culture vessels to be used for suspension culture include, but are not limited to, flasks, tissue culture flasks, dishes, Petri dishes, tissue culture dishes, multidishes, microplates, microwell plates, micropores, multiplates, multiwell plates, chamber slides, Schale, tubes, trays, culture bags, bioreactors, and roller bottles.

In order to enable culture under nonadhesive conditions, it is preferable for culture vesselincubators to be nonadhesive to cells. As an incubator nonadhesive to cells, a culture vessel such that the surface of the incubator has not been artificially treated for the purpose of enhancing the adhesion to cells (e.g., coating treatment with an extracellular matrix or the like), or a culture vessel subjected to coating treatment through treatment to artificially prevent adhesion (e.g., polyhydroxyethyl methacrylate (poly-HEMA), a nonionic surface-active polyol (such as Pluronic F-127), or a phospholipid analog construct (e.g., water-soluble polymer with constituent units of 2-methacryloyloxyethylphosphorylcholine (Lipidure (R))) can be used. Examples of culture vessels to be used in suspension culture, in particular, in the SFEBq method include a PrimeSurface (R) (a low-protein-adhesion 96-well plate manufactured by Sumitomo Bakelite Co., Ltd.).

Herein, suspension culture may be static culture, or shaking culture, rotating culture, or stirring culture.

Herein, static culture is a culture method to culture with conscious avoidance of moving cell aggregates. Specifically, in some cases, local temperature variation in a culture medium causes the convection of the culture medium and the resulting flow moves a cell aggregate; however, such a case is also regarded as static culture in the present invention because the cell aggregate is not consciously allowed to move.

For shaking culture, rotating culture, or stirring culture, instruments well known to those skilled in the art can be appropriately used.

<bFGF>

Heren, bFGF, referring to basic fibroblast growth factor, is a protein also referred to as FGF2.

<TGFβ Signaling Inhibitor>

Herein, TGFβ signaling inhibitors are each a substance that inhibits a series of signaling from binding of TGFβ to a receptor to SMAD, and examples thereof include substances that inhibit binding to the ALK family as the receptor and substances that inhibit phosphorylation of SMAD caused by the ALK family.

Herein, each TGFβ signaling inhibitor is not limited as long as the TGFβ signaling inhibitor is one capable of suppressing signaling mediated by TGFβ, and may be any of nucleic acid, protein, and a low-molecular-weight organic compound.

Examples of the TGFβ signaling inhibitors include substances that directly act on TGFβ (e.g., proteins, antibodies, aptamers), substances that suppress expression of a gene encoding TGFβ (e.g., antisense oligonucleotides, siRNA), substances that inhibit binding between the TGFβ receptor and TGFβ, and substances that inhibit physiological activities due to signaling caused by the TGFβ receptor (e.g., inhibitors for the TGFβ receptor). Examples of the TGFβ signaling inhibitors further include substances that inhibit binding to the ALK family as the receptor and substances that inhibit phosphorylation of SMAD caused by the ALK family. Specific examples of the ALK family include ALK4, ALK5, and ALK7.

Examples of the TGFβ signaling inhibitors include Lefty-1 (mouse: NM_010094, human: NM_020997 in NCBI Accession Nos.), Lefty-2 (mouse: NM_177099, human: NM_003240 and NM_001172425 in NCBI Accession Nos.), SB431542 (4-[4-(1,3-benzodioxol-5-yl)-5-(2-pyridinyl)-1H-imidazol-2-yl]-benzamide), SB202190 (these are from R. K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01 (WO 2009146408), Galunisertib (LY2157299), LY3200882, SB525334, GW788388, RepSox, and derivatives of them. The TGFβ signaling inhibitor to be used in the present invention is preferably SB431542 or A-83-01.

<Wnt Signaling Inhibitor>

Herein, Wnt signaling inhibitors are each a substance that suppresses production of Wnt (e.g., Wnt3) or a substance that inhibits a series of signaling from binding of Wnt to a receptor to accumulation of β-catenin, and examples thereof include substances that inhibit binding to the Frizzled family as the receptor and substances that promote the decomposition of β-catenin.

Examples of the Wnt signaling inhibitors include, but are not limited to, substances that inhibit PORCN (for humans, e.g., proteins specified by NP_001269096, NP_073736, NP_982299, NP_982300, and NP_982301 in NCBI Accession Nos.), which is involved in processing of Wnt protein, DKK1 protein (e.g., for humans, NM_012242 in NCBI Accession No.), sclerostin (e.g., for humans, NM_025237 in NCBI Accession No.), Cerberus protein, Wnt receptor inhibitors, soluble Wnt receptors, anti-Wnt antibodies, casein kinase inhibitors, and dominant-negative Wnt protein, and one or more of the substances may be used in combination.

Specific examples of the Wnt signaling inhibitors include IWR-1-endo ((4-[(3aR,4S,7R,7aS)-1,3,3a,4,7,7a-hexahydro-1,3-dioxo-4,7-methano-2H-isoindol-2-yl]-N-8-quinolinyl-benzamide), Merck Millipore), IWP-2 (Sigma-Aldrich Co. LLC), IWP-3 (Sigma-Aldrich Co. LLC), IWP-4 (Sigma-Aldrich Co. LLC), IWP-L6 (EMD Millipore), C59 (or Wnt-C59) (Cellagen technology), ICG-001 (Cellagen Technology), LGK-974 (or NVP-LGK-974) (Cellagen Technology), FH535 (Sigma-Aldrich Co. LLC), WIKI4 (Sigma-Aldrich Co. LLC), KYO2111 (Minami I. et al., Cell Rep. 2:1448-1460, 2012), PNU-74654 (Sigma-Aldrich Co. LLC), XAV939 (Stemgent), and derivatives of them. Among them, for example, IWR-1-endo, C59, and LGK-974 are preferable.

<Notch Signaling Inhibitor>

Each Notch signaling inhibitor is not limited as long as the Notch signaling inhibitor is a substance capable of suppressing signaling caused by Notch. Examples of Notch signaling inhibitors include γ-secretase inhibitors and Notch transcription complex inhibitors such as MAML-1 inhibitors.

Each γ-secretase inhibitor is not limited as long as the γ-secretase inhibitor is a substance capable of inhibiting the enzymatic activity of y-secretase. Specific examples thereof include DAPT (N-[N-(3,5-difluorophenacetyl)-l-alanyl]-S-phenylglycine t-butyl ester), DBZ (dibenzazepine), MDL28170 (calpain inhibitor III), Compound E (N-[(1S)-2-[[(3S)-2,3-dihydro-1-methyl-2-oxo-5-phenyl-1H-1,4-benzodiazepin-3-yl]amino]-1-methyl-2-oxoethyl]-3,5-difluorobenzeneacetamide), Compound 34 ((2S,3R)-3-(3,4-difluorophenyl)-2-(4-fluorophenyl)-4-hydroxy-N-((3 S)-2-oxo-5-phenyl-2,3-1H-benzo[e] [1,4]diazepin-3-yl)butyramide), γ-secretase inhibitor XI, and γ-secretase inhibitor III. Examples of the Notch transcription complex inhibitors include CB-103 (6-[4-(1,1-dimethylethyl)phenoxy]-3-pyridinamine) and IMR-1 (2-methoxy-4-(4-oxo-2-thioxo-thiazolidin-5-ylidenemethyl)-phenoxy)-acetic acid ethyl ester).

<ROCK Inhibitor>

Each ROCK inhibitor is not limited as long as the ROCK inhibitor is a substance that is an inhibitor to Rho-associated coiled-coil kinase (ROCK) and suppresses the functions of ROCK. Examples of ROCK inhibitors include Y-27632 ((+)-(R)-trans-4-(1-aminoethyl)-N-(4-pyridyl)cyclohexanecarboxamide dihydrochloride), H-1152 ((S)-4-methyl-5-((2-methyl-1,4-diazepan-1-yl)sulfonyl)isoquinoline dihydrochloride), fasudil (HA-1077; 1-(5-isoquinolinesulfonyl)homopiperazine hydrochloride), Wf-536 (4-[(1R)-1-aminoethyl]-N-(pyridin-4-yl)benzamide), thiazovivin (N-benzyl-2-(pyrimidin-4-ylamino)thiazole-4-carboxamide), ripasudil (4-fluoro-5-[[(2S)-hexahydro-2-methyl-1H-1,4-diazepin-1-yl]sulfonyl]isoquinoline), GSK429286 (4-[4-(trifluoromethyl)phenyl]-N-(6-fluoro-1H-indazol-5-yl)-2-methyl-6-oxo-1,4,5,6-tetrahydro-3-pyridinecarboxamide) 6, RKI-1447 (N- [(3 -hydroxyphenyl)methyl] -N'- [4-(4-pyridinyl)-2-thiazolyl]urea), Azaindole 1 (6-chloro-N4-[3,5-difluoro-4-[(3-methyl-1H-pyrrolo[2,3-b]pyridin-4-yl)oxy]phenyl]pyrimidine-2,4-diamine), HA-1100 (1-[(1,2-dihydro-1-oxo-5-isoquinolinyl)sulfonyl]hexahydro-1H-1,4-diazepine), and Y-39983 (4-[(1R)-1-aminoethyl]-N-1H-pyrrolo[2,3-b]pyridin-4-ylbenzamide). A preferable example of the ROCK inhibitors is Y-27632.

<Neurotrophic Factor>

Herein, the term neurotrophic factor is a collective term for secretory proteins having activities to promote the survival of neural cells, elongation of neurites and axons, synaptogenesis, and so on. Examples of neurotrophic factors include Nerve Growth Factor (NGF), Brain-derived Neurotrophic Factor (BDNF), Neurotrophin 3 (NT-3), Neurotrophin 4/5 (NT-4/5), Neurotrophin 6 (NT-6), Glia cell line-derived Neurotrophic Factor (GDNF), and Ciliary Neurotrophic Factor (CNTF). Neurotrophic factors preferable in the present invention are factors selected from the group consisting of GDNF and BDNF. Neurotrophic factors are commercially available from FUJIFILM Wako Pure Chemical Corporation and R&D Systems, Inc. and can be used with ease, and, alternatively, may be obtained by forced expression in cells with a method known to those skilled in the art.

<cAMP Activator>

Herein, examples of cAMP activators include cAMP, dibutyryl-cAMP, and forskolin.

2. Method for Producing Cerebral Organoid

In one embodiment, the method of the present invention for producing a cerebral organoid includes a method for producing a cerebral organoid from a pluripotent stem cell in the absence of a sustentacular cell, comprising step (1) and step (2) below. The situation "in the absence of sustentacular cells" is as described above, and step (1) and step (2) are each performed "in the absence of sustentacular cells".

(1) A step of culturing the pluripotent stem cell in a culture solution, wherein the culture solution is substantially free of bFGF and provokes substantially no TGFβ signal
(2) A step of inducing the cell obtained in step (1) to differentiate into neural cells

<Step (1)>

Herein, culture "in the presence of substance X" is culturing "in a culture medium containing substance X", and the substance X may be one contained in the culture medium as an original component, or one exogenously added. Accordingly, endogenous substance X that can be expressed, secreted, or produced by cells or a tissue during the culture is distinguished from exogenous substance X, and a culture medium containing no exogenous substance X is understood not to fall within the category of "culture medium containing substance X" even if the culture medium contains endogenous substance X.

For example, a "culture medium containing a TGFβ signaling inhibitor" is a culture medium supplemented with a TGFβ signaling inhibitor, or a culture medium containing a TGFβ signaling inhibitor as an original component.

The culture solution that is "substantially free of bFGF" is a culture solution that originally contains no bFGF or has not been exogenously supplemented with bFGF, wherein the presence of bFGF that cells themselves express is not prohibited.

The presence of remaining bFGF below the detection limit is not prohibited for some kinds of cells and operations of culture medium exchange to be used, and such cases are also included in the scope of the present application. In addition, the present application includes culturing with a culture medium containing a low dose of bFGF to such a degree that the dose not affect the efficiency of neural differentiation in the present invention.

The culture solution that "provokes substantially no TGFβ signal" is a culture solution that originally contains no TGFβ or a culture solution that has not been exogenously supplemented with TGFβ, wherein the presence of TGFβ that cells themselves express is not prohibited. A culture solution obtained by adding an effective amount of a TGFβ signaling inhibitor to a culture solution containing exogenously added TGFβ or to a culture solution containing no exogenously added TGFβ also falls within the category of "culture solution that provokes substantially no TGFβ signal".

In using a TGFβ signaling inhibitor to provoke substantially no TGFβ signal, any of the above TGFβ signaling inhibitors can be appropriately selected. The concentration of the TGFβ signaling inhibitor can be appropriately adjusted according to the intensity of TGF(3 signal activity in the culture solution. In other words, the concentration of the TGFβ signaling inhibitor in the culture solution is not limited as long as the concentration is one that inhibits the activity of an ALK4, ALK5, or ALK7 signal.

The TGFβ signaling inhibitor to be added to the culture solution in step (1) may be any of the above TGFβ signaling inhibitors, and is preferably selected from the group consisting of SB431542 and A-83-01. In using a culture medium that is commercially available for the purpose of culturing pluripotent stem cells such as ES cells and iPS cells with the pluripotency maintained and contains no bFGF as the culture solution in step (1), for example, the concentration of the TGFβ signaling inhibitor is a concentration corresponding to any of 100 nM to 1 mM, 100 nM to 500 μM, 100 nM

to 100 μM, 100 nM to 50 μM, 100 nM to 40 μM, 100 nM to 30 μM, 100 nM to 25 μM, 100 nM to 20 μM, 100 nM to 15 μM, 100 nM to 10 μM, 500 nM to 30 μM, 500 nM to 10 μM, 100 nM to 7 μM, 1 μM to 20 μM, 1 μM to 10 μM, 500 nM to 7 μM, 1 μM to 7 μM, 100 nM to 3 μM, 100 nM to 2 μM, 100 nM to 1 μM, 100 nM to 750 nM, and so on in the case that SB431542 is used as the TGFβ signaling inhibitor, but the concentration is not limited thereto. A preferable example is a concentration corresponding to 1 μM to 10 μM. For other TGFβ signaling inhibitors, a concentration that causes the ALK inhibitory activity or TGFβ inhibitory activity corresponding to SB431542 in the aforementioned concentration can be appropriately set. Here, the concentration corresponding to the concentration in using SB431542 is a concentration that elicits an inhibitory effect on the TGFβ signaling pathway (e.g., an effect to inhibit the activity of an ALK4, ALK5, or ALK7 signal) comparable to that caused by the SB431542 concentration. Those skilled in the art could set the concentration with ease.

As the culture solution to be used in step (1), for example, a commercially available culture medium that is a culture medium for culturing pluripotent stem cells and has been made bFGF-free can be used with addition of a TGFβ signaling inhibitor.

As the culture solution that is substantially free of bFGF and provokes substantially no TGFβ signal, for example, a culture solution commercially available as a culture medium obtained by removing bFGF and TGFβ (e.g., Essential 6) from a culture medium that allows maintenance and proliferation of pluripotent stem cells (e.g., Essential 8) can also be used.

The culture solution to be used in step (1) may contain an additional substance to such a degree that the additional substance causes substantially no influence on the formation of a cerebral organoid, which is obtained through step (1) and step (2), but, preferably, it is desirable that a substance that enhances or inhibits signaling having influence on induction of differentiation of pluripotent stem cells, such as a BMP signal and a Sonic Hedgehog signal, be not externally added. The culture solution to be used in step (1) may contain a Wnt signaling inhibitor, but it is desirable that a substance that enhances Wnt signaling be not externally added.

The pluripotent stem cells to be subjected to step (1) are preferably human induced pluripotent stem cells (iPS cells) or human embryonic stem cells (ES cells), and more preferably human induced pluripotent stem cells.

In step (1), the pluripotent stem cells are cultured in the absence of sustentacular cells. Being in the absence of sustentacular cells is also called being feeder-free, and is such a state that no sustentacular cell is present in the culture medium. Specific examples of culture conditions in the absence of sustentacular cells mentioned here include culture conditions without addition of sustentacular cells such as fibroblasts, SNL cells, and STO cells.

The culture solution to be used in step (1) is preferably a serum-free culture solution substantially free of serum, and a serum-free culture solution supplemented with a serum substitute may be used, as necessary. Examples of the serum substitute include those mentioned above, and, preferably, KSR, for example, 1 to 30% KSR can be preferably used.

The culture solution to be used in step (1) is not limited as long as the culture solution is a culture solution, preferably a serum-free culture solution that is substantially free of bFGF and provokes substantially no TGFβ signal, but, for other substances than bFGF and substances that provoke TGFβ signals, it is desirable that the culture solution be a culture medium containing components necessary for culturing pluripotent stem cells with the pluripotency maintained.

Culture media for culturing pluripotent stem cells with the pluripotency maintained, namely, culture media for pluripotent stem cells are widely commercially available, and examples thereof include Essential 8 (manufactured by Thermo Fisher Scientific Inc.), S-medium (manufactured by DS Pharma Biomedical Co., Ltd.), StemPro (manufactured by Thermo Fisher Scientific Inc.), hESF9 (Proc Natl Acad Sci USA. 2008 Sep 9; 105(36): 13409-14), mTeSR1 (manufactured by STEMCELL Technologies), mTeSR2 (manufactured by STEMCELL Technologies), TeSR-E8 (manufactured by STEMCELL Technologies), Cellartis DEF-CS 500 Xeno-Free Culture Medium (manufactured by Takara Bio Inc.), and StemFit (manufactured by Ajinomoto Healthy Supply Co., Inc.).

The pluripotent stem cells to be subjected to step (1) may be cryopreserved pluripotent stem cells immediately after being thawed, and can be preferably cultured and/or passaged in advance with a culture medium suitable for expansion culture of pluripotent stem cells with the pluripotency maintained. The passage number of the pluripotent stem cells to be subjected to step (1) is not limited, and it is desirable that passage be performed twice to eight times.

The culture period in step (1) is less than 5 days, less than 4 days, preferably less than 3 days, more preferably 12 hours or more and 48 hours or less, 18 hours or more and 48 hours or less, even more preferably 24 hours or more and 48 hours or less, 24 hours or more and 36 hours or less, or 18 hours or more and 36 hours or less, and most preferably about 1 day. In the culture period, a period of subculture of pluripotent stem cells as a preparation phase is not included.

The culture in step (1) may be performed under any of suspension culture conditions and adhesion culture conditions, and is preferably performed by adhesion culture.

The incubator to be used in performing adhesion culture is not limited as long as the incubator allows "adhesion culture", and cell-adhesive incubators are preferred. Examples the cell-adhesive incubators include an incubator the surface of which has been artificially treated for the purpose of enhancing the adhesion to cells, specifically, an incubator the inside of which has been coated with a coating agent as described above. Examples of the coating agent include extracellular matrices such as laminin [including laminin $\alpha5\beta1\gamma1$ (hereinafter, laminin 511), laminin $\alpha1\beta1\gamma1$ (hereinafter, laminin 111), laminin $\alpha1\beta1\gamma2$ (laminin 112), laminin $\alpha2\beta1\gamma1$ (laminin 211), laminin $\alpha2\beta1\gamma2$ (laminin 212), laminin $\alpha2\beta2\gamma1$ (laminin 221), laminin $\alpha2\beta2\gamma2$ (laminin 222), and laminin $\alpha5\beta1\gamma2$ (laminin 512), and laminin fragments (such as laminin 511E8)], entactin, collagen, gelatin, vitronectin, Synthemax (Corning Incorporated), and Matrigel, and polymers such as polylysine and polyornithine. In addition, a culture vessel subjected to surface processing such as positive charge treatment can be used. A preferable example is laminin, and more preferable example is laminin 511E8. Commercially available products of laminin 511E8 can be purchased (e.g., iMatrix-511, Nippi, Incorporated).

Culture conditions including culture temperature and $CO_2$ concentration in step (1) can be appropriately set. The culture temperature is, for example, approximately 30°C to approximately 40°C, and preferably approximately 37°C. The $CO_2$ concentration is, for example, approximately 1% to approximately 10%, and preferably approximately 5%.

Step (1) is a maintenance culture step of culturing pluripotent stem cells with the pluripotency maintained in the absence of sustentacular cells, and is a neural-differentiation preparation step that is performed before step (2) (differentiation induction step). This neural-differentiation preparation step is a step of preparing during a period before induction of neural differentiation, or before introduction of neural differentiation, and the pluripotent stem cells cultured in step (1) exhibit suppressed expression of TGFβ-related genes, and this results in high efficiency of cerebral organoid formation when the pluripotent stem cells are induced to differentiate into neural cells.

<Step (2)>

Cells obtained in step (1) can be induced to differentiate into neural cells by a method well known to those skilled in the art, and thereby a cerebral organoid can be obtained.

A method for induction of differentiation that allows pluripotent stem cells to differentiate into a cell population constituting a cerebral organoid can be appropriately selected. Such methods are well known, and, for example, methods described in WO2015/076388, WO2016/167372, Sakaguchi et al., Stem Cell Reports 2019 Vol 13 458-473, Kitahara et al., Stem Cell Reports 2020 Vol. 15, 467-481 (Non Patent Literature 1), and Kadoshima et al., 2013, PNAS, vol. 110, No. 50, 20284-20289 can be used.

Specifically, examples of the method for induction of differentiation to be performed in step (2) include the following step (2a) and step (2b):

(2a) a step of subjecting the cells obtained in step (1) to suspension culture, preferably to static culture, in a culture solution containing a TGFβ signaling inhibitor and a Wnt signaling inhibitor to obtain a cell aggregate; and
(2b) a step of subjecting the cell aggregate obtained in step (2a) to suspension culture in a culture solution substantially free of a TGFβ signaling inhibitor or a Wnt signaling inhibitor, preferably in a culture solution containing neither a TGFβ signaling inhibitor nor a Wnt signaling inhibitor, to obtain a cerebral organoid.

<Step (2a)>

The cells obtained in step (1) are dispersed in a culture solution, preferably in a serum-free culture solution substantially free of serum (raw or unpurified serum), and cultured under nonadhesive conditions (i.e., suspension culture), and a plurality of cells is allowed to assemble to form a cell aggregate. As the culture solution to be used in aggregation, a serum-free culture solution containing a serum substitute may be used.

Examples of the incubator to be used for that cell aggregate formation include, but are not limited to, flasks, tissue culture flasks, dishes, Petri dishes, tissue culture dishes, multidishes, microplates, microwell plates, micropores, multiplates, multiwell plates, chamber slides, Schale, tubes, trays, culture bags, bioreactors, and roller bottles. Another example is a method of cell aggregate formation by embedding with gel such as alginate hydrogel. In order to enable culture under nonadhesive conditions, it is preferable that the incubator be nonadhesive to cells. As an incubator nonadhesive to cells, for example, an incubator the surface of which has been artificially treated to make the surface nonadhesive to cells, or an incubator the surface of which has not been artificially treated for the purpose of enhancing the adhesion to cells (e.g., coating treatment with an extracellular matrix or the like) can be used.

In forming a cell aggregate, the cells obtained in step (1) are first collected from subculture, and they are dispersed into single cells or a state close to such condition. This dispersion is performed with a proper cell dissociation solution. As the cell dissociation solution, for example, a chelating agent such as EDTA, a protease such as trypsin, collagenase IV, and metalloprotease can be used singly or in an appropriate combination. Especially, cell dissociation solutions with less cytotoxicity are preferable, and commercially available products such as Dispase (EIDIA Co., Ltd.), TrypLE (manufactured by Gibco), and Accutase (Millipore Corporation) are available as such cell dissociation solutions. Dispersed cells are suspended in a culture medium obtained by adding Y-27632 to the above culture medium (serum-free culture solution, that is, minimal essential medium to be used in step (2a) described later).

Here, adding an inhibitor for Rho-associated coiled-coil kinase (ROCK inhibitor) from the initiation of culture is preferable in order to prevent the cell death of pluripotent stem cells (in particular, human pluripotent stem cells) that is induced by the dispersion (WO2008/035110, Watanabe, K. et al., Nature Biotechnology, 2007, vol. 25, No. 6, page 681-686).

Examples of the ROCK inhibitor include those shown above, and a preferable example thereof is Y-27632.

The ROCK inhibitor is added, for example, for 20 days or less, for 15 days or less, preferably for 10 days or less, more preferably for 6 days or less from the initiation of culture. The concentration of the ROCK inhibitor may be constant or gradually decreased. In one embodiment, culture is performed in the presence of the ROCK inhibitor for 10 days to 20 days, preferably for 15 days to 20 days, more preferably for approximately 17 days to 19 days, and the concentration of the ROCK inhibitor can be gradually decreased during the culture. In one embodiment, culture is performed in the presence of the ROCK inhibitor for 10 to 25 days, preferably for 12 to 25 days or 10 to 20 days, more preferably for approximately 15 to 20 days or approximately 17 days to 19 days, and the concentration of the ROCK inhibitor may be gradually decreased during the culture.

The concentration of the ROCK inhibitor to be used for suspension culture is such a concentration that the cell death of pluripotent stem cells that is induced by the dispersion can be prevented. Examples of the concentration include a concentration corresponding to approximately 0.1 to 200 $\mu$M, preferably to approximately 2 to 100 $\mu$M, more preferably to approximately 30 to 100 $\mu$M in the case that Y-27632 is used as the ROCK inhibitor.

As described above, the concentration of the ROCK inhibitor may be varied during the period for addition, and, for example, the concentration can be halved during the latter half of the period, and the concentration can be gradually reduced from the time point of initiation of step (2a).

The suspension in which the cells obtained in step (1) have been dispersed is seeded in the above incubator, and culture is performed under nonadhesive conditions, thereby allowing a plurality of cells to assemble to form a cell aggregate.

In one embodiment, it is preferable to allow the dispersed cells to quickly aggregate to form one cell aggregate in each culture compartment (SFEBq method). Examples of methods for allowing dispersed cells to quickly aggregate include the following methods:

1) a method in which dispersed cells are confined in a culture compartment of relatively small volume (e.g., 1 ml or less, 500 $\mu$l or less, 200 $\mu$l or less, 100 $\mu$l or less) to form one cell aggregate in the culture compartment; and
2) a method in which dispersed cells are put in a centrifuge tube and the resultant is centrifuged to allow the cells to precipitate at one place to form one cell aggregate in the tube.

In 1), after the dispersed cells are confined, the culture compartment is preferably left to stand. Examples of the culture compartment include, but are not limited to, a well in a multiwell plate (such as 384-well, 192-well, 96-well, 48-well, and 24-well plates), micropores, and a chamber slide, a tube, and a droplet of culture medium in a hanging drop method. The dispersed cells confined in the compartment precipitate at one place by the action of gravity, or the cells adhere to each other, and as a result one cell aggregate is formed per culture compartment. In order to facilitate the precipitation of the dispersed cells at one place, it is preferable that the bottom shape of a multiwell plate, a micropore, a chamber slide, a tube, or the like be U-shape or V-shape.

The number of cells to be seeded in one culture compartment is not limited as long as one cell aggregate is formed per culture compartment and differentiation into forebrain cells can be induced in the cell aggregate by the method of the present invention, and the cells obtained in step (1) are seeded typically at approximately $1 \times 10^3$ to approximately $5 \times 10^4$ cells, preferably at approximately $1 \times 10^3$ to approximately $2 \times 10^4$ cells, more preferably at approximately $2 \times 10^3$ to approximately $1.2 \times 10^4$ cells per culture compartment. Then, by allowing the cells to quickly aggregate, one cell aggregate typically of approximately $1 \times 10^3$ to approximately $5 \times 10^4$ cells, preferably of approximately $1 \times 10^3$ to approximately $2 \times 10^4$ cells, more preferably of approximately $2 \times 10^3$ to approximately $1.2 \times 10^4$ cells is formed per culture compartment.

Alternatively, the cells obtained in step (1) are seeded typically at approximately $1 \times 10^3$ to approximately $5 \times 10^5$ cells, preferably at approximately $1 \times 10^3$ to approximately $2 \times 10^5$ cells, more preferably at approximately $2 \times 10^3$ to approximately $1 \times 10^5$ cells per culture compartment. Then, by allowing the cells to quickly aggregate, one cell aggregate typically of $5 \times 10^2$ to approximately $5 \times 10^5$ cells, preferably of approximately $5 \times 10^2$ to approximately $2 \times 10^5$ cells, more preferably of approximately $1 \times 10^3$ to approximately $1 \times 10^5$ cells is formed per culture compartment.

The time until cell aggregate formation can be appropriately determined within such a range that one cell aggregate is formed per compartment and differentiation into a cerebral organoid can be induced in the cell aggregate, and a cell aggregate is formed preferably within 24 hours, more preferably within 12 hours. Alternatively, the time until cell aggregate formation is such that a cell aggregate is formed preferably within 48 hours, more preferably within 24 hours.

Other culture conditions including culture temperature and $CO_2$ concentration in cell aggregate formation can be appropriately set. The culture temperature is not limited, and, for example, approximately 30 to 40°C, and preferably approximately 37°C. The $CO_2$ concentration is, for example, approximately 1 to 10%, and preferably approximately 5%.

Furthermore, a population of cell aggregates uniform in quality can be obtained by preparing a plurality of culture compartments under the same culture conditions and forming one cell aggregate in each culture compartment. Evaluation on whether cell aggregates are uniform in quality can be performed for the cell aggregates, for example, on the basis of size and number of cells, macroscopic morphology, microscopic morphology and uniformity thereof found in tissue staining analysis, expression of differentiation and undifferentiation markers and uniformity thereof, and regulation of expression of differentiation markers and synchronism thereof for cell aggregates, and reproducibility of differentiation efficiency among cell aggregates. Here, the statement that a population of cell aggregates is "uniform" means that 80% or more of the cell aggregates in the whole population of cell aggregates each have a parameter of interest within a range of the mean of the parameter for the population of cell aggregates ± 20%, preferably of the mean ± 10%, more preferably of the mean ± 5%.

A TGFβ signaling inhibitor and a Wnt signaling inhibitor are contained in the culture solution to be used in step (2a). As the TGFβ signaling inhibitor, those described above are available, and preferable examples thereof include SB431542, A-83-01, and XAV-939. As the Wnt signaling inhibitor, those described above are available, and preferable examples thereof include IWR-1-end, C59, LGK-974, and DKK-1 (protein).

A preferable combination of a Wnt signaling inhibitor and a TGFβ signaling inhibitor is IWR-1-endo and SB431542.

The concentration of the Wnt signaling inhibitor in the culture medium can be appropriately set within such a range that a cell aggregate can be induced to differentiate into forebrain cells, and examples thereof include a concentration that causes the Wnt signaling inhibitory activity corresponding to 0.1 to 50 μM, preferably to 0.3 to 10 μM, more preferably to 0.3 to 5 μM in the case that IWR-1-endo is used as the Wnt signaling inhibitor.

The concentration of the TGFβ signaling inhibitor in the culture medium can be appropriately set within such a range that a cell aggregate can be induced to differentiate into forebrain cells, and examples thereof include a concentration that causes the TGFβ signaling inhibitory activity corresponding to 0.1 to 100 μM, preferably to 1 to 50 μM, more preferably to 1 to 10 μM in the case that SB431542 is used as the TGFβ signaling inhibitor.

The culture solution to be used in step (2a), specifically, the culture medium to be used in formation of a cell aggregate and suspension culture of a cell aggregate is not limited as long as the culture medium is a culture medium that can be used for culture of animal cells, and any of the culture media to be used for culture of animal cells according to the definition described above can be prepared from minimal essential medium.

Examples of the minimal essential medium to be used in step (2a) include Glasgow MEM culture medium, DMEM culture medium, F-12 culture medium (also referred to as F12 culture medium), and DMEM/F12 culture medium. Glasgow MEM culture medium is preferably used.

The culture medium to be used in forming a cell aggregate may contain a serum substitute. As the serum substitute, those mentioned above can be used, and examples thereof include KSR (knockout serum replacement) (manufactured by Invitrogen), a chemically-defined lipid concentrate (manufactured by Gibco), and Glutamax (manufactured by Gibco).

Specifically, a culture medium containing approximately 10 to 30% serum substitute (e.g., KSR) can be used.

Alternatively, the culture medium to be used in forming a cell aggregate may contain a serum substitute. As the serum substitute, those mentioned above can be used, and examples thereof include KSR (knockout serum replacement) (manufactured by Invitrogen), a chemically-defined lipid concentrate (manufactured by Gibco), and Glutamax (manufactured by Gibco). Specifically, a culture medium containing a serum substitute in an appropriate amount according to the instruction manual of the corresponding product (e.g., 1 to 30% KSR) can be used.

The culture medium to be used for suspension culture of a cell aggregate can contain an additional additive unless the additional additive adversely affects induction of differentiation into forebrain cells. Examples of the additive include, but are not limited to, insulin, iron sources (e.g., transferrin), minerals (e.g., sodium selenate), saccharides (e.g., glucose), organic acids (e.g., pyruvic acid, lactic acid), serum proteins (e.g., albumin), amino acids (e.g., L-glutamine), reducing agents (e.g., 2-mercaptoethanol), vitamins (e.g., ascorbic acid, d-biotin), antibiotics (e.g., streptomycin, penicillin, gentamicin), and buffers (e.g., HEPES).

In one embodiment, it is preferable from the viewpoint of avoiding adversely affecting induction of differentiation into forebrain cells that the culture medium to be used for suspension culture of a cell aggregate be free of pattern-forming factors such as Fgf, Wnt, Nodal, Notch, and Shh and growth factors such as insulin and lipid-rich albumin.

Other culture conditions including culture temperature, $CO_2$ concentration, and $O_2$ concentration in suspension culture of an cell aggregate can be appropriately set. The culture temperature is, for example, approximately 30 to 40°C, and preferably approximately 37°C. The $CO_2$ concentration is, for example, approximately 1 to 10%, and preferably approximately 5%. The $O_2$ concentration is, for example, approximately 20%.

The suspension culture in step (2a) may be static culture, or shaking culture or rotating culture or stirring culture, and is preferably static culture. Alternatively, static culture may be performed only for a part of the period of step (2a).

Step (2a) is performed for a sufficient period such that the direction of differentiation into a forebrain region can be ensured and a cell aggregate positive for a forebrain marker (e.g., a Foxg1-positive cell aggregate) can be induced. Thus, a cell aggregate including cells positive for a forebrain marker can be obtained through step (2a).

In one embodiment, step (2a) is performed until a stage in which at least one cell positive for a forebrain marker is generated. Preferably, step (2a) is performed until 50% or more, preferably 70% or more of cell aggregates under culture become positive for a forebrain marker.

In one embodiment, step (2a) is performed until a stage in which a cell aggregate is generated such that 30% or more, preferably 50% or more, more preferably 70% or more of cells included in the cell aggregate are positive for a forebrain marker.

Although the culture period in step (2a) can vary depending on the types of the Wnt signaling inhibitor and TGFβ signaling inhibitor and culture conditions and hence cannot be definitely specified, the culture period, for example, in the case that human pluripotent stem cells are used is 7 days to 30 days, and preferably 15 days to 20 days (e.g., 18 days).

<Step (2b)>

In step (2b), a cerebral organoid is obtained by subjecting the cell aggregate obtained in step (2a) to suspension culture in a culture solution substantially free of a TGFβ signaling inhibitor and a Wnt signaling inhibitor.

In one embodiment, the suspension culture in step (2b) may be performed under high-oxygen-partial-pressure conditions. The high-oxygen-partial-pressure conditions are conditions with an oxygen partial pressure exceeding the atmospheric oxygen partial pressure (20%). In one embodiment, the oxygen partial pressure in step (2b) is, for example, 30 to 60%, preferably 35 to 60%, and more preferably 38 to 60%.

As with the case of the culture medium to be used in step (2a), the culture medium to be used in step (2b) is not limited as long as the culture medium is a culture medium to be used for culture of animal cells, and the culture medium according to the definition described above can be prepared as a minimal essential medium.

Examples of the minimal essential media include Glasgow MEM culture medium, DMEM culture medium, F-12 culture medium, and DMEM/F12 culture medium. DMEM/F12 culture medium is preferably used.

The culture medium to be used in step (2b) is preferably a serum-free culture medium, and may contain a serum substitute, as necessary. As the serum substitute, those mentioned above can be used, and examples thereof include N2 supplement, B27 supplement, Neurocult SM1 Neuronal supplement, and KSR.

The concentration of the serum substitute can be appropriately adjusted; specifically, for example, a culture solution containing a serum substitute at a concentration of approximately 0.1 to 3%, preferably of approximately 1% for N2 supplement, at a concentration of approximately 0.1 to 10%, preferably of 2% for B27 supplement, at a concentration preferably of approximately 1 to 30% for KSR can be used.

In step (2b), a Wnt signaling inhibitor and a TGFβ signaling inhibitor, which are used previously in step (2a), are not needed. In one embodiment, neither a Wnt signaling inhibitor nor a TGFβ signaling inhibitor is contained in the culture medium to be used in step (2b).

In one embodiment, the culture solution in step (2b) is preferably a serum-free culture solution substantially free of serum, and more preferably a serum-free culture solution containing a serum substitute.

In order to promote induction of differentiation into cerebral cortical cells, it is preferable that the culture medium to be used in step (2b) contain N2 supplement as a serum substitute. N2 supplement is a known serum substitute composition containing insulin, transferrin, progesterone, putrescine, and sodium selenite, and can be purchased from, for example, Gibco/Thermo Fisher Scientific Inc. The loading of N2 supplement can be appropriately set so that induction of differentiation into forebrain tissue or precursor tissue thereof, induction of differentiation into neural cells, and/or induction of differentiation into cells constituting the cerebral cortex or progenitor cells thereof can be promoted.

It is preferable that the culture medium to be used in step (2b) contain a chemically defined lipid concentrate for long-term maintenance culture of the ventricular zone. Chemically defined lipid concentrates are lipid mixtures containing cholesterol, DL-α-tocopherol, arachidonic acid, linolenic acid, linoleic acid, myristic acid, oleic acid, palmitic acid, palmitoleic acid, and stearic acid each purified. Commercially available chemically defined lipid concentrates can be used, and can be purchased from, for example, Gibco/Thermo Fisher Scientific Inc.

The culture medium to be used for suspension culture of a cell aggregate can contain an additional additive unless the additional additive adversely affects induction of differentiation into cerebral cortical cells. Examples of the additive include, but are not limited to, insulin, iron sources (e.g., transferrin), minerals (e.g., sodium selenate), saccharides (e.g., glucose), organic acids (e.g., pyruvic acid, lactic acid), serum proteins (e.g., albumin), amino acids (e.g., L-glutamine), reducing agents (e.g., 2-mercaptoethanol), vitamins (e.g., ascorbic acid, d-biotin), antibiotics (e.g., streptomycin, penicillin, gentamicin), and buffers (e.g., HEPES).

In one embodiment, the culture medium in step (2b) may contain serum. The serum can contribute to long-term maintenance culture of the ventricular zone. Examples of the serum include, but are not limited to, FBS. It is preferable that the serum have been inactivated. The concentration of the serum in the culture medium can be appropriately adjusted within a range that allows contribution to long-term maintenance culture of the ventricular zone, and is typically 1 to 20% (v/v).

In one embodiment, the culture medium in step (2b) may contain heparin. Heparin can contribute to long-term maintenance culture of the ventricular zone. The concentration of heparin in the culture medium can be appropriately adjusted within a range that allows contribution to long-term maintenance culture of the ventricular zone, and is typically 0.5 to 50 $\mu$g/ml, and preferably 1 to 10 $\mu$g/ml (e.g., 5 $\mu$g/ml).

In one embodiment, the culture medium in step (2b) may contain an extracellular matrix component. The extracellular matrix can contribute to long-term maintenance culture of the ventricular zone. The term "extracellular matrix components" refers to components typically found in the extracellular matrix. In the method of the present invention, it is preferable to use a basement membrane component. Examples of main components of the basement membrane include type IV collagen, laminin, heparan sulfate proteoglycan, and entactin. Commercially available extracellular matrix components can be used as the extracellular matrix component to be added to the culture medium, and examples thereof include Matrigel (BD Biosciences) and human-type laminin (Sigma-Aldrich Co. LLC). Matrigel is a basement membrane preparation derived from Engelbreth Holm Swarn (EHS) mouse sarcoma. The main components of Matrigel are type IV collagen, laminin, heparan sulfate proteoglycan, and entactin, and additionally TGF$\beta$, fibroblast growth factor (FGF), tissue plasminogen activator, and growth factors that EHS tumor naturally produces are contained in Matrigel. Growth factor-reduced products of Matrigel have lower growth factor concentrations than normal Matrigel, and the standard concentrations are <0.5 ng/ml for EGF, <0.2 ng/ml for NGF, <5 pg/ml for PDGF, 5 ng/ml for IGF-1, and 1.7 ng/ml for TGF$\beta$. In the method of the present invention, it is preferable to use such a growth factor-reduced product.

The concentration of the extracellular matrix component in the culture medium can be appropriately adjusted within a range that allows contribution to long-term maintenance culture of the ventricular zone, and, in using Matrigel, it is preferable to add a volume of 1/500 to 1/20 of that of the culture solution, and it is more preferable to add a volume of 1/100 of that of the culture solution.

In one embodiment, the culture medium in step (2b) contains serum and heparin in addition to N2 supplement and a chemically defined lipid concentrate. In this embodiment, the culture medium may further contain an extracellular matrix. The culture medium in the present embodiment is suitable for observation of induction of differentiation into the telencephalon or partial tissue thereof, or precursor tissue of any of them over a long period.

In this case, a culture medium containing N2 supplement, a chemically defined lipid concentrate, serum, and heparin (optionally, further containing an extracellular matrix) may be used throughout step (2b), and the culture medium in this embodiment may be used only during a part of the period. In one embodiment, in step (2b), a culture medium containing N2 supplement and a chemically defined lipid concentrate and not containing serum, heparin, and an extracellular matrix may be first used, and the culture medium may be switched to a culture medium containing N2 supplement, a chemically defined lipid concentrate, serum, and heparin (and optionally containing an extracellular matrix) at a certain time point (e.g., after a stage in which a neuroepithelium-like structure (pseudostratified columnar epithelium) having a cerebroventricle-like cavity, for example, a hemispherical or spherical pseudo stratified columnar epithelium having a plurality of cavities has been formed in a Foxg1-positive cell aggregate).

Other culture conditions including culture temperature and $CO_2$ concentration in step (2b) can be appropriately set. The culture temperature is, for example, approximately 30 to 40°C, and preferably approximately 37°C. The $CO_2$ concentration is, for example, approximately 1% to 10%, and preferably approximately 5%.

Step (2b) is performed for a sufficient period such that at least a cell positive for a forebrain marker (e.g., Foxg1) is generated and a cerebral cortex-like structural body is formed. The cerebral cortex-like structural body can be confirmed through microscopy.

Although the culture period in step (2b) can vary depending on the types of the Wnt signaling inhibitor and TGF$\beta$ signaling inhibitor, etc., in step (2a) and hence cannot be definitely specified, the culture period, for example, in the case that human pluripotent stem cells are used is at least 10 days or 10 to 40 days, preferably 10 to 31 days, and more preferably 15 to 24 days.

Herein, stable self-assembly of a cerebral cortex-like structural body can be provoked in a cell aggregate by performing the culture step of step (2b) over a long period (e.g., for 20 days or more, preferably for 50 days or more, more preferably for 70 days or more); if step (2b) is continued, the differentiation stage of the cerebral tissue included in a cell aggregate proceeds as time passes. Accordingly, it is preferable to continue step (2b) until achievement of a desired differentiation stage, specifically, the formation of a cerebral organoid including a plurality of cerebral cortex-like structural bodies.

Examples of the incubator to be used for suspension culture of a cell aggregate in step (2b) include, but are not limited to, flasks, tissue culture flasks, dishes, Petri dishes, tissue culture dishes, multidishes, microplates, microwell plates, micropores, multiplates, multiwell plates, chamber slides, Schale, tubes, trays, culture bags, bioreactors, and roller bottles. In order to enable culture under nonadhesive conditions, it is preferable that the incubator be nonadhesive to cells. As an incubator nonadhesive to cells, for example, an incubator the surface of which has been artificially treated to make the surface nonadhesive to cells, or an incubator the surface of which has not been artificially treated for the purpose of enhancing the adhesion to cells (e.g., coating treatment with an extracellular matrix or the like) can be used.

In one embodiment, as the incubator to be used for suspension culture of a cell aggregate in step (2b), an oxygen-permeable incubator may be used. Supply of oxygen to a cell aggregate is enhanced by using an oxygen-permeable incubator. In step (2b), an oxygen-permeable incubator may be used to avoid the risk of insufficient supply of oxygen to cells inside of a cell aggregate as a result of significant growth of the cell aggregate.

In suspension culture in step (2b), each cell aggregate may be subjected to static culture, and each cell aggregate may be consciously moved by rotating culture or shaking culture, as long as the nonadhesive state of the cell aggregate to the incubator can be maintained. Static culture may be performed for the whole period of step (2b), and static culture may be performed only during a part of the period.

In one embodiment, the suspension culture in step (2b) is static culture. In this case, it is preferable to culture under the high-oxygen-partial-pressure conditions described above.

In one embodiment, the suspension culture in step (2b) is shaking culture. In this case, culture under the high-oxygen-partial-pressure conditions is not needed.

Through step (2b), a cerebral cortex-like structural body that is positive for a forebrain marker is formed in the cell aggregate. In one embodiment, 70% or more of the cells included in the cell aggregate including a cerebral cortex-like structural body are positive for a forebrain marker (e.g., Foxg1-positive).

In one embodiment, the cerebral cortex-like structural body formed in the cell aggregate through step (2b) is a rosette-like structural body including a neural cell layer external to a neuroepithelium. In one embodiment, the cerebral cortex-like structural body has a cell layer that is positive for a neural stem cell marker, specifically, PAX6- and/or SOX2-positive in the lumenal side, and includes phosphorylated histone H3-positive mitotic cells in the most lumenal part. In one embodiment, cells expressing βIII-tubulin (βTubIII, TUBB3, or TUJ1), which is a marker for postmitotic neural cells, and expressing Ctip2 or Tbr1, each of which is a marker for the early-stage cortical plate of the cerebral cortex, are included in the outer side of the neuroepithelium-like cell layer. These include Reelin-positive Cajal-Retzius cells, which are neural cells in the layer 1 of the cerebral cortex, and can include a laminin-rich layer near the surface layer. That is, in a preferred embodiment, cerebral cortex precursor tissue is included in the cell aggregate obtained by the production method of the present invention.

<Step (3)>

The method of the present invention for producing a cerebral organoid may further comprise step (3), which is a step of screening for a cerebral organoid. Step (3) is a step of screening a desired cerebral organoid from a plurality of cell aggregates obtained in step (2) on the basis of, as indices, one or more selected from the group consisting of the shape, internal structure, size, surface coloring or patterning, and gene expression of a cell aggregate. It is preferable to employ two or more, three or more, or four or more of those indices, and the shape, internal structure, surface coloring or patterning, and/or gene expression of a cell aggregate are/is preferable as such indices or an index, the shape, internal structure, and/or gene expression of a cell aggregate are/is preferable as such indices or an index, and the shape of a cell aggregate is particularly preferable as such an index. For example, if the shape of a cell aggregate is spherical (sphere-like), the cell aggregate can be selected as a desired cerebral organoid.

It is preferable that each index for screening for a cerebral organoid accord with the common definition of cerebral organoids, and, for example, a cell aggregate satisfying at least one, at least two, at least three, or four of the following (1) to (5) may be determined as a cerebral organoid:

(1) being a spherical cell aggregate;
(2) having a cerebral cortex-like structural body inside of the cell aggregate;
(3) having no pigmentation in the surface of the cell aggregate;
(4) having none of cystoid shape, protruding shape, and balloon-like shape in a part of the cell aggregate; and
(5) the cell aggregate is expressing at least one, at least two, at least three, or at least five genes selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, andDLL3.

Regarding (5), it is preferable that the cerebral organoid be expressing one or more genes, preferably all genes selected from the group consisting of SLC17A7, NEUROD6, and EMX1.

Moreover, it is preferable that at least one, at least two, at least three or more, or at least five or more of genes that are expressed in non-target cells, the genes shown in Table 6, be substantially unexpressed. Furthermore, it is preferable that one or more genes selected from the group consisting of GAD2, COL1A1, TYR, TTR, and HOXA2 be substantially unexpressed, and it is more preferable that two or more, three or more, four or more, or all of those markers be not expressed.

Herein, the statement that a gene is "substantially unexpressed" means that the expression level of a protein as an expression product of the gene is less than an expression level that allows the physiological functions to be exerted.

Specific examples of the state "being substantially unexpressed" include such a state that the expression level is 1/10 or less of the expression level of any control gene constitutively expressed in cells (examples of constitutively expressed genes include GAPDH, ACTB, B2M, and 18S ribosomal RNA), as a reference.

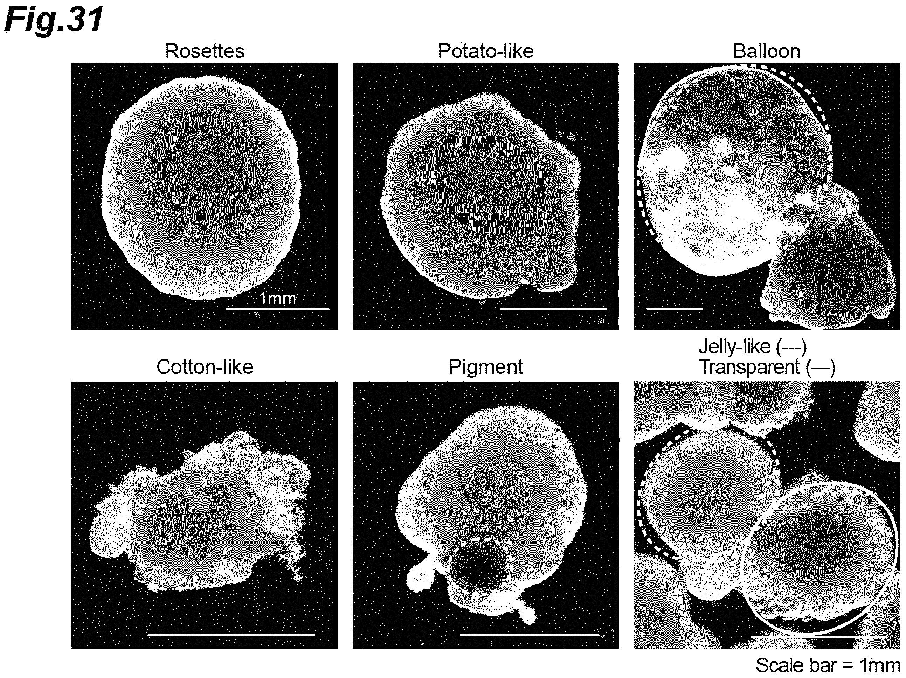

The term spherical in (1) and the cerebral cortex-like structural body in (2) are as described above. Here, a representative example of cell aggregates being spherical but having no cerebral cortex-like structural body is a cell aggregate referred to as "Potato-like" or "Jelly-like" in Figure 31.

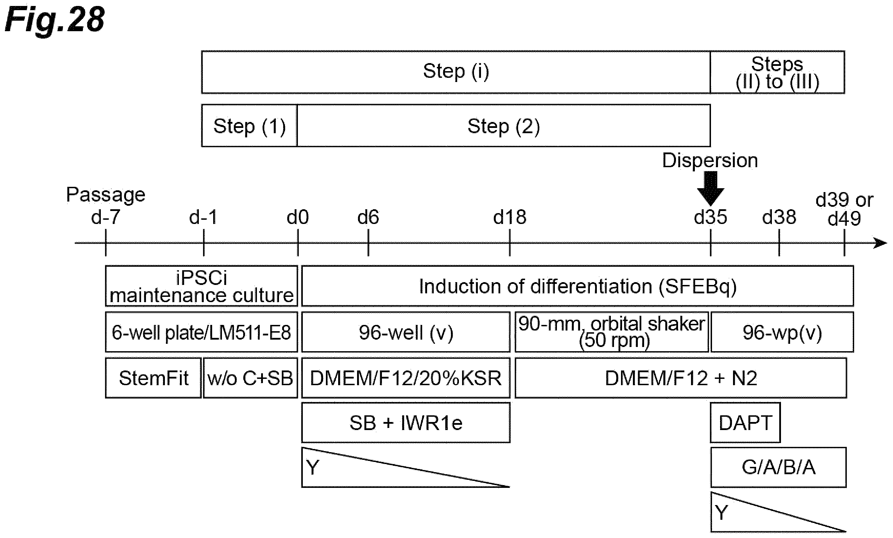

The term pigmentation in (3) means having a black or brown region in a part of a cell aggregate. Here, a representative example of cell aggregates having pigmentation is a cell aggregate referred to as "Pigment" in Figure 28.

The statement of having none of cystoid shape, protruding shape, and balloon-like shape in a part of the cell aggregate means that none of bag-like structure with high degree of transparency, fibrous epithelial structure, and protrusion-like structure is present in the cell aggregate.

Here, a representative example of the cystoid shape is a shape referred to as "Transparent" in Figure 31. Here a representative example of the protruding shape is a shape referred to as "Cotton-like" in Figure 31. Here, a representative example of the balloon-like shape is a shape referred to as "Balloon" in Figure 31. The characteristics of (1) to (4) can be visually determined, optionally with magnification, to exclude cell aggregates. Alternatively, the shapes may be determined from enlarged images through a microscope by using an apparatus with software capable of image analysis. In this case, the precision of determination of the shapes may be enhanced by using a method of deep learning or the like.

The cerebral organoid may be a spherical cell aggregate having a diameter (equivalent circle diameter) of approximately 100 $\mu$m to 10000 $\mu$m, preferably of approximately 500 $\mu$m to 5000 $\mu$m. The cerebral cortex-like structural body included inside of the cell aggregate may have a diameter (equivalent circle diameter) of approximately 10 $\mu$m to 1000 $\mu$m, preferably of approximately 50 $\mu$m to 500 $\mu$m.

Expression of a gene (marker) can be determined from the expression level thereof. The expression level of a gene can be evaluated from the amount of an expression product of the gene (mRNA, or a protein or a fragment thereof), preferably from the expression level of mRNA. Specifically, determination can be performed, for example, by a quantitative RT-PCR method, an RT-PCR method, next-generation sequence analysis, microarray analysis, a Western blotting method, an ELISA method, an immunostaining method, or flow cytometry.

The determination may be performed for every cell aggregate, and, for the same lot, a specific number (e.g., one or more, three or more, five or more) of cell aggregates may be sampled for the determination. The shape, internal structure, size, and surface coloring or patterning of a cell aggregate can be determined by visually checking through inverted microscopy, and it is preferable to determine them for every cell aggregate. For gene expression, it is preferable to determine it not for every cell aggregate but for sampled ones. Specific examples of the determination include determination for cell aggregates selected on the basis of shape and/or internal structure, for example, by a quantitative RT-PCR method, an RT-PCR method, next-generation sequence analysis, microarray analysis, a Western blotting method, an ELISA method, an immunostaining method, or flow cytometry.

In step (3), cell aggregates determined to be cerebral organoids by the determination method are collected and selected as cerebral organoids. Cerebral organoids selected in step (3) are concentrated as compared to those obtained without screening. That is, the proportion of cerebral organoids in the population of cell aggregates can be more increased through step (3).

3. Cerebral Organoid

The cell culture of the present invention is a cell culture produced by the above-described method for producing a cerebral organoid, comprising step (1) and step (2), wherein the cell culture comprises a plurality of spherical cell aggregates, and the proportion of cerebral organoids in the plurality of spherical cell aggregates is 40% or more, preferably 50% or more, and more preferably 60% or more. The proportion can be calculated as the number of cerebral organoids relative to the total number of spherical cell aggregates in a sample obtained by sampling a cell culture.

A cerebral organoid is a spherical cell aggregate including one or more, preferably a plurality of cerebral cortex-like structural bodies each including a neural cell layer external to a neuroepithelium. The determination method for cerebral organoids accords with the method of step (3).

The proportion of cerebral cortex-like structural bodies occupying each cerebral organoid may be 20% or more, preferably 40% or more, and more preferably 50% or more. Here, the proportion of cerebral cortex-like structural bodies occupying a cerebral organoid is the proportion of cerebral cortex-like structural bodies occupying the area or volume of the entire cerebral organoid, and it is preferable from the viewpoint of easy evaluation that the proportion be in terms of area. The proportion may be the mean for a plurality of (e.g., 2 to 20, preferably 5 or 10) cerebral organoids. The proportion may be evaluated with the proportion of cerebral cortex-like structural bodies occupying the cross-sectional area of a section including a central part of a cerebral organoid. Here, a central part is a central position of a cross-section of a cerebral organoid with the maximum diameter. The evaluation may be performed, for example, in such a manner that a cerebral organoid section is subjected to immunostaining and cells positive for a neural stem cell marker and/or neural progenitor cell marker, and a neuronal marker are determined as a cerebral cortex-like structural body, and the proportion of the area of cerebral cortex-like structural bodies to the area of the section is calculated. Alternatively, the proportion may be calculated as the proportion of the volume of cerebral cortex-like structural bodies to the volume of the cerebral organoid.

Here, examples of the neural stem cell and/or neural progenitor cell marker include Pax6, and examples of the neuronal marker include the forebrain marker Foxg1 and the cerebral cortex marker Ctip2.

In a preferred embodiment of the cerebral organoid, 20% or more, preferably 40% or more, preferably 70% or more of all the cells are Foxg1-positive cells.

In one embodiment, each cerebral cortex-like structural body has a cell layer positive for the neural stem cell marker(s) PAX6 and/or SOX2 in the lumenal side, and includes phosphorylated histone H3-positive mitotic cells in the most lumenal part.

In one embodiment, cells expressing βIII-tubulin (βTubIII, TUBB3, or TUJ1), which is a marker for postmitotic neural cells, and expressing Ctip2 and/or Tbr1, each of which is an early-stage cortical plate marker for the cerebral cortex, are included in the outer side of the neuroepithelium-like cell layer. These include Reelin-positive Cajal-Retzius cells, which are neural cells in the layer 1 of the cerebral cortex, and can include a laminin-rich layer near the surface layer.

In one embodiment, each cerebral organoid may be a cerebral organoid produced by using the above method for producing a cerebral organoid, comprising step (1) and step (2). Each cerebral organoid in one embodiment may be a cerebral organoid selected through step (3), and may be a cell aggregate having the following characteristics of (1) to (5):

(1) being a spherical cell aggregate;
(2) having a cerebral cortex-like structural body inside of the cell aggregate;
(3) having no pigmentation in the surface;
(4) having none of cystoid shape, protruding shape, and balloon-like shape in a part of the cell aggregate; and
(5) expressing at least one, at least two, at least three, or at least five genes selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

For (5), it is preferable that the cerebral organoid be expressing one or more, two or more, or all selected from the group consisting of SLC17A7, NEUROD6, and EMX1. Furthermore, it is preferable that one or more genes selected from the group consisting of GAD2, COL1A1, TYR, TTR, and HOXA2 be substantially unexpressed, and it is more preferable that two or more, three or more, four or more, or all of those markers be not expressed.

In one embodiment, each cerebral organoid is as described above, and the number of cells per cell aggregate may be approximately $5 \times 10^3$ to $5 \times 10^6$, and preferably approximately $1 \times 10^4$ to $3 \times 10^6$.

4. Method 1 for Producing Cerebral Cortical Cell aggregate

One embodiment of the present invention includes a method for producing a cerebral cortical cell aggregate from a pluripotent stem cell in the absence of a sustentacular cell, comprising the following step (i) and step (ii):

(i) a step of obtaining a cerebral organoid from the pluripotent stem cell; and
(ii) a step of culturing the cerebral organoid obtained in step (i) in a culture solution containing a Notch signaling inhibitor, preferably a γ-secretase inhibitor, to obtain a cerebral cortical cell aggregate.

In step (i), the method for obtaining a cerebral organoid from a pluripotent stem cell is not limited, and preparation can be performed by using a method well known to those skilled in the art. Examples of such well-known methods include step (2) in the method described in the above section "2" for producing a cerebral organoid.

One embodiment of the present invention includes "method 1 for producing a cerebral cortical cell aggregate", comprising obtaining a cerebral organoid from pluripotent stem cells by the method described in the above section "2" for producing a cerebral organoid in step (i). Specifically, method 1 for producing a cerebral cortical cell aggregate is a method for producing a cerebral cortical cell aggregate from pluripotent stem cells in the absence of sustentacular cells, comprising treating a cerebral organoid obtained by the method described in the above section "2" for producing a cerebral organoid with the following step (i) and step (ii).

(i) A step of obtaining a cerebral organoid by the method described in the above section "2" for producing a cerebral organoid (i.e., including the method according to any one of claims 1 to 10); and
(ii) a step of culturing the cerebral organoid obtained in step (i) in a culture solution containing a Notch signaling inhibitor, preferably a γ-secretase inhibitor, to obtain a cerebral cortical cell aggregate.

<Step (i)>

Step (i) is as described for step (1), step (2a), and step (2b) in the above section "2". Step (i) may further include step (3) in the above section "2".
In one embodiment, step (2b) is performed for approximately 7 to 31 days, preferably for approximately 7 to 21 days.

<Step (ii)>

Step (ii) can be performed in such a manner that, after performing step (2b) described above, screening by step (3) is optionally performed, and culture medium exchange is performed with a culture medium containing a Notch inhibitor, preferably a γ-secretase inhibitor. Alternatively, after performing step (2b) described above, step (ii) and then screening by step (3) may be performed.
As the Notch signaling inhibitor in step (ii), any of those according to the definition can be appropriately selected for use. Preferable examples of the Notch signaling inhibitor to be used in step (ii) include a γ-secretase inhibitor. As the γ-secretase inhibitor, any of those according to the definition can be appropriately selected for use. Preferable examples of the γ-secretase inhibitor include N-[N-(3,5-difluorophenacetyl)-L-alanyl]-S-phenylglycine t-butyl ester (DAPT) and Compound E.
The culture solution to be used in step (ii) can be the same as the culture solution used in step (i), that is, step (2b) of the production method in the above section "2", except that a Notch signaling inhibitor is contained. Alternatively, a culture solution differing from the culture solution used in step (2b) may be appropriately selected with reference to the culture conditions described for step (2b) of the production method in the above section "2".
The culture conditions for step (ii) may accord with those for step (2b), as appropriate.
The concentration of a Notch signaling inhibitor, preferably a γ-secretase inhibitor, in the culture solution can be appropriately set within a range that enables reduction of proliferative cells that can be included in a cell aggregate, such as neural stem cells. Specific examples of the concentration include a concentration that causes the γ-secretase activity corresponding to 0.1 to 1000 $\mu$M, 1 to 100 $\mu$M, preferably to 1 to 30 $\mu$M, more preferably to 5 to 20 $\mu$M in the case that DAPT is used as the γ-secretase inhibitor, or Notch signal inhibitory activity based on the γ-secretase activity.
Step (ii) is performed approximately 28 days to 49 days, preferably approximately 28 days to 44 days after the time point of initiation of suspension culture in step (i) (initiation of induction of differentiation into neural cells).
The culture period in step (ii) is 1 to 7 days, preferably 2 to 6 days, and more preferably 2 to 4 days.

5. Method 2 for Producing Cerebral Cortical Cell aggregate

One embodiment of the present invention includes a method for producing a cerebral cortical cell aggregate from pluripotent stem cells in the absence of sustentacular cells, and the method comprises:

(i) a step of obtaining a cerebral organoid from pluripotent stem cells;
(ii) a step of culturing the cerebral organoid obtained in step (i) in a culture solution;
(iii) a step of dispersing a cell culture obtained in step (ii) into single cells or two- to five-membered cell clumps; and
(iv) a step of culturing a cell culture obtained in step (ii) or a cell population obtained in step (iii) in a culture solution containing one or more neurotrophic factors, ascorbic acid, and a cAMP activator, wherein step (iii), which is a step of dispersing into single cells or two- to five-membered cell clumps, is an optional step, and the culture solution in step (ii) and/or the culture solution in step (iv) contain(s) a Notch signaling inhibitor.

In step (i), the method for obtaining a cerebral organoid from pluripotent stem cells is not limited, and preparation can be performed by using a method well known to those skilled in the art. In step (i), a cerebral organoid is obtained from the pluripotent stem cell preferably by the method described in the above section "2" for producing a cerebral organoid, in particular, the production method comprising step (1), step (2a), and step (2b).
An embodiment in which the culture solution in step (ii) and/or the culture solution in step (iv) contain(s) a Notch signaling inhibitor during a part or the whole of the period of the corresponding step is acceptable. In the case that the time span of treatment with a Notch signaling inhibitor is a part of the period, a culture solution containing no Notch signaling inhibitor is used in exchanging the culture solution in step (ii) or the culture solution in step (iv). The part of the period is not limited as long as the effect of treatment with a Notch signaling inhibitor is obtained, and may be several hours to several days of the culture period in step (ii) or step (iv), specifically, 1 to 7 days, preferably 2 to 6 days, and more preferably 2 to 4 days. Repetition of very short time span of treatment (e.g., 4 hours, 12 hours, 24 hours, 2 days) is also acceptable. Alternation of a period with a Notch signaling inhibitor and a period without a Notch signaling inhibitor and combination with varied concentrations of a Notch signaling inhibitor also fall within the scope of the present application. For the Notch signaling inhibitor to be used here and the concentration thereof, the description of step (ii) in method 1 for producing a cerebral cortical cell aggregate can be referred.

One embodiment of the present invention includes "method 2-1 for producing a cerebral cortical cell aggregate", wherein step (i) is performed according to the method described in the above section "2" for producing a cerebral organoid and method 2-1 comprises step (iii). Specifically, method 2-1 for producing a cerebral cortical cell aggregate is a method for producing a high-purity cerebral cortical cell aggregate, comprising treating through step (iii) and step (iv-1) below in addition to step (i) and step (ii) in method 1 described in the above section "4" for producing a cerebral cortical cell aggregate. The high-purity cerebral cortical cell aggregate is one resulting from dispersion and reaggregation. Here, in step (ii), a cerebral organoid obtained in step (i) is cultured in a culture solution containing a Notch signaling inhibitor, preferably a γ-secretase inhibitor. This step (ii) can be performed in the same manner as step (ii) in method 1 in the above section "4" for producing a cerebral cortical cell aggregate.

(iii) A step of dispersing a cerebral organoid obtained in step (ii) into single cells or two- to five-membered cell clumps; and

(iv-1) a step of culturing a cell population obtained in step (iii) in a serum-free culture solution containing one or more neurotrophic factors, ascorbic acid, and a cAMP activator (e.g., dibutyryl cAMP (dbcAMP)) to obtain a cell aggregate (e.g., consisting of 20 or more cells).

<Step (iii)>

A cerebral organoid obtained in step (ii) can be dispersed into single cells or two- to five-membered cell clumps physically, for example, by pipetting, or through enzymatic treatment. Here, a cell population obtained by dispersing can be a population of single cells and/or two- to five-membered cell clumps. That is, in step (iii), a cerebral organoid obtained in step (ii) may be dispersed into single cells or two- to five-membered cell clumps by a method well known to those skilled in the art.

<Step (iv-1)>

A cell aggregate including cerebral cortical cells at high purity (high-purity cerebral cortical cell aggregate) can be obtained by reaggregating a cell population obtained in step (iii) through suspension culture.

The culture solution to be used in step (iv-1) is a culture solution containing one or more neurotrophic factors, ascorbic acid or a derivative thereof, and cAMP activator.

As the neurotrophic factors, one to four, one to three, preferably one or two factors can be selected from those according to the above definition. Preferable examples of the neurotrophic factors include BDNF and/or GDNF. Examples of the concentration of BDNF include 1 to 100 ng/mL, preferably 10 to 30 ng/mL. Examples of the concentration of GDNF include 1 to 100 ng/mL, preferably 5 to 20 ng/mL.

Examples of the concentration of ascorbic acid or a derivative thereof (e.g., ascorbic acid-2-phosphate) include a concentration corresponding to 10 to 500 μM, preferably to 50 to 300 μM, for ascorbic acid, and the same concentration also for a derivative thereof.

Examples of the concentration of the cAMP activator include a concentration that allows cAMP activation corresponding to 10 to 1000 μM, preferably to 100 to 600 μM, more preferably to 300 to 500 μM in the case that dbcAMP is used.

The culture period in step (iv-1) is 1 to 21 days, preferably 2 to 15 days, 2 to 10 days, 2 to 8 days, 2 to 6 days, and more preferably approximately 4 days. Alternatively, the culture period in step (iv-1) is 1 to 40 days, preferably 2 to 28 days, and more preferably 2 to 14 days.

The suspension culture in step (iv-1) can be performed with reference to the method described for step (2b) in the above section "2". For example, a culture medium prepared by adding one or more neurotrophic factors, ascorbic acid or a derivative thereof, and a cAMP activator to the same culture medium as in step (2b) in the above section "2" can be used.

The culture solution to be used in step (iv-1) may optionally contain a ROCK inhibitor. As the ROCK inhibitor, any of those according to the above definition can be appropriately used, and preferable examples thereof include Y-27632.

Examples of the concentration of the ROCK inhibitor to be used in this case include a concentration corresponding to approximately 0.1 to 200 μM, preferably to approximately 2 to 100 μM, more preferably to approximately 30 to 100 μM in the case that Y-27632 is used as the ROCK inhibitor. Here, a cerebral cortical cell aggregate to be obtained through step (iii) is a high-purity cell aggregate of cerebral cortical cells formed through the step of dispersing-reaggregating.

6. Method 2-2 for Producing Cerebral Cortical Cell aggregate

One embodiment of the present invention includes an embodiment such that method 2-1 described in the above section "5" for producing a cerebral cortical cell aggregate lack step (iii). Specifically, such an example is a method for producing a cerebral cortical cell aggregate, comprising treating through step (iv-2) below in addition to step (i) and step (ii) in method 1 described in the above section "4" for producing a cerebral cortical cell aggregate. Here, in step (ii), a cerebral organoid obtained in step (i) is cultured in a culture solution containing a Notch signaling inhibitor, preferably a γ-secretase inhibitor.
(iv-2) A step of subjecting a cell culture containing a cerebral organoid obtained in step (ii) to suspension culture in a culture solution containing one or more neurotrophic factors, ascorbic acid or a derivative thereof (e.g., ascorbic acid-2-phosphate), and a cAMP activator (e.g., dibutyryl cAMP (dbcAMP)) to obtain a cerebral cortical cell aggregate.

<Step (iv-2)>

A cell aggregate including cerebral cortical cells (cerebral cortical cell aggregate) can be obtained by subjecting a cell culture containing a cerebral organoid obtained in step (ii) to suspension culture.
Examples of the culture solution to be used in step (iv-2) include the culture solution described for step (iv-1) in the above section "5", and, similarly, examples of conditions and culture period for the suspension culture include those described for step (iv-1) in the above section "5".

7. Methods 3-1 to 3-7 for Producing Cerebral Cortical Cell aggregate

One embodiment of the present invention includes "method 3-1 for producing a cerebral cortical cell aggregate", wherein the culture solution in step (ii) contains a Notch signaling inhibitor. Specific examples of method 3-1 for producing a cerebral cortical cell aggregate include a method for producing a cerebral cortical cell aggregate, comprising the following step (I). (I) A step of culturing a cerebral organoid derived from pluripotent stem cells in a culture solution containing a Notch inhibitor, preferably a γ-secretase inhibitor.
Here, step (I) can be performed in the same manner as step (ii) in method 1 for producing a cerebral cortical cell aggregate.
One embodiment of the present invention includes "method 3-2 for producing a cerebral cortical cell aggregate", comprising treating through the following steps in addition to step (I).

(II) A step of dispersing a cell culture containing a cerebral organoid obtained in step (I) into single cells or two- to five-membered cell clumps; and
(III) a step of culturing a cell population obtained in step (II) in a culture solution containing one or more neurotrophic factors, ascorbic acid or a derivative thereof, and a cAMP activator to obtain a cerebral cortical cell aggregate.

Here, step (I) can be performed in the same manner as step (ii) in method 1 for producing a cerebral cortical cell aggregate, and step (II) and step (III) can be performed in the same manner as step (iii) and step (iv-1) in method 2 for producing a cerebral cortical cell aggregate. A cerebral cortical cell aggregate to be obtained through step (III) is a high-purity cell aggregate of cerebral cortical cells, which has been formed through the step of dispersing-reaggregating.
As one embodiment of the present invention includes an embodiment without step (II). Specifically, such an example is "method 3-3 for producing a cerebral cortical cell aggregate", comprising treating through the following step in addition to step (I).
(III-2) A step of subjecting a cell culture containing a cerebral organoid obtained in step (I) to suspension culture in a culture solution containing one or more neurotrophic factors, ascorbic acid or a derivative thereof (e.g., ascorbic acid-2-phosphate), and a cAMP activator (e.g., dibutyryl cAMP (dbcAMP)) to obtain a cerebral cortical cell aggregate.
Here, step (I) can be performed in the same manner as step (ii) in method 1 for producing a cerebral cortical cell aggregate, and step (III-2) can be performed in the same manner as step (iv-1) in method 2 for producing a cerebral cortical cell aggregate.
One embodiment of the present invention includes "method 3-4 for producing a cerebral cortical cell aggregate", comprising step (I-2), step (II), and step (III-3) below. This method comprises:

(I-2) a step of culturing a cerebral organoid derived from pluripotent stem cells in a culture solution;

(II) a step of dispersing a cell culture containing a cerebral organoid obtained in step (I-2) into single cells or two-to five-membered cell clumps; and

(III-3) a step of culturing a cell population obtained in step (II) in a culture solution containing one or more neurotrophic factors, ascorbic acid or a derivative thereof, and a cAMP activator to obtain a cerebral cortical cell aggregate, wherein the culture solution in step (I-2) contains no Notch signaling inhibitor, and the culture solution in step (III-3) contains a Notch signaling inhibitor. Here, step (I-2) can be performed in the same manner as step (2b) in the method described in the above section "2" for producing a cerebral organoid. Step (I-2) may be culture corresponding to a part of the step of producing a cerebral organoid derived from pluripotent stem cells, and the culture period is not limited. Step (II) can be performed in the same manner as step (iii) in method 2-1 described in the above section "5" for producing a cerebral cortical cell aggregate.

In some embodiment, the culture solution in step (III-3) may contain a Notch signaling inhibitor during a part or the whole of the period of the step. In the case that the time span of treatment with a Notch signaling inhibitor is a part of the period, a culture solution containing no Notch signaling inhibitor is used in exchanging the culture solution in step (III-3). The part of the period is not limited as long as the effect of treatment with a Notch signaling inhibitor is obtained, and may be several hours to several days of the culture period in step (III-3), specifically, 1 to 7 days, preferably 2 to 6 days, and more preferably 2 to 4 days. Repetition of very short time span of treatment (e.g., 4 hours, 12 hours, 24 hours, 2 days) is also acceptable. Alternation of a period with a Notch signaling inhibitor and a period without a Notch signaling inhibitor and combination with varied concentrations of a Notch signaling inhibitor also fall within the scope of the present application. As with the case of the culture solution in step (III-3), in some embodiment, culture solutions in step (III-4) and step (III-5) below may contain a Notch signaling inhibitor during a part or the whole of the period of the step.

<Step (III-3)>

A cell aggregate including cerebral cortical cells at high purity (high-purity cerebral cortical cell aggregate) can be obtained by reaggregating a cell population obtained in step (II) through suspension culture.

Examples of the culture solution to be used in step (III-3) include the culture solution described for step (iv-1) in method 2 described in the above section "5" for producing a cerebral cortical cell aggregate, and, similarly, examples of conditions and culture period for the suspension culture include those described for step (iv-1) in the above section "5".

The culture solution in step (III-3) contains a Notch signaling inhibitor, preferably a γ-secretase inhibitor, during a part or the whole of the period of the suspension culture.

As the Notch signaling inhibitor to be used here, any of those according to the definition can be appropriately selected for use, and preferable examples of the Notch signaling inhibitor include a γ-secretase inhibitor. As the γ-secretase inhibitor, any of those according to the definition can be appropriately selected for use, and preferable examples of the γ-secretase include N-[N-(3,5-difluorophenacetyl)-L-alanyl]-S-phenylglycine t-butyl ester (DAPT) and Compound E.

The concentration of a Notch signaling inhibitor, preferably a γ-secretase inhibitor, in the culture solution can be appropriately set within a range that enables reduction of proliferative cells that can be included in a cell aggregate to be generated. Specific examples of the concentration include a concentration that causes the γ-secretase activity corresponding to 0.1 to 1000 μM, 1 to 100 μM, preferably to 1 to 30 μM, more preferably to 5 to 20 μM in the case that DAPT is used as the γ-secretase inhibitor, or Notch signal inhibitory activity based on the γ-secretase activity.

In step (III-3), the time at which culture in the presence of a Notch signaling inhibitor, preferably a γ-secretase inhibitor, is initiated is not limited, and examples thereof include day 28 to 42 after initiation of differentiation. The period of the culture is preferably approximately 1 day to approximately 20 days, and more preferably 2 days to 8 days. A cerebral cortical cell aggregate to be obtained through step (III-3) is a high-purity cell aggregate of cerebral cortical cells, which has been formed through the step of dispersing-reaggregating.

One embodiment of the present invention includes an embodiment comprising step (I) in the above, step (II) in the above, and step (III-4), wherein the culture solution in step (I) and a culture solution in step (III-4) both contain a Notch signaling inhibitor (method 3-5 for producing a cerebral cortical cell aggregate). Here, step (I) can be performed in the same manner as step (ii) in method 1 described in the above section "4" for producing a cerebral cortical cell aggregate, step (II) can be performed in the same manner as step (iii) in method 2 described in the above section "5" for producing a cerebral cortical cell aggregate, and step (III-4) can be performed in the same manner as step (III-3) in method 3-4 for producing a cerebral cortical cell aggregate. A cerebral cortical cell aggregate to be obtained through step (III-4) is a high-purity cell aggregate of cerebral cortical cells, which has been formed through the step of dispersing-reaggregating.

One embodiment of the present invention includes an embodiment comprising step (I) in the above and step (III-5), wherein the culture solution in step (III) contains a Notch signaling inhibitor (method 3-6 for producing a cerebral cortical cell aggregate). Here, step (I) can be performed in the same manner as step (ii) in method 1 described in the above section "4" for producing a cerebral cortical cell aggregate, and step (III-5) can be performed in the same manner as step (III-3) in method 3-4 for producing a cerebral cortical cell aggregate.

Here, the statement of culturing in a culture solution containing a Notch signaling inhibitor in step (I) and/or step (III-5) means that the culture solution to be used during a part of the culture period of step (I) and/or step (III-5) or the whole of the culture period thereof contains a Notch signaling inhibitor.

In the case that the time span of treatment with a Notch signaling inhibitor is a part of the period, a culture solution containing no Notch signaling inhibitor is used in exchanging the culture solution in step (I) or the culture solution in step (III-5). The part of the period is not limited as long as the effect of treatment with a Notch signaling inhibitor is obtained, and may be several hours to several days of the culture period in step (I) or step (III-5), specifically, 1 to 7 days, preferably 2 to 6 days, and more preferably 2 to 4 days. Repetition of short time span of treatment (e.g., 4 hours, 12 hours, 24 hours, 2 days) is also acceptable. Alternation of a period with a Notch signaling inhibitor and a period without a Notch signaling inhibitor and combination with varied concentrations of a Notch signaling inhibitor also fall within the scope of the present application.

One embodiment of the present invention includes an embodiment such that step (I) of method 3-6 for producing a cerebral cortical cell aggregate is changed to step (I-2) in the above (method 3-7 for producing a cerebral cortical cell aggregate). This embodiment is performed according to method 3-6 for producing a cerebral cortical cell aggregate, except that step (I) is changed to step (I-2). The culture solution in step (III-5) is a culture solution containing one or more neurotrophic factors, ascorbic acid or a derivative thereof, and a cAMP activator, and further containing a Notch signaling inhibitor.

In methods 3-1 to 3-7 for producing a cerebral cortical cell aggregate, the method for producing a cerebral organoid to be used in step (I) or (I-2) is not limited, and a cerebral organoid can be prepared by a method well known to those skilled in the art. For example, a cerebral organoid can be produced according to the method described in the above section "2" for producing a cerebral organoid. Alternatively, step (2) can be performed by using pluripotent stem cells in place of "cells to be obtained in step (1)" in step (2), without performing step (1) in the method described in the above section "2" for producing a cerebral organoid.

In methods 3-1 to 3-7 for producing a cerebral cortical cell aggregate, culture may be performed by the method described for step (2b) in the above section "2" or with a culture medium that allows neural cells to survive in place of the treatment through the steps of (III) to (III-5). In this case, any of the case with addition of a Notch signaling inhibitor and the case without addition of a Notch signaling inhibitor can be selected.

Herein, representative embodiments of implementation of the method for producing a cerebral cortical cell aggregate have been shown, whereas it is contemplated that the efficiency of cerebral cortical cell aggregate formation varies with the characteristics of a cerebral organoid to be used (e.g., genotype, tissue shape, proportions of cell types included, maturity), and apparatuses, instruments, and places for performing the steps. Accordingly, concentrations in treatment with an agent (e.g., a Notch signaling inhibitor), time of initiation, period, and culture period in an agent-free culture medium before and after treatment can be appropriately adjusted as long as a desired cerebral cortical cell aggregate is obtained.

One embodiment of the present invention includes a method for producing a cerebral cortical cell preparation from pluripotent stem cells in the absence of sustentacular cells, comprising a step of collecting a cerebral cortical cell aggregate obtained by any of method 1 in the above section "4" for producing a cerebral cortical cell aggregate, method 2 in the above section "5" for producing a cerebral cortical cell aggregate, method 2-2 in the above section "6" for producing a cerebral cortical cell aggregate, and methods 3-1 to 3-7 in the above section "7" for producing a cerebral cortical cell aggregate and preparing a cerebral cortical cell preparation containing the cell aggregate and a medium.

Examples of the medium to prepare the cerebral cortical cell preparation for unfrozen type include, but are not limited to, solutions including physiological saline, phosphate buffer (PBS(-)), Hanks' balanced salt solution (HBSS), Earle's balanced salt solution, and ARTCEREB.

Examples of the medium to prepare the cerebral cortical cell preparation for frozen type include, but are not limited to, solutions containing a cryoprotective agent such as DMSO, glycerol, polyethylene glycol, propylene glycol, glycerin, polyvinylpyrrolidone, sorbitol, dextran, and trehalose, and commercially available cryopreservation liquids such as Cell banker, Stem cell banker, and Bambanker.

8. Cerebral Cortical Cell aggregate

One embodiment of the present invention includes a cerebral cortical cell aggregate having characteristics below. The cerebral cortical cell aggregate can be produced by method 1 described in the above section "4" for producing a cerebral cortical cell aggregate.

One particular embodiment of the present invention includes a cerebral cortical cell aggregate having the following characteristics (a) to (c).

(a) The number of cells positive for a proliferation marker is 10% or less of the total number of cells;

(b) the number of cells positive for one or more markers selected from the group consisting of a neuronal marker, a cortical layer V/VI marker, and a forebrain marker is 60% or more, preferably 70% or more, more preferably 80% or more of the total number of cells; and

(c) the cerebral cortical cell aggregate includes substantially no neuroepithelium or cerebral cortex-like structure.

Examples of the proliferation marker include Ki67. Examples of the neuronal marker include βIII-tubulin (βTubIII). Examples of the cortical layer V/VI marker include Ctip2. Examples of the forebrain marker include Foxg1.

One embodiment includes a cerebral cortical cell aggregate having the following characteristics:

(a') the number of Ki67-positive cells is 5% or less of the total number of cells;

(b') the number of cells positive for all of βIII-tubulin (βTubIII), Ctip2, and Foxg1 is 60% or more, preferably 70% or more, more preferably 80% or more of the total number of cells; and

(c) the cerebral cortical cell aggregate includes substantially no neuroepithelium or cerebral cortex-like structure.

The neuroepithelium and cerebral cortex-like structure in the cerebral organoid can be visually checked through inverted microscopy.

In one embodiment, examples of the situation of including substantially no neuroepithelium or cerebral cortex-like structure include a situation in which the neuroepithelium or cerebral cortex-like structure mentioned above cannot be visually identified through inverted microscopy or the like.

In one embodiment, the cerebral cortical cell aggregate is further expressing at least one marker selected from the group consisting of:

(d) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3. The cerebral cortical cell aggregate is preferably expressing one or more genes selected from the group consisting of SLC17A7, NEUROD6, and EMX1, more preferably substantially unexpressing one or more genes selected from the group consisting of GAD2, COL1A1, TYR, TTR, and HOXA2.

In one embodiment, the cerebral cortical cell aggregate further has a characteristic:

(e) the number of cells positive for a neural stem cell marker is 10% or less of the total number of cells, wherein examples of the neural stem cell marker include Pax6, Sox1, and Sox2.

In one embodiment, the cerebral cortical cell aggregate further has a characteristic:

(f) being negative for a pluripotency marker.

Examples of the situation of being negative for a pluripotency marker include a situation in which substantially no pluripotent stem cell is detected, specifically, the number of cells positive for a pluripotency marker is 1% or less of the total number of cells. Examples of the pluripotency marker include Oct4.

In one embodiment, optionally, the cerebral cortical cell aggregate may further have a characteristic: (g) cells positive for a cortical layer II-IV marker (SATB2) are present.

One embodiment of the present invention includes a cell population including the above cerebral cortical cell aggregate in a proportion of 10% or more, preferably of 20% or more, more preferably of 40% or more, even more preferably of 50% or more of total cell aggregates.

In one embodiment of the present invention, each cerebral cortical cell aggregate may be a spherical cell aggregate having a diameter (equivalent circle diameter) of approximately 100 μm to 1000 μm, preferably of approximately 300 μm to 600 μm. The number of cells per cell aggregate may be approximately $1 \times 10^3$ to $5 \times 10^4$, and preferably approximately $5 \times 10^3$ to $2 \times 10^4$. In one embodiment of the present invention, alternatively, each cerebral cortical cell aggregate may be a spherical cell aggregate having a diameter (equivalent circle diameter) of approximately 100 μm to 5000 μm, preferably of approximately 300 μm to 2000 μm. The number of cells per cell aggregate may be approximately $5 \times 10^3$ to $5 \times 10^6$, and preferably approximately $1 \times 10^4$ to $3 \times 10^6$.

In one embodiment of the present invention, the mean of diameter (equivalent circle diameter) for cerebral cortical cell aggregates in the cell population thereof is approximately 300 μm to 2000 μm.

In one embodiment of the present invention, the cerebral cortical cell aggregate and the cell population thereof are characterized in that, when being transplanted into the brain in vivo, they survive at the site of transplantation and the proliferation of transplant-derived cells is suppressed. For example, in transplantation of the cerebral cortical cell aggregate into the mouse brain, the volume of the transplant 3 months after transplantation of the cerebral cortical cell aggregate in Week 5 of induction of differentiation was as small as 2% to 50% of that of a transplant of a cerebral organoid at the same stage.

As described later, the cerebral cortical cell aggregate and the cell population thereof are useful in cell transplantation therapy for patients affected by cerebrovascular disorder or patients with head trauma.

9. High-Purity Cerebral Cortical Cell Aggregate

One embodiment of the present invention includes a high-purity cerebral cortical cell aggregate having characteristics below. The high-purity cerebral cortical cell aggregate, which has characteristics below with a reduced number of cells positive for a proliferation marker and an enhanced content of target neural cells, can be produced by any of method 2-1 described in the above section "5" for producing a cerebral cortical cell aggregate, method 2-2 described in the above section "6" for producing a cerebral cortical cell aggregate, and methods 3-1 to 3-7 described in the above section "7" for producing a cerebral cortical cell aggregate.
One particular embodiment of the present invention includes a high-purity cerebral cortical cell aggregate having the following characteristics.

(A) The number of cells positive for a proliferation marker is 5% or less, preferably 3% or less, more preferably 1% or less of the total number of cells;
(B) the number of cells positive for one or more markers selected from a neuronal marker, a cortical layer V/VI marker, and a forebrain marker is 60% or more, preferably 70% or more, more preferably 80% or more of the total number of cells; and
(C) the high-purity cerebral cortical cell aggregate includes substantially no neuroepithelium or cerebral cortex-like structure.

Examples of the proliferation marker include Ki67. Examples of the neuronal marker include βIII-tubulin (βTubIII). Examples of the cortical layer V/VI marker include Ctip2. Examples of the forebrain marker include Foxg1.
One embodiment includes a high-purity cerebral cortical cell aggregate having the following characteristics:

(A) the number of Ki67-positive cells is 5% or less of the total number of cells;
(B) the number of cells positive for all of βIII-tubulin (βTubIII), Ctip2, and Foxg1 is 60% or more, preferably 70% or more, more preferably 80% or more of the total number of cells; and
(C) the high-purity cerebral cortical cell aggregate includes substantially no neuroepithelium or cerebral cortex-like structure.

In one embodiment, examples of the situation of including substantially no neuroepithelium or cerebral cortex-like structure include a situation in which the neuroepithelium or cerebral cortex-like structure mentioned above cannot be visually identified through inverted microscopy or the like.
In the present invention, examples of a method for obtaining a high-purity cerebral cortical cell aggregate, specifically, a step effective for achieving a larger content of cerebral cortical cells and a smaller content of non-target cells such as proliferative cells in a cerebral cortical cell aggregate include the steps described above in Step (iv) or Step (III) (a Notch signaling inhibitor may be present but is not essential), more preferably, a step including, in addition to that step, the step of dispersing a cerebral cortical cell aggregate and reaggregating the resultant, described above in Step (iii) or Step (II).
In one embodiment, the high-purity cerebral cortical cell aggregate is further expressing at least one, at least two, at least three, or at least five markers selected from the group consisting of:
(D) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3. The cerebral cortical cell aggregate is preferably expressing one or more genes or all genes selected from SLC17A7, NEUROD6, and EMX1, more preferably substantially unexpressing one or more genes selected from the group consisting of GAD2, COL1A1, TYR, TTR, and HOXA2.
In one embodiment, the high-purity cerebral cortical cell aggregate further has a characteristic:
(E) the number of cells positive for a neural stem cell marker is 10% or less, preferably 5% or less, more preferably 3% or less of the total number of cells, wherein
examples of the neural stem cell marker include Pax6, Sox1, and Sox2.
In one embodiment, the high-purity cerebral cortical cell aggregate further has a characteristic:
(F) the high-purity cerebral cortical cell aggregate is negative for a pluripotency marker.
Examples of the situation of being negative for a pluripotency marker include a situation in which substantially no pluripotent stem cell is detected, specifically, the number of cells positive for a pluripotency marker is 1% or less of the total number of cells.

Examples of the pluripotency marker include Oct4.

In one embodiment, optionally, the high-purity cerebral cortical cell aggregate may further have a characteristic: (G) cells positive for a cortical layer II-IV marker (SATB2) may be present.

One embodiment of the present invention includes a cell population including the above cerebral cortical cell aggregate in a proportion of 10% or more, preferably o 20% or more, more preferably of 40% or more, even more preferably of 50% or more of total cell aggregates.

In one embodiment of the present invention, each high-purity cerebral cortical cell aggregate may be a spherical cell aggregate having a diameter (equivalent circle diameter) of approximately 100 $\mu$m to 10000 $\mu$m, preferably of approximately 200 $\mu$m to 3000 $\mu$m. The number of cells per cell aggregate may be approximately $1 \times 10^3$ to $5 \times 10^6$, and preferably approximately $5 \times 10^3$ to $3 \times 10^6$.

In one embodiment of the present invention, the mean of diameter (equivalent circle diameter) for high-purity cerebral cortical cell aggregates in the cell population thereof is approximately 300 $\mu$m to 500 $\mu$m.

In one embodiment of the present invention, the high-purity cerebral cortical cell aggregate and the cell population obtained therefrom are characterized in that, when being transplanted into the brain in vivo, they survive at the site of transplantation and the proliferation of transplant-derived cells is suppressed. For example, in transplantation into the mouse brain, the volume of the transplant 5 weeks after transplantation can be 2% to 50% of that immediately after transplantation.

As described later, the high-purity cerebral cortical cell aggregate and the cell population obtained therefrom are useful in cell transplantation therapy for patients affected by cerebrovascular disorder.

One embodiment of the present invention includes a cell population of high-purity cerebral cortical cell aggregates not only having the above characteristics but also with the high-purity cerebral cortical cell aggregates being homogenous in size, constituent cell composition, or shape. The cell population can be produced by any of methods 2-1, 2-2, and 3-1 to 3-6 described in sections 5 to 7 for producing a cerebral cortical cell aggregate. Specifically, the cell population of high-purity cerebral cortical cell aggregates homogenous in size, constituent cell composition, or shape can be produced by dispersing cerebral organoids and reaggregating the resultant according to the production method.

Here, the homogeneity can be determined on the basis of, as indices, variations of numerical values of the expression levels of markers for different types of cells, size, numbers of cells included in one cell aggregate, and others in high-purity cerebral cortical cell aggregates, wherein the high-purity cerebral cortical cell aggregates are homogenous if each of the variations is ±20% or less, preferably ±10% or less, more preferably ±5% or less.

As described later, the high-purity cerebral cortical cell aggregate and the cell population thereof are useful in cell transplantation therapy for patients affected by cerebrovascular disorder or patients with head trauma.

10. Pharmaceutical Composition

One embodiment of the present invention includes a pharmaceutical composition containing, as an active ingredient, a cerebral organoid obtained by the method described in the above section "2" for producing a cerebral organoid or the cerebral organoid described in the above section "3", a cell population including cerebral cortical cells obtained from any of these cerebral organoids, a cerebral cortical cell aggregate obtained by any of the methods described in sections 4 to 7 for producing a cerebral cortical cell aggregate, the cerebral cortical cell aggregate described in the above section "8" or "9", or a cell population including cerebral cortical cells obtained from any of these cell aggregates. In other words, a cerebral organoid obtained by the method described in the above section "2" for producing a cerebral organoid or the cerebral organoid described in the above section "3", a cell population including cerebral cortical cells obtained from any of these cerebral organoids, a cerebral cortical cell aggregate obtained by any of the methods described in sections 4 to 7 for producing a cerebral cortical cell aggregate, the cerebral cortical cell aggregate described in the above section "8" or "9", or a cell population including cerebral cortical cells obtained from any of these cell aggregates is applicable as an active ingredient for cell transplantation therapy to use as a cell aggregate for transplantation (also referred to as a transplant) or cells for transplantation or tissue for transplantation.

The effective amount of the active ingredient depends on the purpose of administration, methods of administration, and the condition of the subject (e.g., sex, age, body weight, disease state), and, can be, for example, $1 \times 10^4$ to $1 \times 10^{10}$, $1 \times 10^5$ to $1 \times 10^9$, $1 \times 10^6$ to $1 \times 10^7$, $3 \times 10^6$ to $3 \times 10^7$, or $1 \times 10^6$ to $1 \times 10^9$ in number of cells.

The pharmaceutical composition, or cell aggregate for transplantation, cells for transplantation, or tissue for transplantation (hereinafter, the pharmaceutical composition or the like) may herein contain a pharmaceutically acceptable carrier in addition to an effective amount of the active ingredient. For the pharmaceutically acceptable carrier, physiological aqueous solvent (e.g., physiological saline, buffer, serum-free culture solution) can be used. In transplantation therapy, the pharmaceutical composition or the like may contain, as necessary, a preservative, a stabilizer, a reducing agent, an isotonic agent, and so on that are commonly used for drugs containing a cell aggregate for transplantation or cells for transplantation.

The cell aggregate for transplantation, cells for transplantation, or tissue for transplantation can be produced as a cell suspension by suspending in suitable physiological aqueous solvent. If necessary, the cell population for transplantation may be cryopreserved with addition of a cryopreservative, thawed and washed with buffer before use, or preserved under low-temperature conditions and washed with buffer before use, and used for transplantation therapy.

Specifically, after performing the method in sections 2 for producing a cerebral organoid or any of the methods in the above sections "4" to "7" for producing a cerebral cortical cell aggregate, all the cerebral organoids or cell aggregates are collected, and the cell aggregates collected are washed with the culture medium used, another culture medium, phosphate buffer, or the like, as necessary, and then can be suspended in a medium to be used for the pharmaceutical composition.

The pharmaceutical composition or the like of the present invention may be a suspension in which a cell aggregate has been dispersed and suspended, or a suspension or sheet, for example, in which the cell aggregate has been dispersed into cells.

The pharmaceutical composition or the like obtained by the production method of the present invention can be prepared as a cell tissue structural body as a two-dimensional tissue by performing the method in the above section "2" for producing a cerebral organoid or any of the methods in sections 4 to 7 for producing a cerebral cortical cell aggregate and then forming into a sheet through culture on a plate, or prepared as a cell tissue structural body as a three-dimensional tissue by three-dimensionally forming through culture on a scaffold.

As described later, the pharmaceutical composition or the like of the present invention containing a cell aggregate or cell population including cerebral cortical cells provides a significant effect of repairing the nervous system with injury in an injured site and recovering motor function (e.g., amelioration of symptoms including motor paralysis) through administration to a cerebrovascular disorder model animal (e.g., a mouse). Accordingly, the pharmaceutical composition or the like of the present invention is useful as a therapeutic drug for cerebrovascular disorder or head trauma. In addition, the pharmaceutical composition or the like of the present invention is useful as a motor function-recovering agent for patients affected by cerebrovascular disorder.

Moreover, the cell aggregate or cell population that is contained in the pharmaceutical composition or the like as described herein and includes cerebral cortical cells is produced from established pluripotent stem cells, specified with markers or the like, and quality-controlled. Accordingly, cell populations for transplantation can be mass-produced with stable quality and used for transplantation. Since the cell population for transplantation can be stored, the cell population for transplantation can be prepared according to the date of transplantation for a patient.

11. Therapeutic Method

One embodiment of the present invention includes a therapeutic method for a disease for which supplement or functional recovery of cerebral cortical cells is needed, specifically, cerebrovascular disorder, comprising: transplanting (administering) an effective amount of a cerebral organoid obtained by the method described in the above section "2" for producing a cerebral organoid or the cerebral organoid described in the above section "3", a cell population including cerebral cortical cells obtained from any of the cerebral organoids, a cerebral cortical cell aggregate obtained by any of the methods described in sections 4 to 7 for producing a cerebral cortical cell aggregate or the cerebral cortical cell aggregate described in the above section "8" or "9", a cell population including cerebral cortical cells obtained from any of the cell aggregates, or the pharmaceutical composition described in the above section "10" according to the present invention to a subject in need of transplantation. Here, the site of administration (transplantation) may be the cerebral cortex or the basal ganglion. The subject may be a mammal, and is preferably a rodent (e.g., a mouse, a rat) or a primate (e.g., a human, a simian), and more preferably a human. The cerebrovascular disorder may be head trauma. The concept of the therapeutic method is meant to include a method for recovering motor function (e.g., amelioration of symptoms including motor paralysis) in a patient with cerebrovascular disorder and a method for supplementing with cerebral cortical cells in a patient with cerebrovascular disorder.

While a problem of rejection due to difference in histocompatibility antigens often arises in transplantation therapy, the problem can be overcome by using autologous pluripotent stem cells (e.g., induced pluripotent stem cells) established from somatic cells of a recipient in transplantation. That is, in a preferred embodiment of the present invention, a cell aggregate (cell population) that is immunologically autologous for a recipient is produced by using pluripotent stem cells (e.g., induced pluripotent stem cells) established from somatic cells of the recipient, and the cell aggregate (cell population) or a cell population for transplantation including cells obtained from the cell aggregate (cell population) is transplanted into the recipient.

It is also acceptable that an allogeneic (alien) cell aggregate (cell population) is produced by using pluripotent stem cells (e.g., induced pluripotent stem cells) established from somatic cells of another individual immunologically compatible (e.g., compatible with respect to HLA type or MHC type) with a recipient, and the cell aggregate (cell population) or a cell population for transplantation including cells obtained from the cell aggregate (cell population) is transplanted into the recipient.

Even in allotransplantation of cells, rejection can be avoided with production of the cell aggregate (cell population) of the present invention by using iPS cells in which expression of histocompatibility antigens (e.g., antigen proteins constituting HLA Class I and HLA Class II) or factors necessary for expression of the antigens is suppressed.

The pharmaceutical composition described above can be used as a therapeutic drug that is administered to or transplanted into a patient or a recipient in the therapeutic method of the present invention.

One embodiment of the present invention includes use of the cell aggregate or cell population of the present invention including cerebral cortical cells for use in treating cerebrovascular disorder.

12. Method for Evaluating Toxicity or Drug Efficacy

One embodiment of the present invention includes a method for evaluating a toxicity or drug efficacy of a test substance, comprising contacting the test substance with a cerebral organoid obtained by the method described in the above section "2" for producing a cerebral organoid or the cerebral organoid described in the above section "3" or a cell population including cerebral cortical cells obtained from any of the cerebral organoids, or a cerebral cortical cell aggregate obtained by any of the methods described in sections 4 to 7 for producing a cerebral cortical cell aggregate or the cerebral cortical cell aggregate described in the above section "8" or "9" or a cell population obtained by dispersing any of the cell aggregates, and detecting or quantifying an influence of the test substance on the cell aggregate or the cell population.

A cerebral organoid obtained by the method described in the above section "2" for producing a cerebral organoid or the cerebral organoid described in the above section "3" or a cell population including cerebral cortical cells obtained from any of the cerebral organoids, or a cerebral cortical cell aggregate obtained by any of the methods described in sections 4 to 7 for producing a cerebral cortical cell aggregate or the cerebral cortical cell aggregate described in the above section "8" or "9" or a cell population obtained by dispersing any of the cell aggregates can be used as disease model cells for screening or drug efficacy evaluation for a therapeutic drug for a disease involving cerebrovascular disorder or a prophylactic drug therefor.

A cerebral organoid obtained by the method described in the above section "2" for producing a cerebral organoid or the cerebral organoid described in the above section "3" or a cell population including cerebral cortical cells obtained from any of the cerebral organoids, or a cerebral cortical cell aggregate obtained by any of the methods described in sections 4 to 7 for producing a cerebral cortical cell aggregate or the cerebral cortical cell aggregate described in the above section "8" or "9" or a cell population obtained by dispersing any of the cell aggregates can be used as healthy model cells for safety test, stress test, toxicity test, adverse effect test, or infection or contamination test for chemicals or the like. Since the cell aggregate or cell population of the present invention includes cells of cortical layer V/VI tissue, the cell aggregate or cell population of the present invention can be used even for functional test for nerve tissues with these cells (e.g., cerebral cortex), specifically, functional evaluation for glutamatergic neurons and so on, and evaluation of proliferative capacity or differentiation potential for cerebral cortical cells.

Examples of the evaluation methods include stimulation/toxicity test such as apoptosis evaluation, and test to evaluate the influence of a chemical on differentiation into cerebral cortical cells, and axonal growth ability and firing ability thereof (RT-PCR for various gene markers, analysis of expressed proteins by ELISA or the like for cytokines, phagocytotic ability test, patch-clamp methods, electrophysiological analysis with a multielectrode array (MEA) or the like). For example, the cell aggregate or cell population of the present invention can be used for search for a compound that promotes or inhibits neural differentiation, axonal growth ability, and firing ability, or seek for a compound, protein, or the like that rescues a disease-specific phenotype for cells formed by differentiation of iPS cells derived from a patient affected by cerebrovascular disorder.

For a cell material for these tests, for example, a plate obtained by dispersing the cells of the cell aggregate of the present invention and seeding the cells to adhere, a cell suspension, or a sheet or formed product thereof can be provided.

A cerebral organoid obtained by the method described in the above section "2" for producing a cerebral organoid or the cerebral organoid described in the above section "3" or a cell population including cerebral cortical cells obtained from any of the cerebral organoids, or a cerebral cortical cell aggregate obtained by any of the methods described in sections 4 to 7 for producing a cerebral cortical cell aggregate or the cerebral cortical cell aggregate described in the above section "8" or "9" or a cell population obtained by dispersing any of the cell aggregates according to the present invention can be used for extrapolation test to human or animal test.

13. Quality Assessment Method for Cerebral Organoid or Cerebral Cortical Cell Aggregate (Including High-Purity Cerebral Cortical Cell Aggregate)

One embodiment of the present invention includes a quality assessment method for a cerebral organoid or a cerebral cortical cell aggregate, comprising step (aa) and step (bb) below. Here, the high-purity cerebral cortical cell aggregate of the present invention is included in the definition of the cerebral cortical cell aggregate.

(aa) A step of measuring the expression level(s) of at least one or all genes selected from the group consisting of GAD2, COL1A1, TYR, TTR, and HOXA2 in a cerebral organoid or a cerebral cortical cell aggregate; and
(bb) a step of determining with reference to a measurement result in step (aa) that the amount of non-target cells included in the cerebral organoid or the cerebral cortical cell aggregate is equal to or less than a reference value if the expression levels of the genes are each equal to or less than a reference value.

One embodiment of the present invention includes a quality assessment method for a cerebral organoid or a cerebral cortical cell aggregate, comprising the following step (AA) and step (BB).

(AA) A step of measuring the expression level(s) of at least one, at least two, at least three, or at least five genes selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3 in a cerebral organoid or a cerebral cortical cell aggregate; and
(BB) a step of determining with reference to a measurement result in step (AA) that the amount of target cells included in the cerebral organoid or the cerebral cortical cell aggregate is equal to or more than a reference value if the expression levels of the genes are each equal to or more than a reference value.

The expression levels of the genes (i.e., the expression levels of mRNA or proteins) and measurement method therefor are as described above. Examples of reference values for the expression levels of genes that serve as a marker for non-target cells such as the genes shown in (aa) above include a value determined for an authentic sample of a cerebral organoid or cell aggregate of the cerebral cortex, and evaluation can be performed on the basis of, as a criterion, whether a value is comparable to or less than a reference value. Examples of reference values for the expression levels of genes that serve as a marker for target cells such as the genes shown in (AA) above include a value determined for an authentic sample of a cerebral organoid or cell aggregate of the cerebral cortex, and evaluation can be performed on the basis of, as a criterion, whether a value is comparable to or more than a reference value.

In one embodiment of the present invention, a part of a cell population of cerebral cortical cell aggregates obtained by any of the methods described in sections 4 to 7 for producing a cerebral cortical cell aggregate is sampled and subjected to evaluation by the quality assessment method for a cerebral organoid or a cerebral cortical cell aggregate, and, with reference to the evaluation result, a population of cerebral cortical cell aggregates for which the amount of non-target cells is equal to or less than a reference value and/or the amount of target cells is equal to or more than a reference value can be selected (identified) as a population applicable to transplantation.

Examples of the methods described in sections 4 to 7 for producing a cerebral cortical cell aggregate as an embodiment of the present invention include a method for producing a cerebral cortical cell aggregate, further comprising the quality assessment method for a cerebral organoid or a cerebral cortical cell aggregate.

Specific examples thereof include a method for producing a cerebral cortical cell aggregate, comprising the following steps:

(1) a step of producing a cerebral cortical cell aggregate by any of the production methods described in sections 4 to 7;
(2) a step of determining by the quality assessment method whether the amount of target cells is equal to or more than a reference value and/or the amount of non-target cells is equal to or less than a reference value; and
(3) a step of selecting or identifying a cerebral cortical cell aggregate applicable to transplantation with reference to the evaluation result in (2).

Examples

(Maintenance Culture of Human Induced Pluripotent Stem Cells (hiPSC))

hiPSCs were subjected to maintenance culture with StemFit (R) AK02N culture medium or AK03N culture medium (hereinafter, occasionally referred to as "StemFit"; manufactured by Ajinomoto Healthy Supply Co., Inc.) on a 6-well plate coated with iMatrix-511 (Nippi, Incorporated), which is an E8 fragment of laminin 511, wherein the plate was prepared by adding iMatrix-511 at 0.5 $\mu$g/cm$^2$. For passage, the hiPSCs were treated with 0.5 $\times$ Tryple Select at 37°C for 8 minutes to separate the cells into single cells, which were then seeded on a 6-well plate at a cell density of 1 to 1.5 $\times$ 10$^4$ cells. Passage was performed every 7 days.

(Immunostaining Analysis)

Organoids were fixed with 4% paraformaldehyde for 30 minutes, subjected to dehydration reaction with 30% (w/v) sucrose in PBS, and embedded with O.C.T. compound (Sakura Finetek Japan Co., Ltd.). Frozen sections were prepared in a thickness of 16 $\mu$m by using a cryostat (CM1850, Leica Biosystems Nussloch GmbH). Permeabilization was performed with 0.3% or 2% (v/v) Triton-X100, and, as necessary, antigen retrieval reaction was performed. Blocking was performed with Block ACE (KAC Co., Ltd.), and double- or triple-label staining was then performed. Primary antibodies and secondary antibodies for use in immunostaining were diluted to recommended concentrations before use.

The primary antibodies and secondary antibodies used in the immunostaining are shown in the following.

L1CAM: 554273 (BD)
Ctip2: ab18465 (Abeam)
Pax6: EPR15858 (Abeam), 561482 (BD)
Foxg1: M227 (Takara)
Ki67: NCL (Leica)
Emx1: M196 (Takara)
Gad65: 559931 (BD)
Col1a1: AF6220 (R&D)
TTR: A0002 (DAKO)
TYR: MA5-14177 (Thermo Fisher)
Alexa Fluor® 488 donkey anti-mouse IgG (H+L): A21202 (Thermo Fisher)
Alexa Fluor® 647 donkey anti-mouse IgG (H+L): 1900251 (Thermo Fisher)
Alexa Fluor® 594 donkey anti-rat IgG (H+L): 1979379 (Thermo Fisher) Alexa Fluor® 488 donkey anti-rabbit IgG (H+L): A21206 (Thermo Fisher)
Alexa Fluor® 647 donkey anti-rabbit IgG (H+L): A32795 (Thermo Fisher)
Alexa Fluor® 594 donkey anti-sheep IgG (H+L): A11016 (Thermo Fisher)

(Microscopic and Image Analyses)

Images (bright field images) of organoids during culture were acquired by photographing with a digital inverted microscope (Leica Biosystems Nussloch GmbH, DMS1000). Confocal fluorescence microscopy images were acquired by photographing with the confocal microscope LSM800 (Carl Zeiss AG), and analyzed with the image processing software ZENBlue.

(Flow Cytometry)

Cerebral organoids, cerebral cortical cell aggregates, and high-purity cerebral cortical cell aggregates were dispersed into single cells by using dispersing solution for neural cells (FUJIFILM Wako Pure Chemical Corporation), fixed by using Fixation Buffer (BD Biosciences) at 4°C for 30 minutes, then treated with Perm/Wash buffer (BD Biosciences) at room temperature for 15 minutes, subjected to double or triple staining, and analyzed by using an Aria III. The analysis software used was FACSDiva software (BD). The primary antibodies and secondary antibodies used are shown in the following.

PerCP-Cy5.5 Mouse IgG1 k isotype control: 550795 (BD Biosciences)
Alexa 647 Mouse Anti-human Oct3/4 antigen: 560329 (BD Biosciences)
Tra-2-49/6E-FITC antibody: FCMAB133F (Merck)
Alexa647 mouse anti β-tubulin Class III: 560394 (BD)
PerCP-Cy5.5 Mouse anti-Human Sox1: 561549 (BD)
Alexa647 mouse anti-Human Pax6: 561165(BD)
Alexa488 mouse anti-Ki67: 562249 (BD)
Alexa488 rat anti-Ctip2 antibody: ab123449 (Abeam)

(Gene Expression Analysis by Quantitative Reverse Transcription-PCR (RT-qPCR))

Total RNA was obtained with an RNeasyMicroKit (QIAGEN), and reverse transcription reaction was performed with a SuperScript III First-Strand Synthesis System (QIAGEN) in accordance with a protocol from the provider. Quantitative PCR was carried out by using TaqMan (TM) Gene Expression Master Mix (Thermo Fisher Scientific Inc.) or SYBR Green Master Mix (Thermo Fisher Scientific Inc.) in accordance with instruction from the manufacturer. The expression levels of genes were normalized to that for GAPDH by using the ΔΔCt method.

The primer sets and Taqman Probes used are shown in the following.

Oligo DNA primers

POU5F1:

|  |  |
|---|---|
| Forward: | AGACCATCTGCCGCTTTGAG (SEQ ID No. 1) |
| Reverse: | GCAAGGGCCGCAGCTT (SEQ ID No. 2) |

NANOG:

|  |  |
|---|---|
| Forward: | GGCTCTGTTTTGCTATATCCCCTAA (SEQ ID No. 3) |
| Reverse: | CATTACGATGCAGCAAATACGAGA (SEQ ID No. 4) |

BMP4:

|  |  |
|---|---|
| Forward: | ATGATTCCTGGTAACCGAATGC (SEQ ID No. 5) |
| Reverse: | CCCCGTCTCAGGTATCAAACT (SEQ ID No. 6) |

NODAL :

|  |  |
|---|---|
| Forward: | TGAGCCAACAAGAGGATCTG (SEQ ID No. 7) |
| Reverse: | TGGAAAATCTCAATGGCAAG (SEQ ID No. 8) |

TGFB1:

|  |  |
|---|---|
| Forward: | TACCTGAACCCGTGTTGCTCTC (SEQ ID No. 9) |
| Reverse: | GTTGCTGAGGTATCGCCAGGAA (SEQ ID No. 10) |

SOX1:

|  |  |
|---|---|
| Forward: | GCGGAGCTCGTCGCATT (SEQ ID No. 11) |
| Reverse: | GCGGTAACAACTACAAAAAACTTGTAA (SEQ ID No 12) |

PAX6:

|  |  |
|---|---|
| Forward: | CTGGCTAGCGAAAAGCAACAG (SEQ ID No. 13) |
| Reverse: | CCCGTTCAACATCCTTAGTTTATCA (SEQ ID No. 14) |

T (TBXT):

|  |  |
|---|---|
| Forward: | ATGGAGGAACCCGGAGACA (SEQ ID No. 15) |
| Reverse: | TGAGGATTTGCAGGTGGACA (SEQ ID No. 16) |

SOX17:

|  |  |
|---|---|
| Forward: | CGCTTTCATGGTGTGGGCTAAGGACG (SEQ ID No. 17) |
| Reverse: | TAGTTGGGGTGGTCCTGCATGTGCTG (SEQ ID No. 18) |

hCGalpha:

|  |  |
|---|---|
| Forward: | ACCGCCCTGAACACATCCTGC (SEQ ID No. 19) |
| Reverse: | GCGTGCATTCTGGGCAATCCTGC (SEQ ID No. 20) |

SOX2:

Forward:     GCCGAGTGGAAACTTTTGTCG (SEQ ID No. 21)

Reverse:     GGCAGCGTGTACTTATCCTTCT (SEQ ID No. 22)

Taqman probe

SLC17A7:    Hs01574213

EMX1:    Hs00417957

GAD2:    Hs00609536

DLX2:    Hs00269993

TTR:    Hs00174914

COL1A1:    Hs00164004

TYR:    Hs00165976

HOXA2:    Hs00534579

<Preliminary Test 1> Induction of Differentiation of Pluripotent Stem Cells Subjected to Maintenance Culture in Presence or Absence of Sustentacular Cells into Neural Cells

Human embryonic stem cells (hESC) or human iPS cell lines (hiPSC) were induced to differentiate into neural cells in the presence of sustentacular cells (on feeder) or in the absence thereof (feeder-free), and spherical cell aggregates generated were analyzed to compare the efficiencies of induction of differentiation into organoids.

KhES-1 and KhES-2, which are each human ES, were obtained from Institute for Frontier Life and Medical Sciences, Kyoto University, and 201B7 cells, 1231A3 cells, and Ff-I01s04 cells, which are human iPS cell lines, were obtained from Kyoto University.

In on-feeder cases, mouse embryonic fibroblasts (MEF, Oriental Yeast Co., Ltd.) were used as sustentacular cells. Stem cell lines including KhES-1 were seeded at a density of $1.2 \times 10^6$ cells per 90-mm dish, and cultured with a culture medium prepared by adding 5 ng/ml bFGF to DMEM/F12 culture medium containing 1% (v/v) NEAA, 1% (v/v) L-glutamine, 1% (v/v) 2-mercaptoethanol, and 20% (v/v) KSR in the presence of MEFs at 37°C under 5% $CO_2$ for 3 to 7 days. In feeder-free cases, stem cell lines were subjected to maintenance culture with StemFit (manufactured by Ajinomoto Healthy Supply Co., Inc.) on a 6-well plate coated with iMatrix-511 (manufactured by Nippi, Incorporated) as a matrix at 37°C under 5% $CO_2$ for 7 days.

The cells after the maintenance culture were induced to differentiate into cerebral organoids by a method described in Non Patent Literature 1 with partial modification. At the initiation of differentiation, the pluripotent stem cells were treated with $0.5 \times$ TrypLE Select to disperse into single cells. Thereafter, the culture medium was replaced with differentiation culture medium (described below) supplemented with 50 $\mu$M Y-27632 (FUJIFILM Wako Pure Chemical Corporation), and the cells were seeded on a non-cell-adsorbable V-bottom 96-well plate (Sumitomo Bakelite Co., Ltd.) at 9,000 cells/well. The differentiation culture medium was DMEM/F-12 GlutaMAX culture medium (Gibco) supplemented with 20% (v/v) KSR, 5 $\mu$M SB431542 (TGF$\beta$ inhibitor; Tocris Bioscience), and 3 $\mu$M IWR1e (Wnt inhibitor; Calbiochem). From day 3 to day 15 of differentiation, half-volume culture medium exchange was performed the with differentiation culture medium every 3 days. On day 18, aggregated cells were transferred into a 90-mm suspension culture dish (Sumitomo Bakelite Co., Ltd.), and further cultured with DMEM/F-12 GlutaMAX (Gibco) supplemented with 1% (v/v) N2-supplement (Gibco), 1% (v/v) chemically defined lipid concentrate (CDLC; Gibco), 0.25 $\mu$g/ml amphotericin B (Gibco), 100 U/ml penicillin, and 100 $\mu$g/ml streptomycin until day 35 after the initiation of induction of differentiation (Day 35). Until Day 35, whole-volume culture medium exchange was performed every 3 days.

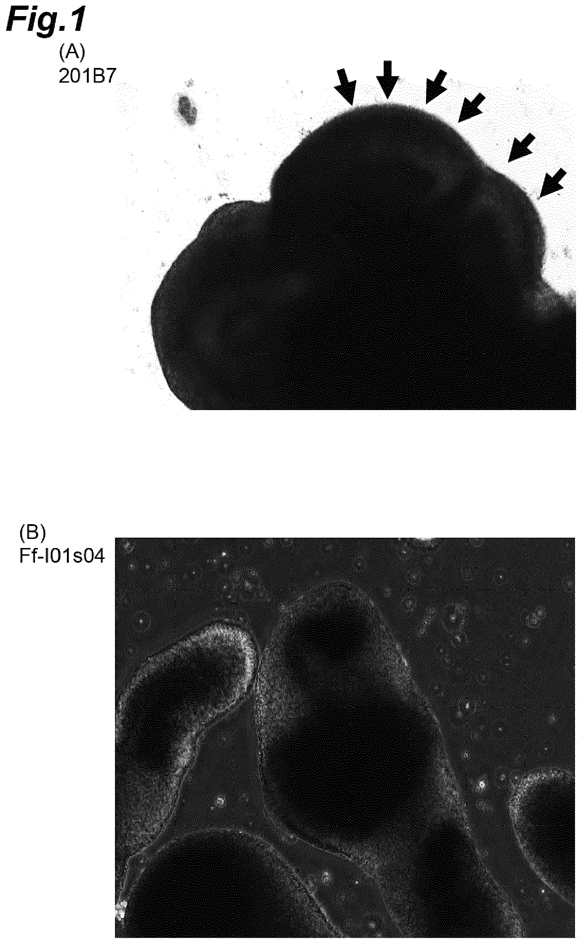

For spherical cell aggregates obtained on day 33 to 35 after induction of differentiation (Day 33 to 35), cases with the numerical proportion of cerebral organoids being approximately 5% or more were determined as "cerebral organoids were formed (good organoid)", and cases with the numerical proportion of cerebral organoids being less than 5% were determined as "cerebral organoids were unformed (bad organoid)". An example of good organoid is shown in Figure 1(A) (201B7 strain), and an example of bad organoid is shown in Figure 1(B) (Ff-I01s04 strain). Arrows in (A) indicate rosette structure. Table 1 summarizes results for the different cell lines.

[Table 1]

| Matrix | Cell line | MEF | iMatrix-511 (LM511E8) |
|---|---|---|---|
| Culture medium | | hESC | StemFit |
| hESC | KhES-1 | 3/3 | 1/3 |
| | KhES-2 | 3/5 | 0/3 |
| hiPSC | 201B7 | 3/3 | 1/3 |
| | 1231A3 | 0/3 | 0/4 |
| | Ff-I01s04 | 3/3 | 0/4 |

For the numbers in the table, each denominator indicates the number of experiments in which induction of differentiation was performed, and each numerator indicates the number of the occurrence of the formation of a cerebral organoid. For example, "1/3" means that induction of differentiation was performed three times and the formation of a cerebral organoid occurred once. It was confirmed for all the ES and iPS cell lines that the efficiency of cerebral organoid formation was lower in feeder-free cases than in on-feeder cases.

<Preliminary Test 2> Analysis of Gene Expression

In the same manner as in Preliminary Test 1, the cell lines KhES-1, 201B7, Ff-I01s04, and 1231A3 were cultured under three different conditions: on feeder, feeder-free, and with feeder conditioned medium, and induced to differentiate. For the conditions with feeder conditioned medium, MEFs were cultured with DMEM/F12 culture medium containing 1% (v/v) NEAA, 1% (v/v) L-glutamine, 1% (v/v) 2-mercaptoethanol, and 20% (v/v) KSR for 24 hours, the supernatant of the culture medium was collected, and subjected to filtration for use in feeder-free culture of cells. Expression of FGF2 and TGFβ pathway-related genes (FGF2, LEFTY, NODAL, TGFβ1, activin) in those resulting cells before post-culture induction of differentiation and after the induction of differentiation was analyzed with microarrays.

In the microarray analysis, RNA was extracted from four iPS cell lines (KhES1, 201B7, 1231A3, Ff-I01s04) cultured under three different conditions: on feeder, feeder-free, and with MEF conditioned medium by using NucleoSpin RNA Plus XS from Takara Bio Inc. in accordance with a predetermined protocol, and gene expression analysis was carried out by using the microarray Clariom S (Applied Biosystems). Analysis of gene expression levels was performed with Transcriptome viewer (Kurabo Industries Ltd.).

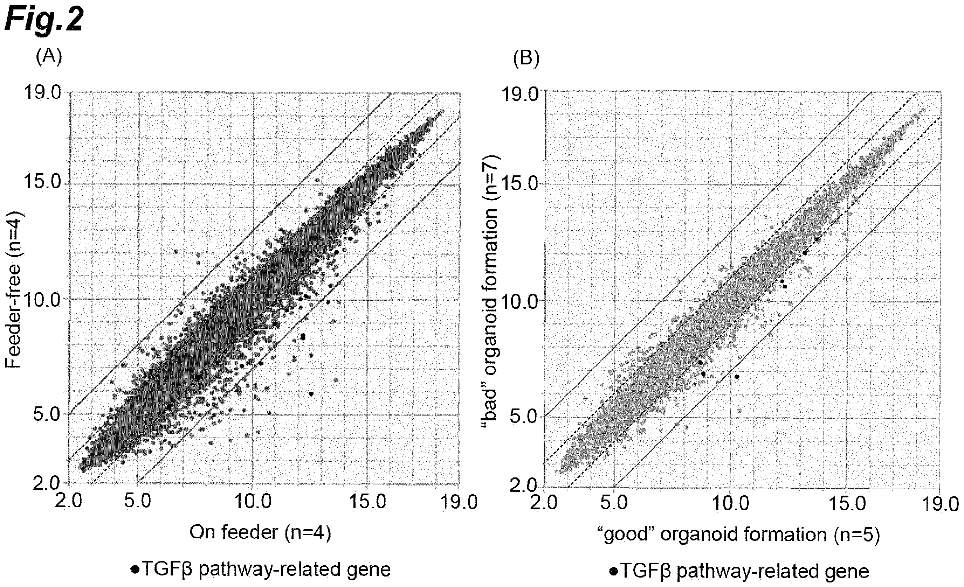

The analysis results are shown in Figure 2. Figure 2(A) shows the results before post-culture induction of differentiation, as results of comparative analysis with the microarray for a group of the four cell lines cultured under the on-feeder conditions (abscissa) and a group thereof cultured under the feeder-free conditions (ordinate) (n indicates the number of cell line types). The on-feeder culture group exhibited values of expression of FGF2 and TGFβ pathway-related genes (FGF2, LEFTY, NODAL, TGFβ1, activin) twice or higher than those for the feeder-free culture group.

Figure 2(B) shows results of comparative analysis of gene expression between two groups, wherein those four cell lines were induced to differentiate according to Preliminary Test 1 under the three different conditions: on feeder, feeder-free, and feeder conditioned medium, and divided into the two groups: a group under conditions that resulted in the formation of a cerebral organoid (good organoid; abscissa) and a group under conditions that did not result in the formation of a cerebral organoid (bad organoid; ordinate) (n indicates the number of conditions that resulted in high efficiency of cerebral organoid formation). The group with the formation of a cerebral organoid exhibited higher values of expression of FGF2 and TGFβ pathway-related genes (FGF2, LEFTY, NODAL, TGFβ1, activin) than the group without the formation. It was found that undifferentiated stem cells subjected to on-feeder maintenance culture and those with high efficiency of cerebral organoid formation exhibited high expression of endogenous bFGF and TGFβ-related genes. That is, it was revealed that ES/iPS cells and culture conditions that allow easy formation of a cerebral organoid result in high expression levels of bFGF and TGFβ-related genes in pluripotent stem cells.

Table 2 below shows the compositions of known maintenance culture media for human pluripotent stem cells. It is understood that bFGF and TGFβ are typically added at high concentrations to maintenance culture media for feeder-free culture. It was suggested that ES/iPS cells cultured with such culture media would exhibit lower expression of bFGF and TGFβ-related genes than those cultured on-feeder conditions. Accordingly, it was expected that the difference in the expression levels of bFGF and TGFβ-related genes was a possible cause for the difference in cerebral organoid formation efficiency.

[Table 2]

| | on feeder | feeder-free | | |
|---|---|---|---|---|
| | DMEM/F-12 GlutaMAX (Gibco) | Essential 8 (Thermo) | mTeSR1 (STEMCELL) | StemFit (Ajinomoto) |
| Additive | 20% KSR NEAA L-glutamine 2-ME | 2ng/mL TGFβ | 23.5pM TGFβ | non-disclosed |
| bFGF | 5 ng/mL | 10 ng/mL | 100 ng/mL | non-disclosed |
| Matrix | MEF | Matrigel | Geltrex Matrigel Vitronectin | iMatrix-511 (LMS 11-E8) |

In view of that, the present inventors cultured ES/iPS cells in the presence of various compounds and then induced the cells to differentiate into neural cells, and analyzed the efficiencies of cerebral organoid formation to search for compounds that can have influence on the formation efficiency. The results surprisingly found that significantly enhanced efficiency of cerebral organoid formation resulted when iPS cells were cultured with a "culture medium containing no bFGF, and containing no TGFβ or supplemented with a TGFβ signaling inhibitor" (step (1)) and then induced to differentiate into neural cells (step (2)).

<Example 1> Influence of Step (1) On Organoid Formation Efficiency

1-1. Maintenance Culture Medium in Step (1)

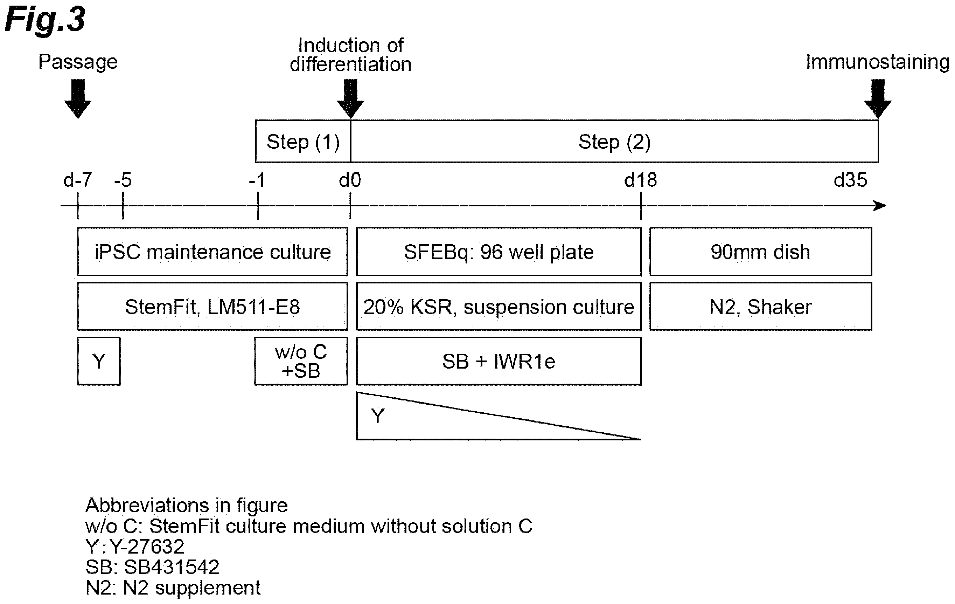

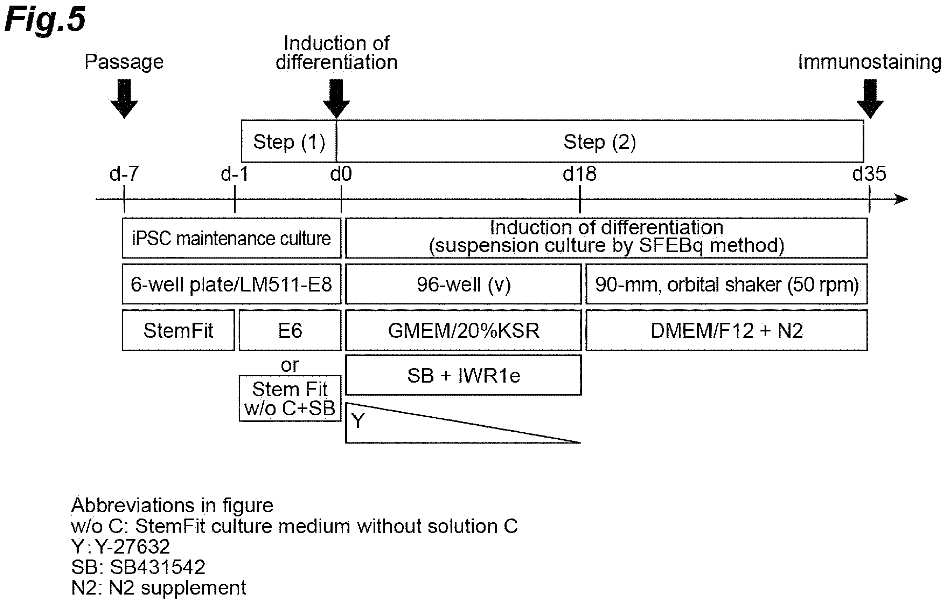

Step (1) and step (2) were performed according to a scheme illustrated in Figure 3 or Figure 5. Maintenance culture of human iPS cells (S2WCB1 strain, S2WCB3 strain) was performed in StemFit culture medium on a 6-well plate coated with iMatrix-511 (feeder-free).

The culture medium was exchanged with:

(1) a culture medium obtained by adding 5 μM SB431542 to StemFit culture medium without solution C (i.e., a bFGF-free culture medium) (Figure 3); or
(2) Essential 6 culture medium (i.e., a bFGF-free, TGFβ-free culture medium) (Figure 5),
and adhesion culture was performed for 1 day.

Step (2a) Induction of differentiation into neural cells was performed in a serum-free culture medium by using a method by Sakaguchi et al. (Stem Cell Reports, 13:458-473, 2019. doi: 10.1016/j.stemcr.2019.05.029.). Specifically, human iPS cells cultured in step (1) were dispersed into single cells by enzymatic treatment. The human iPS cells dispersed into single cells were seeded on a non-cell-adhesive 96-well culture plate (PrimeSurface 96 V-bottom plate, manufactured by Sumitomo Bakelite Co., Ltd.) (9,000 cells/well/100 μL), and subjected to suspension culture with Glasgow MEM (GMEM; Thermo Fisher Scientific Inc.) culture medium supplemented with 0.1 mM non-essential amino acids, 1 mM pyruvate, 0.1 mM 2-mercaptoethanol, 20% (v/v) KnockOut (TM) serum replacement, 100 U/mL penicillin, and 100 μg/ml streptomycin (hereinafter, also referred to as 20GMK) with further addition of 5 μM SB431542 (TGFβ signaling inhibitor; Tocris Bioscience) and 3 μM IWR1e (Wnt signaling inhibitor; Calbiochem), at 37°C under 5% $CO_2$ for 18 days. Culture medium exchange was performed every 3 days. Y-27632 was added to reach 50 μM on Day 0, and not added in the subsequent culture medium exchanges. Through the culture, cell aggregates were eventually obtained.

Step (2b) On Day 18, the cell aggregates obtained were transferred into a 90-mm dish, and subjected to shaking culture with an Orbital Shaker in DMEM/F12/GlutaMAX culture medium supplemented with 1 × N2 supplement (Thermo Fisher Scientific Inc.), a chemically defined lipid concentrate (Invitrogen), 0.25 mg/mL fungizone (Gibco), 1% penicillin/streptomycin, and 0.1% amphotericin B at 37°C under 20% $O_2$ and 5% $CO_2$ from Day 35 to Day 42. Culture medium exchange was performed every 3 to 4 days.

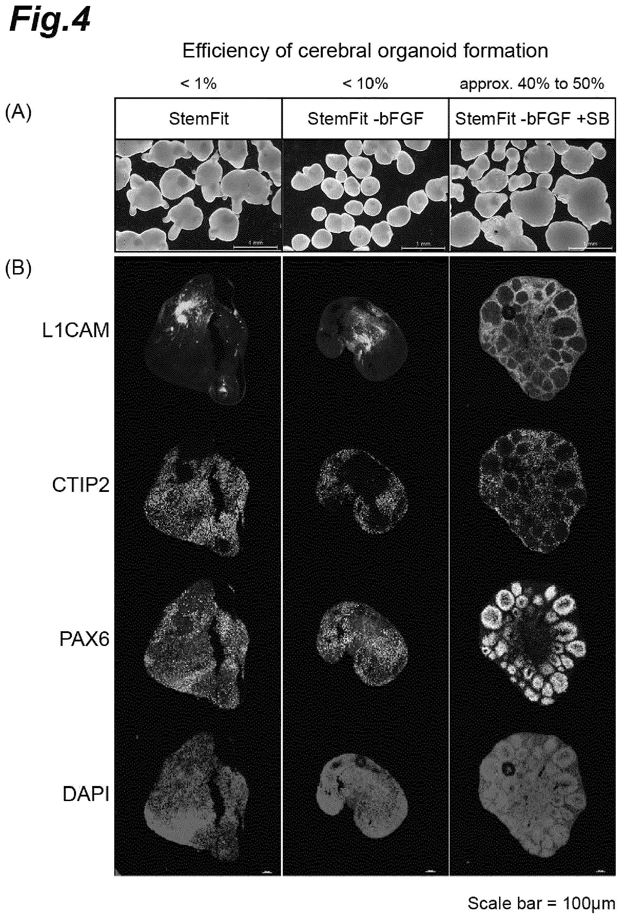

Cell cultures on Day 35 were observed with an inverted microscope; Figure 4(A) shows the representative images. For the cell cultures, immunofluorescence staining was performed on different markers (L1CAM, CTIP2, PAX6). In the immunofluorescence staining, the above primary antibodies and fluorescence-labeled secondary antibodies corresponding to them were used. In addition, nuclear staining was performed with DAPI. The representative fluorescence staining images (confocal fluorescence microscopy images) are shown in Figure 4(B).

Efficiency (%) of cerebral organoid formation was calculated by the following expression.

[Expression 1]

Efficiency of cerebral organoid formation (%) = [Number of cerebral organoids / number of spherical cell aggregates] × 100

In maintenance culture with bFGF-free StemFit culture medium supplemented with SB431542 (hereinafter, occasionally referred to as SB) (StemFit -bFGF +SB), a cell aggregate of clear, dense rosette structure (rightmost in (B)) was confirmed. A Pax6-positive neural progenitor cell layer (radial glial cells) was localized in the inner side of the cerebral cortex-like structure, and a neural cell layer expressing Ctip2 and L1CAM was found in the outer side. When maintenance culture (step (1)) was performed with bFGF-free culture medium ("StemFit - bFGF" or "StemFit -bFGF +SB"), significantly higher efficiency of cerebral organoid formation (<10% for "StemFit -bFGF", approximately 40% to 50% for "StemFit -bFGF +SB") resulted than in maintenance culture with bFGF-containing culture medium (StemFit, <1%).

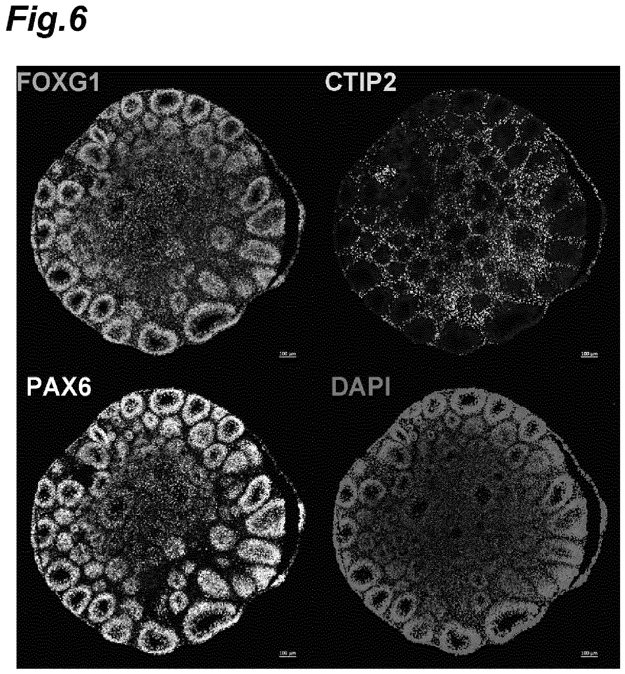

Maintenance culture was performed with use of Essential 6 (E6) culture medium supplemented with SB431542 as a culture medium in step (1), and induction of differentiation was performed in the same manner as in the above. Immunostaining was performed in the same manner as above; Figure 6 shows confocal fluorescence microscopy images of a representative cell aggregate. As can be seen from Figure 6, a cerebral organoid was obtained when E6 culture medium supplemented with SB431542 was used as with the case with StemFit culture medium supplemented with SB431542. Efficiency of cerebral organoid formation (%) = (22/39) × 100% = 56.9%.

1-2. Additives for Maintenance Culture Medium in Step (1)

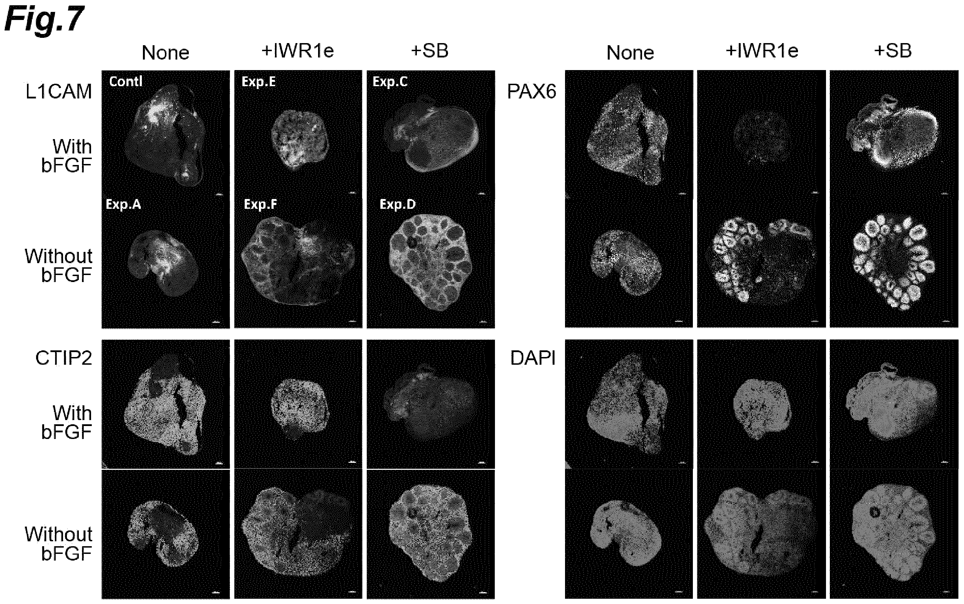

In step (1) of the scheme in Figure 3, culture was performed with a culture medium supplemented with any additive below during a period of -Day 1 to Day 0, induction of differentiation into neural cells was performed on Day 0, and the efficiencies of cerebral organoid formation were evaluated on Day 35. Figure 7 shows results of immunostaining, and Table 3 shows the efficiencies of cerebral organoid formation. In Figure 7, "None" indicates the absence of an additive.

[Table 3]

| Experimental group (Exp.) | Conditions | | Result |
| | Step (1) | | Efficiency (%) of cerebral organoid formation |
| | bFGF | Additive | |
| Control | + | - | <1% |
| A | - | - | <10% |
| B | - | LDN-193189 | <5% |
| C | + | SB-431542 | <5% |
| D | | SB-431542 | approx. 40% to 50% |
| E | + | IWR1e | <1% |
| F | - | IWR1e | <5% |
| G | - | LDN+SAG | <1% |
| H | - | BMP4, TGFβ, Nodal, Activin | <1% |
| I | + | BMP4, TGFβ, Nodal, Activin | <1% |

Control, Exp. C, E, I: culture medium obtained by adding additive to culture medium supplemented with solution C of StemFit (= bFGF-containing culture medium)
Exp. A-B, D, F-H: culture medium obtained by adding additive to culture medium not supplemented with solution C of StemFit (= bFGF-free culture medium)

The experimental groups with use of the bFGF-containing culture medium exhibited low cerebral organoid formation efficiency irrespective of the type of an additive. Even the group with addition of SB431542 exhibited a low value less than 5% (Exp. C). For the groups with use of the bFGF-free culture medium, by contrast, only the group with addition of SB431542 gave cerebral organoids of a plurality of rosette structures with high efficiency (Exp. D). For the group with addition of IWR1e, an abnormal cell culture in which a neuroepithelium and neural cells were generated but no cerebral cortex-like structure was formed was found (Exp. F). In the inner side of the cerebral cortex-like structure observed for Exp. D, a Pax6-positive neural progenitor cell layer (radial glial cells) was localized, and a neural cell layer expressing Ctip2 and L1CAM was found in the outer side.

It was demonstrated by the results in Example 1 that significantly enhanced efficiency of cerebral organoid formation results if human pluripotent stem cells are cultured with a "culture medium containing no bFGF, and containing no TGFβ or supplemented with a TGFβ signaling inhibitor" and then induced to differentiate into neural cells. Thus, it was revealed that significantly enhanced efficiency of cerebral organoid formation results if pluripotent stem cells are cultured in a "culture solution that is substantially free of bFGF and provokes substantially no TGFβ signal" and then induced to differentiate into neural cells.

<Example 2> Culture Period in Step (1)

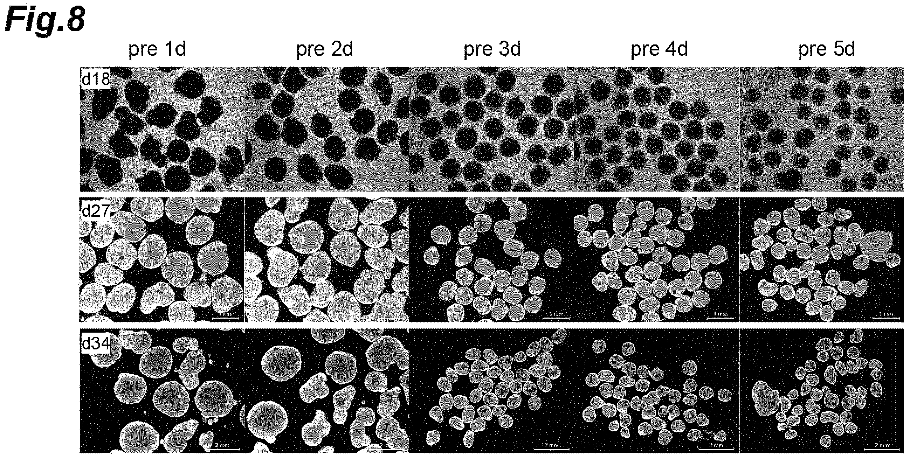

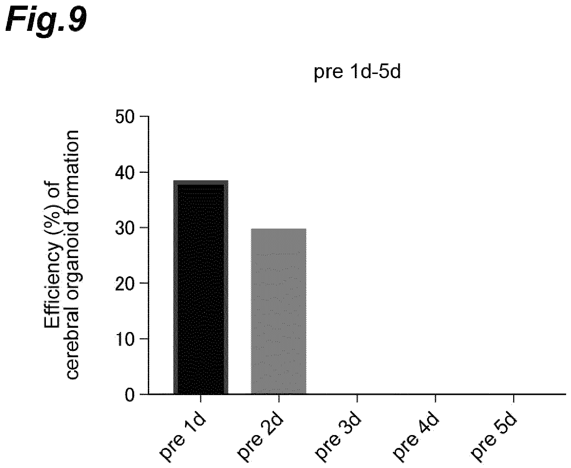

Under the experimental system with (1) "bFGF-free-and-SB431542-supplemented culture medium", which was obtained by adding 5 μM SB431542 to StemFit culture medium without solution C, in Example 1, periods for culture of iPS cells with "bFGF-free-and-SB431542-supplemented culture medium" were set as in a table below. Figure 8 shows representative bright field images of cultures on Day 18, Day 27, and Day 34. Figure 9 shows results of analysis of the efficiencies of cerebral organoid formation on Day 35.

[Table 4]

| Conditions | Day -6 | Day -5 | Day -4 | Day -3 | Day -2 | Day -1 | Day 0 | Day 3-18 | Day 18-35 |
|---|---|---|---|---|---|---|---|---|---|
| pre 1d | StemFit | | | | | StemFit w/o C SB 5μM | 20GMK SB 5μM IWR1e 3μM Y | 20GMK SB 5μM IWR1e 3μM | DMEM/ F12 N2 |
| pre 2d | StemFit | | | | StemFit w/o C SB 5μM | | | | |
| pre 3d | StemFit | | | StemFit w/o C SB 5μM | | | | | |
| pre 4d | StemFit | | StemFit w/o C SB 5μM | | | | | | |
| pre 5d | Stem Fit | StemFit w/o C SB 5μM | | | | | | | |
| pre 6d | StemFit w/o C SB 5μM | | | | | | | | |
| StemFit w/o C: culture medium not supplemented with solution C of StemFit (= bFGF-free culture medium) | | | | | | | | | |

As shown in Figure 8 and Figure 9, treatment with "bFGF-free- and-SB431542-supplemented culture medium" for 1 day or 2 days resulted in an efficiency of cerebral organoid formation of about 30% to 40%, whereas almost no formation of a cerebral organoid was found for such treatment for 3 days or more. The conditions involving treatment with "bFGF-free-and-SB431542-supplemented culture medium" for 6 days resulted in significantly reduced proliferation of iPS cells, leading to failure in obtaining a sufficient number of cells for induction of differentiation. Thus, it was revealed that the culture period in step (1) is preferably less than 3 days, more preferably 12 hours or more and 2 days or less, and most preferably about 1 day.

<Example 3> Influence of Step (1) on Gene Expression

Under the experimental system with (1) "bFGF-free-and-SB431542-supplemented culture medium", which was obtained by adding 5 μM SB431542 to StemFit culture medium without solution C, in Example 1, gene expression analysis was performed for human iPS cells (S2WCB1) on Day 0. Control was iPS cells cultured with StemFit culture medium (bFGF-containing culture medium), which is a common maintenance culture medium.

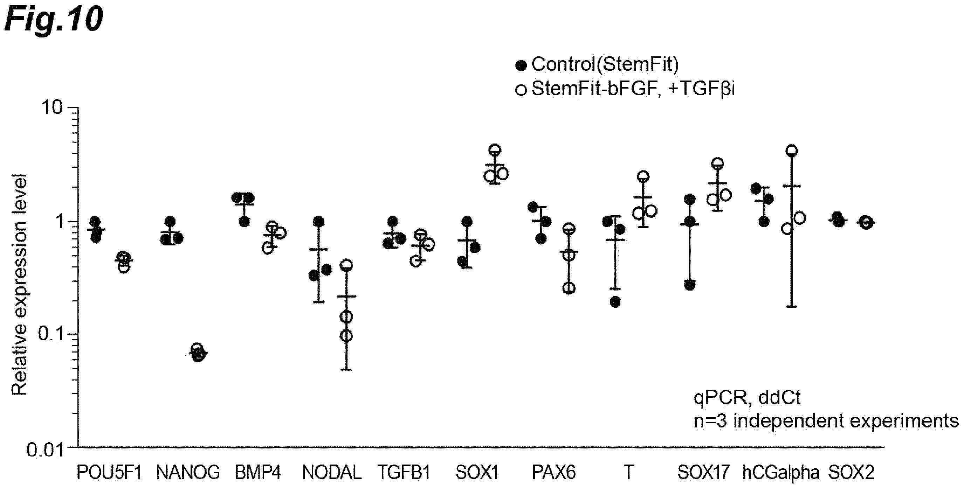

Organoids obtained through three operations of induction of differentiation were analyzed for expression of different marker genes by RT-qPCR. Results of the gene analysis are shown in Figure 10. Each point corresponds to a sample in an operation, solid circles correspond to organoids formed through induction of differentiation of iPS cells cultured with StemFit culture medium (bFGF-containing culture medium), which is a common maintenance culture medium, and open circles correspond to organoids formed through culture with StemFit (-bFGF, +TGFβ inhibitor) followed by induction of differentiation. The ordinate represents relative expression levels calculated by normalizing to GAPDH and one lot of Control by the ΔΔCt method. "TGFβi" in the figure means a TGFβ inhibitor.

It was found from Figure 10 that iPS cells that had undergone step (1) (for 1 day) (open circles) exhibited lower expression levels of undifferentiation markers (POU5F1, NANOG), and higher expression levels of neuroectodermal markers (SOX1, PAX6), a mesodermal marker (TBXT), and an endodermal marker (SOX17) than Control (solid circles). "T" in the figure means TBXT (also known as BRACHYURY).

Thus, it was suggested that iPS cells possibly reach a state with a tendency to differentiate into triploblasts through step (1). Specifically, it was suggested that pluripotent stem cells grow to a state with a tendency to differentiate into triploblasts through culture in a "culture solution that is substantially free of bFGF and provokes substantially no TGFβ signal" for a proper period.

<Example 4> Effect of Steps (ii) to (iv) in Step (2) - Part 1: Increase in Number of Layer V/VI Neural Cells and Decrease in Number of Proliferative Cells

4-1. With Dispersion and Reaggregation

Culture was performed in the same manner as in Example 1 from -Day 7 to Day 35. For step (1), (1) a culture medium obtained by adding 5 μM SB431542 to StemFit culture medium without solution C (bFGF-free culture medium) was used.

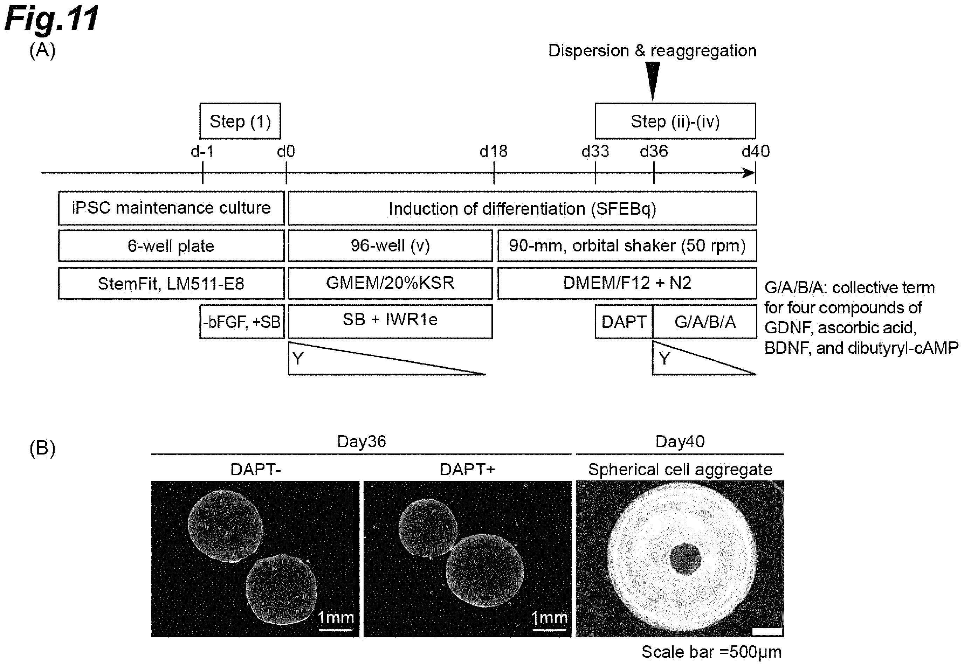

As illustrated by a scheme shown in Figure 11(A), in step (ii), cerebral organoids were screened on day 33 after the initiation of induction of differentiation (Day 33), the culture medium was replaced with one obtained by adding 10 μM DAPT to the culture medium in step (2b), and suspension culture was performed at 37°C under 5% $CO_2$ for 3 days. As a result, cerebral cortical cell aggregates were obtained.

In step (iii), the cerebral cortical cell aggregates obtained in step (ii) were incubated in 0.5 × TrypLE Select, 0.25 mM EDTA solution at 37°C for 20 minutes, and further incubated in a solution obtained by adding 25 U/mL DNase I to the culture medium in step (2b) at 37°C for 10 minutes. Thereafter, pipetting was performed to disperse into single cells, and the dispersed cells were then suspended in a culture medium obtained by adding 10 ng/mL GDNF, 20 ng/mL BDNF, 200 μM ascorbic acid, 400 μM dibutyryl-cAMP, and 50 μM Y-27632 to the culture medium in step (2b).

In step (iv), the cell suspension obtained in step (iii) was aliquoted into a non-cell-adhesive 96-well plate at 30000 cells/well, and subjected to suspension culture at 37°C under 5% $CO_2$ for 4 days. As a result, high-purity cerebral cortical cell aggregates were obtained. Figure 11(B) shows bright field images of cerebral cortical cell aggregates (Day 36) and a high-purity cerebral cortical cell aggregate (Day 40).

The cerebral cortical cell aggregates (Day 36) and high-purity cerebral cortical cell aggregates (Day 40) were further immunostained for the cortical layer V/VI progenitor cell (deep layer) marker Ctip2, the telencephalon marker Foxg1, the neural stem cell (radial glia) marker Pax6/Ki67, and the proliferative cell marker Ki67.

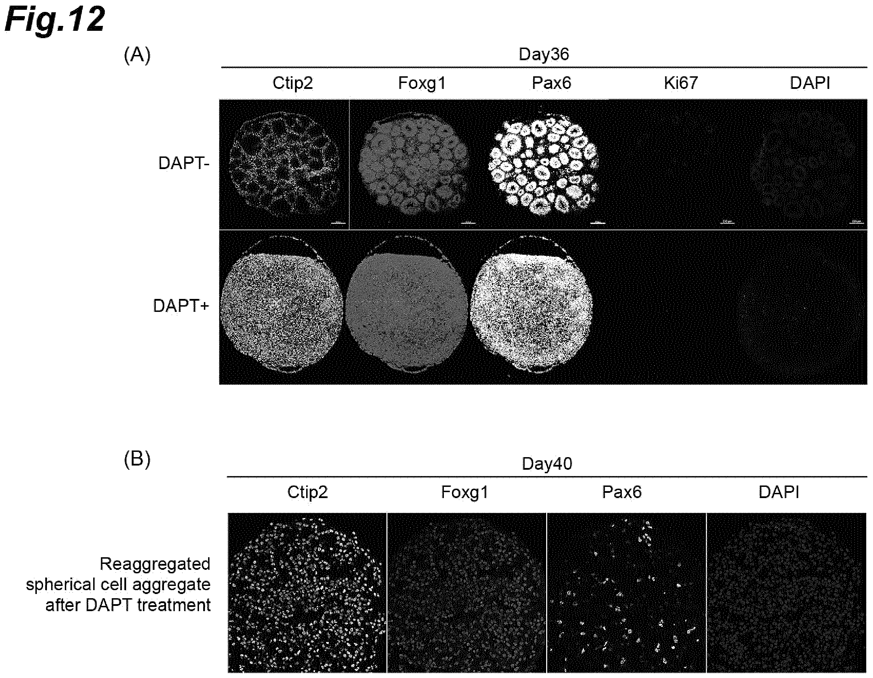

Figure 12 shows representative confocal fluorescence microscopy images of the immunostaining. In Figure 12(A), the top shows immunostaining images of a cerebral cortical cell aggregate on Day 36 without DAPT and the bottom shows a cerebral cortical cell aggregate on Day 36 with DAPT (both obtained through steps (i) to (ii)), and Figure 12(B) shows immunostaining images of a high-purity cerebral cortical cell aggregate on Day 40 (obtained through steps (i) to (iv)).

For the group cultured until Day 36 without DAPT treatment (top of (A)), many cerebral cortex-like layered structures were found, each including a Pax6/Ki67-positive neural stem cell (radial glia) layer, which is a feature of cerebral organoids, and a layer of Ctip2/Foxg1-double-positive cortical layer V/VI neural cells external to the neural stem cell layer; for the group with DAPT treatment (bottom of (A)), by contrast, although Pax6-positive cells were found, almost no expression of Ki67 was found, and no cerebral cortex-like layered structure was found. Whereas, Ctip2/Foxg1-positive cells were found throughout the cell aggregates. Thus, the cerebral cortical cell aggregates were revealed to lack the cerebral cortex-like layered structure and include Ctip2/Foxg1-double-positive cortical layer V/VI neural cells widely distributed in the inside.

Moreover, for the high-purity cerebral cortical cell aggregates (Figure 12(B)), which were obtained by dispersing cerebral cortical cell aggregates into single cells and reaggregating the single cells, Ctip2/Foxg1-double-positive cortical layer V/VI neural cells were found throughout the inside, and the number of Pax6-positive cells was much smaller.

4-2. Without Dispersion and Reaggregation

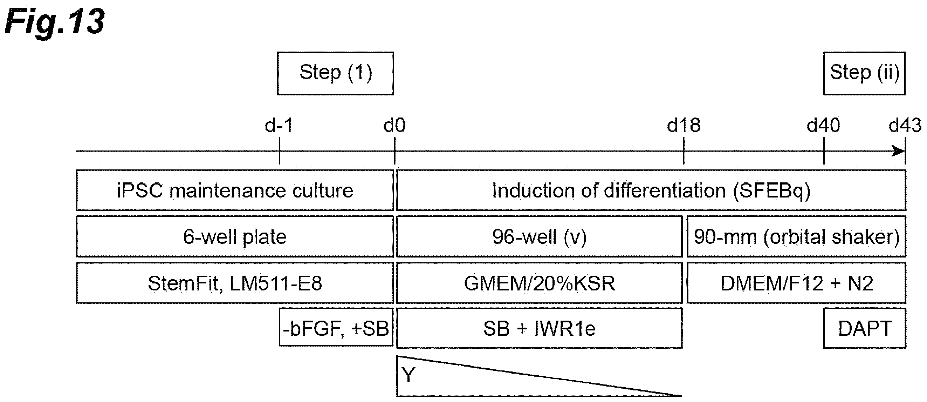

Next, cell aggregates immediately after DAPT treatment (immediately after the completion of step (ii)) were analyzed for the proportions of constituent cells by means of flow cytometry. The differentiation induction scheme is shown in Figure 13. Cerebral organoids on Day 40 were subjected to DAPT treatment for 3 days, and expressions of marker genes were then analyzed by flow cytometry.

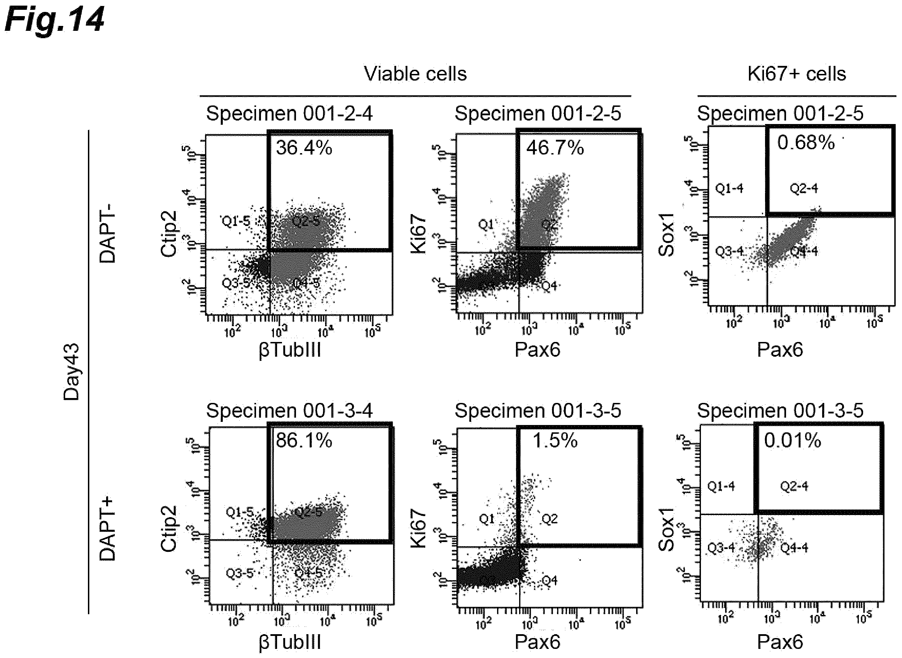

Figure 14 shows results of expression analysis by flow cytometry for the cortical layer V/VI progenitor cell (deep layer) marker Ctip2, the neuronal marker βIII-tubulin (βTubIII), the neural stem cell (radial glia) marker Pax6/Sox1/Ki67, and the proliferative cell marker Ki67.

Each dot in Figure 14 corresponds to a cell. The left and center panels show results for all viable cells, and the right panels show results only for Ki67-positive cells. The proportion of Ctip2/βIII-tubulin-double-positive cells (left panels) was 36.4% in the DAPT-untreated group (top), and as high as 86.1% in the DAPT-treated group (bottom). On the other hand, the proportion of Pax6/Ki67-double-positive neural stem cells (radial glia) (center panels) was 46.7% in the DAPT-untreated group (top), and as low as 1.5% in the DAPT-treated group (bottom). Furthermore, the proportion of Pax6-positive/Sox1-positive/Ki67-positive cells (right panels), which are proliferative cells, was 0.68% in the DAPT-untreated group (top), and as low as 0.01% in the DAPT-treated group (bottom).

Thus, it was revealed that culture of a cerebral organoid in the presence of the Notch signaling inhibitor DAPT leads to the absence of a cerebral cortex-like structure and significant reduction in number of proliferative cells, and, on the other hand, leads to increase in number of neural cells (in particular, cortical layer V/VI neural cells).

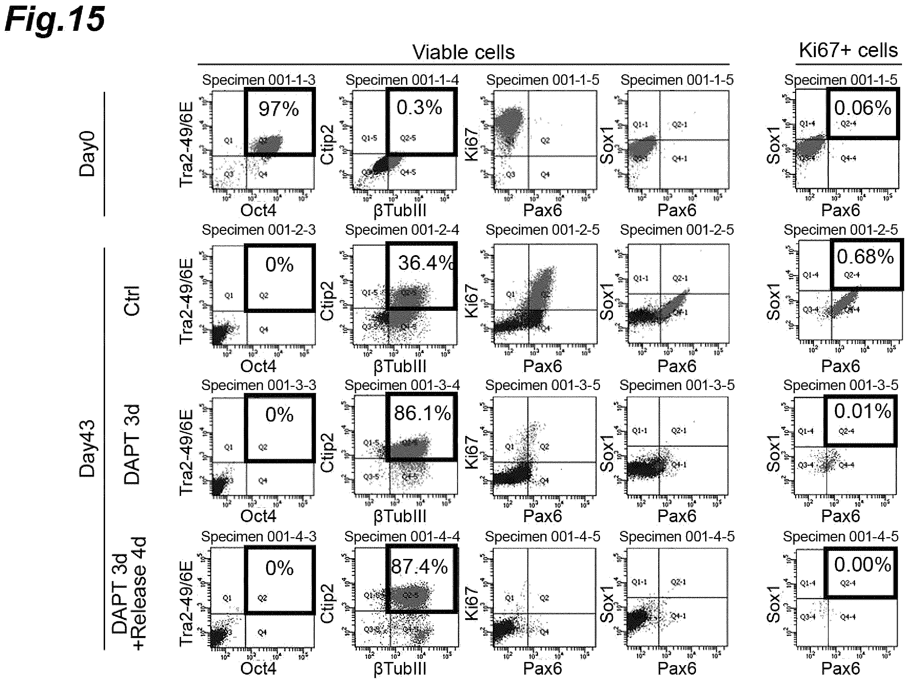

Furthermore, iPS cells on Day 0 (undifferentiated iPS cells), organoids not subjected to DAPT treatment (Ctrl, without step (ii)), organoids subjected to DAPT treatment for 3 days (Day 40 to 43) (DAPT 3d, with step (ii)), and organoids subjected to DAPT treatment for 3 days and subsequently cultured again, as cell aggregates without being dispersed into cells, with a culture medium removed of DAPT for 4 days (DAPT 3d +Release 4d, with step (ii) and step (iv), and without step (iii)) were analyzed by flow cytometry; the results are shown in Figure 15.

In Figure 15, the panels in the first to fourth column from the left show results for all viable cells, and the rightmost panels show results only for Ki67-positive cells. Cells with co-expression of Tra2-49/6E and Oct4, which is an indicator of an undifferentiated iPS cell, were not detected in organoids under any of the conditions (the first column from the left). Cells co-positive for Ctip2 and βIII-tubulin accounted for 36.4% in "Ctrl" (the second row from the top), and the proportion was as high as 86.1% in "DAPT 3d" (the third row from the top) and 87.4% in "DAPT 3d +Release 4d" (the lowermost row) (the second column from the left). Ki67/Sox1/Pax6-triple-positive cells accounted for 0.68% in "Ctrl", and the proportion was as low as 0.01% in "DAPT 3d" and 0% in "DAPT 3d +Release 4d" (the first column from the right). The proportion of Pax6/Sox1/Ki67-triple-positive cells in "DAPT 3d +Release 4d" was much lower than that in "DAPT 3d", from which it was found that the number of proliferative cells can be reduced only by culturing a cell aggregate as it is after DAPT treatment.

4-3. Organoid DAPT Method and Single-Cell DAPT Method (2)

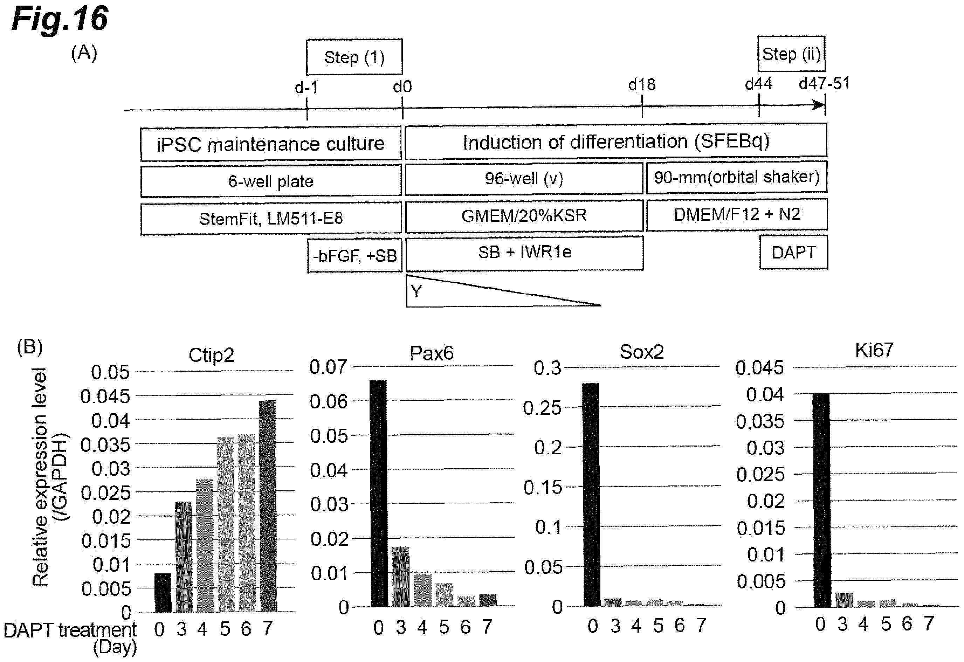

To analyze the influence of the DAPT treatment period on differentiation, cerebral organoids in week 7 of induction of differentiation were treated with 10 μM DAPT for 3 to 7 days according to a method or scheme shown in Figure 16(A) (organoid DAPT method), and the variation of gene expression was analyzed by RT-qPCR; the results are shown in Figure 16(B). The ordinate represents relative expression levels to GAPDH.

It was found from Figure 16(B) that the longer the DAPT treatment period, the more the maturation progressed and the number of proliferative cells decreased. The expression of Ctip2, which is a marker for cortical layer V/VI progenitor cells, was enhanced as the DAPT treatment period was prolonged. On the other hand, the expressions of Pax6, Sox2, and Ki67, which are markers for neural progenitor cells (radial glial cells), declined as the DAPT treatment period was extended. From these results, it was revealed that the neural differentiation and maturation proceeds and the proportion of proliferative cells decreases as DAPT treatment is continued.

<Example 5> Examination of Timing of Step (ii)

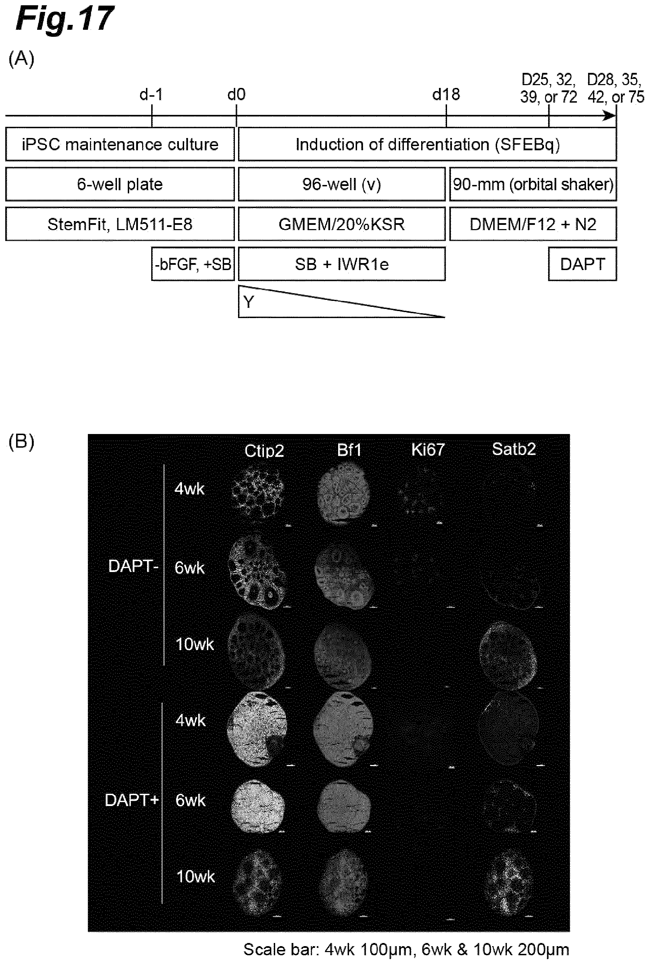

Figure 17(A) shows a scheme of induction of differentiation. Organoids on Day 25, Day 32, Day 39, and Day 72 (the periods of induction of differentiation: about 4 weeks, 5 weeks, 6 weeks, and 10 weeks, respectively) were cultured in the presence or absence of DAPT for 3 days, and the organoids on Day 28 (4 wk), Day 42 (6 wk), and Day 75 (10 wk) were analyzed for different markers by immunostaining. Figure 17(B) shows the results of immunostaining (confocal fluorescence microscopy images). Satb2 is a cortical layer II-IV progenitor cell (upper layer) marker. With reference to Figure 17(B), a rosette structure consisting of Ctip2Bf1-co-positive cells and Ki67-positive cells was appreciable in the organoids in week 4 to week 6 in the absence of DAPT, whereas no rosette structure was appreciable after the DAPT treatment, and the DAPT treatment was found to lead to an increased number of Ctip2Bf1-co-positive cells and a decreased number of Ki67-positive cells. For the organoids in week 10, an unclear rosette structure resulted after the DAPT treatment, but the DAPT treatment was found to cause no significant change in the proportion of Ctip2-positive cells and that of Satb2-positive cells.

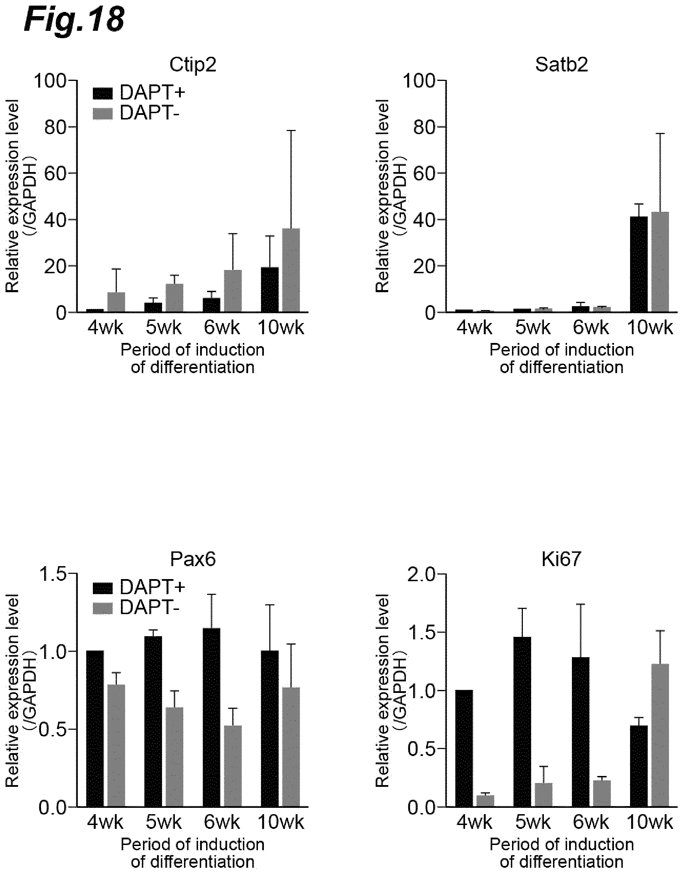

Furthermore, organoids on Day 25, Day 32, Day 39, and Day 72 (the periods of induction of differentiation: about 4 weeks, 5 weeks, 6 weeks, and 10 weeks, respectively) were cultured in the presence or absence of DAPT for 3 days, and the cerebral organoids on Day 28 (4 wk), Day 35 (5 wk), Day 42 (6 wk), and Day 75 (10 wk) were analyzed for the expression levels of marker genes by RT-qPCR; the results of analysis of the relative expression levels of the markers are shown in Figure 18. It was found from Figure 18 that the organoids in week 4 to week 6 tended to exhibit enhanced expression of Ctip2 and reduced expression of Pax6 and Ki67 after the DAPT treatment. For the organoids in week 10, no reduction in expression of Ki67 was found, and no influence was found in expression of Satb2, too.

Those results revealed that it is preferable that the timing of initiation of step (ii) be approximately day 20 to 44 after the initiation of induction of differentiation (Day 20 to Day 44), and more preferably Day 25 to Day 39. It was also revealed that it is preferable that the period of step (ii) be approximately 2 days to 4 days, and it is more preferable that the period of step (ii) be around 3 days (e.g., 60 to 84 hours).

<Example 6> Effect of Steps (ii) to (iv) in Step (2) - Part 2: Transplant Expansion-Suppressing Effect

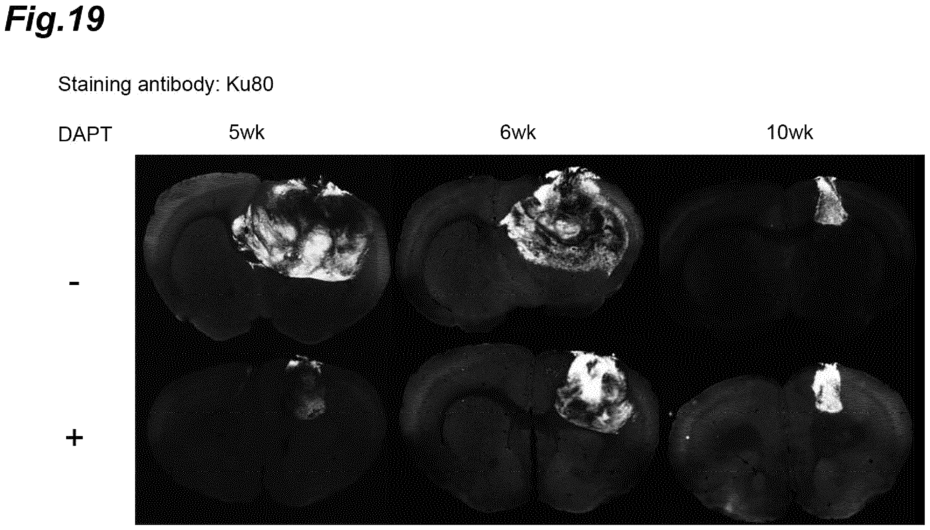

Cerebral cortical cell aggregates obtained through the differentiation induction scheme in Figure 17(A) were transplanted into the brains of Scid mice (CLEA Japan, Inc.) at $1.5 \times 10^5$ cells by a stereotactic brain transplantation method, and 3 months after the transplantation the brains were fixed by perfusion with 4% paraformaldehyde and frozen sections of 35 $\mu$m in thickness were prepared. The brain sections prepared were evaluated for the survival of human cells by immunostaining with Ku80. Figure 19 shows representative confocal fluorescence microscopy images of the immunostained transplants.

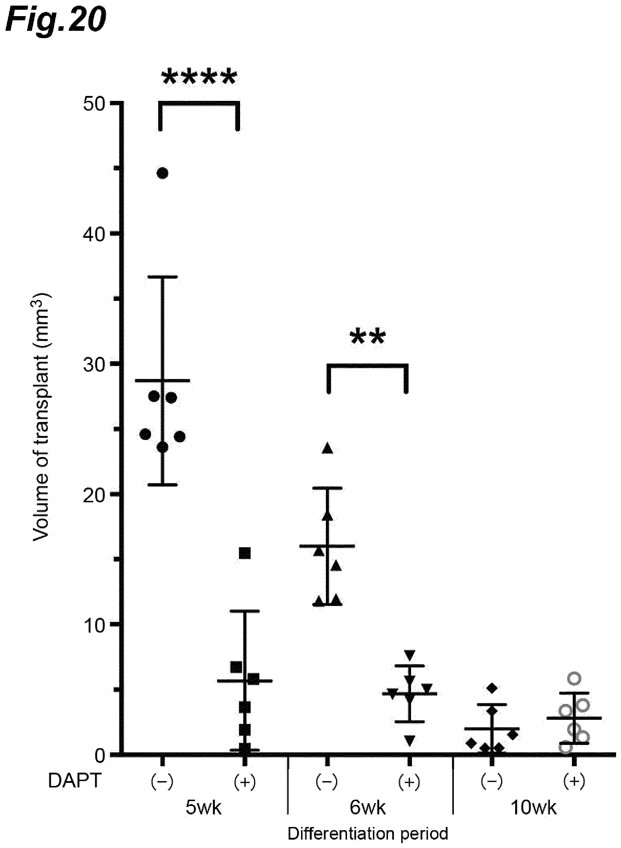

In addition, Figure 20 shows results of analysis of the volumes of the stained transplants. The volumes of the transplants were calculated in such a manner that immunostained brain sections were observed at intervals of 350 $\mu$m, the area of a transplant was calculated from the Ku80-stained image in each section, and the area multiplied by the section-to-section interval was added up. Test by one-way ANOVA (Tukey multiple test) was performed (****$p < 0.0001$, **$p < 0.01$).

It was demonstrated from Figure 19 and Figure 20 that the cerebral cortical cell aggregates obtained through step (ii) approximately 5 weeks or 6 weeks after the initiation of induction of differentiation exhibited higher survival rates after intracerebral transplantation than the cerebral cortical cell aggregates obtained without step (ii), and exhibited very little volume increase.

Thus, it was revealed that cerebral cortical cell aggregates formed by culturing a cerebral organoid, which has been obtained by induction of differentiation of pluripotent stem cells, in the presence of DAPT and then culturing in a culture solution containing one or more neurotrophic factors, ascorbic acid, and a cAMP activator exhibit high survival rates after intracerebral transplantation, and are less likely to expand (less cell growth).

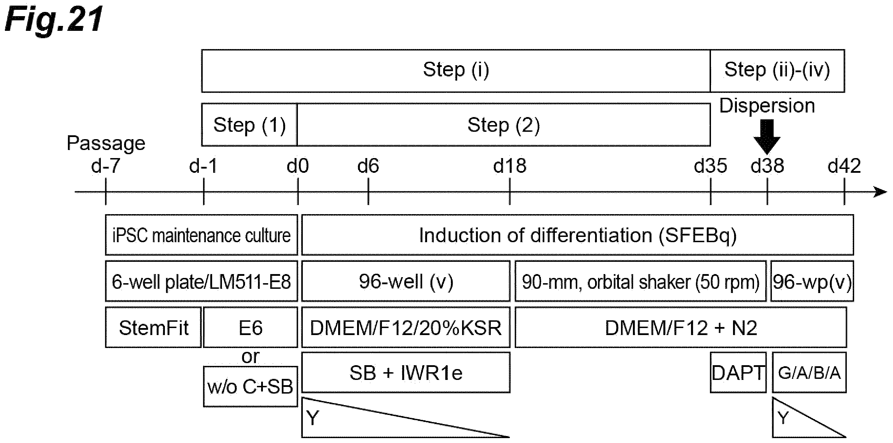

On the basis of Examples 1 to 6, Figure 21 shows a preferred embodiment of the method of the present invention.

A preferred method for producing a cerebral organoid from pluripotent stem cells in the absence of sustentacular cells comprises the following steps.

Step (1): a step of culturing pluripotent stem cells in a culture solution that is substantially free of bFGF and provokes substantially no TGFβ signal; and
step (2): a step of inducing cells obtained in step (1) to differentiate into a cerebral organoid.

Step (2) comprises the following steps.

Step (2a): a step of subjecting the cells obtained in step (1) to suspension culture in a culture solution containing a TGFβ signaling inhibitor and a Wnt signaling inhibitor to obtain a cell aggregate; and
step (2b): a step of subjecting the cell aggregate obtained in step (2a) to suspension culture in a culture solution substantially free of a TGFβ signaling inhibitor or a Wnt signaling inhibitor to obtain a cerebral organoid.

A preferred method for producing a cerebral cortical cell aggregate, even a high-purity cerebral cortical cell aggregate, from pluripotent stem cells in the absence of sustentacular cells comprises the following steps.

Step (i): a step of obtaining a cerebral organoid by a method comprising step (2) or a method comprising step (1) and step (2);
step (ii): a step of culturing the cerebral organoid obtained in step (i) in a culture solution containing a Notch signaling inhibitor (such as DAPT) to obtain a cerebral cortical cell aggregate;
step (iii): a step of dispersing a cell culture obtained through step (ii) into single cells or two- to five-membered cell clumps; and
step (iv): a step of culturing cells obtained in step (iii) in a culture solution containing one or more neurotrophic factors, ascorbic acid, and a cAMP activator to obtain a high-purity cerebral cortical cell aggregate.

A high-purity cerebral cortical cell aggregate can be obtained even if the cell culture obtained through step (ii) is cultured in the culture solution in step (iv) with step (iii) skipped.

<Example 7> Examination of Effect of DAPT on Organoids Induced with Preparation for Neural Differentiation

Culture was performed in the same manner as the scheme in Figure 3 as in Example 1 from -Day 7 to Day 35. A culture medium obtained by adding 5 $\mu$M SB431542 to StemFit culture medium without solution C (= bFGF-free culture medium) was used in step (1) in the case of "with preparation for neural differentiation" (with step (1)), and StemFit culture medium was used in the case of "without preparation for neural differentiation" (without step (1)). Thereafter, cerebral cortical cell aggregates were induced by the method described for step (ii) in Example 4, and evaluated by immunostaining. In addition, high-density cerebral cortical cell aggregates were induced by dispersing into single cells and then reaggregating the single cells by the method described for step (iii) and step (iv) in Example 4, and analyzed by flow cytometry.

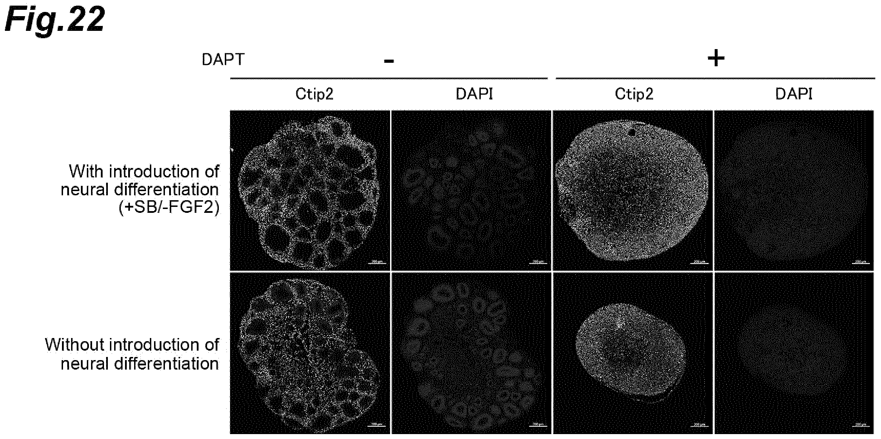

Figure 22 shows representative confocal fluorescence microscopy images of the immunostaining. Even when preparation for neural differentiation was not performed, a cerebral cortex-like structure including a Pax6-positive neuroepithelium and a layer of Ctip2-positive cortical layer V/VI neural cells external to the neuroepithelium was formed, even though the efficiency was low. When the organoid was subjected to DAPT treatment, Ctip2-positive cells became appreciable throughout the cell aggregate as with the case with preparation for neural differentiation.

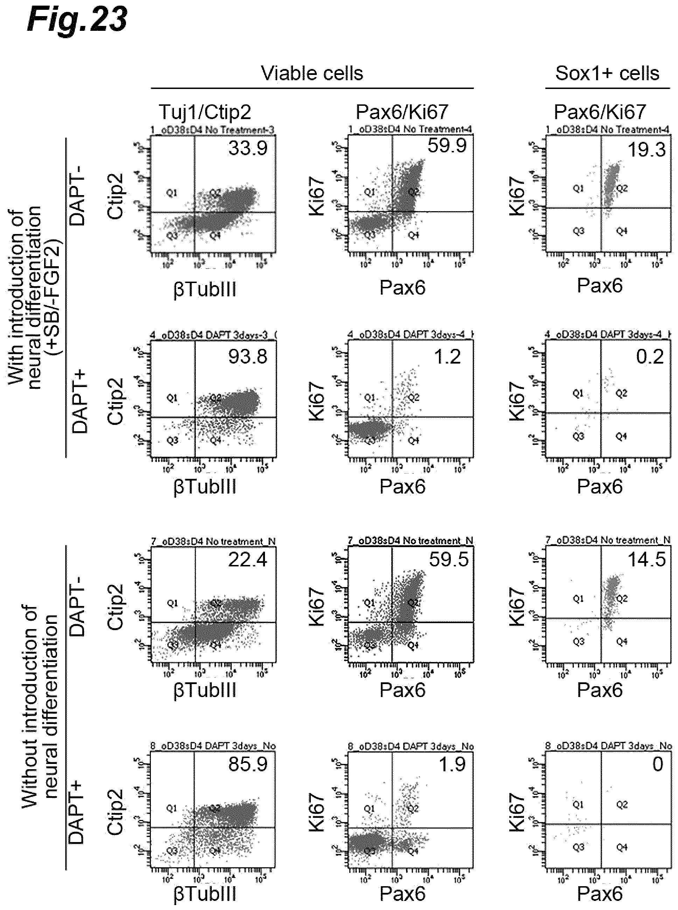

Figure 23 shows results of the analysis by flow cytometry. With reference to Figure 23, the proportion of Ctip2/$\beta$TubIII-double-positive cells (left panels) increased from 22.5% to 85.9% by the DAPT treatment, even in the case without preparation for neural differentiation. The proportion of Pax6/Ki67-double-positive neural stem cells (radial glia) (middle panels) decreased from 59.5% to 1.9% by the DAPT treatment. Moreover, the proportion of Pax6-positive/Sox1-positive/Ki67-positive cells (right panels), which are proliferative cells, decreased from 14.5% to 0% by the DAPT treatment.

Thus, it was revealed that, irrespective of the presence or absence of preparation for neural differentiation, not only the cerebral cortex-like structure but also the neuroepithelium is unformed in cerebral cortical cell aggregates, Ctip2/$\beta$TubIII-double-positive cortical layer V/VI neural cells are widely distributed in the inside, and a decreased number of proliferative cells are given. That is, it was found that cerebral cortical cell aggregates and high-density cerebral cortical cell aggregates can be produced even if step (1) of the method for producing a cerebral organoid from pluripotent stem cells in the absence of sustentacular cells on the basis of Example 7 is omitted.

<Example 8> Examination of Timing of DAPT Treatment and Optimization of Method

8-1. Organoid DAPT Method and Single-Cell DAPT Method (1)

Culture was performed in the same manner as in Example 1 from -Day 7 to Day 35. A culture medium obtained by adding 5 $\mu$M SB431542 to StemFit culture medium without solution C (= bFGF-free culture medium) was used in step (1).

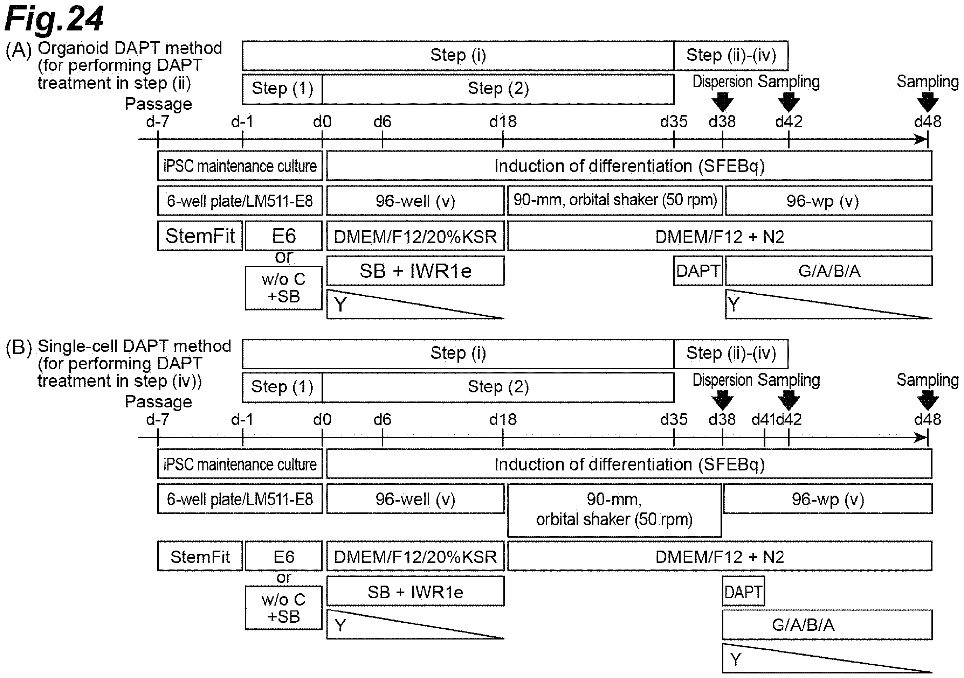

In the "organoid DAPT method (DAPT treatment is performed in step (ii))" in Figure 24(A), cerebral organoids were screened on day 35 after the initiation of induction of differentiation, the culture medium was replaced with a culture medium obtained by adding 10 $\mu$M DAPT to the culture medium in step (2b), and suspension culture was performed at 37°C under 5% CO2 for 3 days. The resulting cerebral cortical cell aggregates were dispersed into single cells and the single cells were then regathered by the method described for step (iii) and step (iv) in Example 4, giving high-purity cerebral cortical cell aggregates.

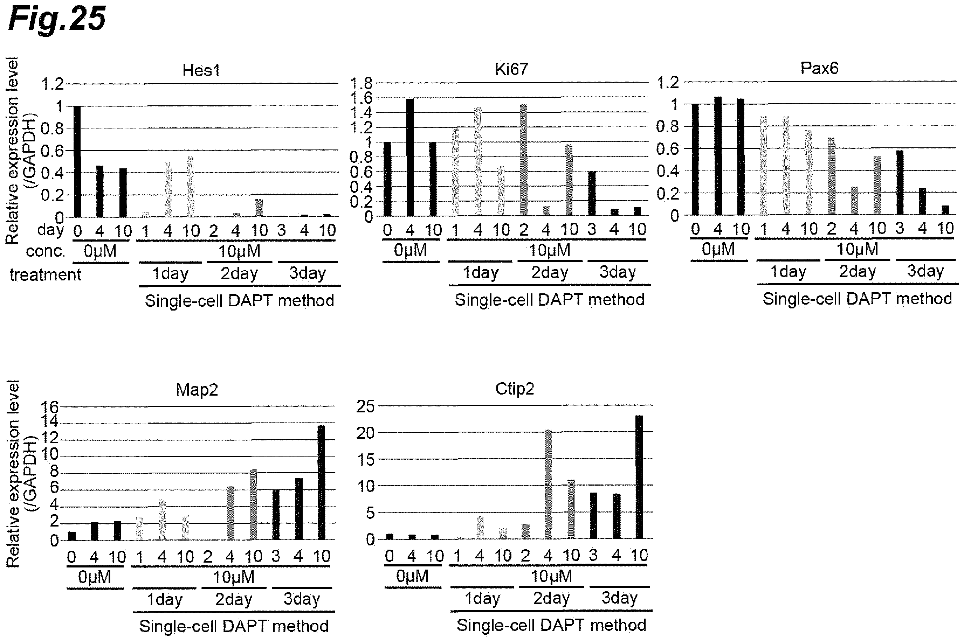

In the "single-cell DAPT method (DAPT treatment is performed in step (iv))" in Figure 24(B), cerebral organoids were screened on day 38 after the initiation of induction of differentiation and dispersed into single cells by the method described for step (iii) in Example 4, and the cells were then suspended in a culture medium obtained by adding 10 ng/mL GDNF, 20 ng/mL BDNF, 200 $\mu$M ascorbic acid, 400 $\mu$M dibutyryl-cAMP, and 30 $\mu$M Y-27632 to the culture medium in step (2b) with addition of DAPT at a concentration of 0 $\mu$M, 0.1 $\mu$M, 1 $\mu$M, or 10 $\mu$M. Subsequently, the cells were regathered by the method described for step (iv) in Example 4 in the presence of DAPT, and cultured for 1 day, 2 days, or 3 days. Thereafter, the culture medium was switched to a culture medium obtained by adding 10 ng/mL GDNF, 20 ng/mL BDNF, 200 $\mu$M ascorbic acid, and 400 $\mu$M dibutyryl-cAMP to the culture medium in step (2b), and culture was continued until day 10 after the initiation of reaggregation. The cells on day 3, day 4, or day 10 after the initiation of reaggregation were analyzed by RT-qPCR, and the cells on day 10 were analyzed by flow cytometry; the results are shown in Figures 25 to 27.

Figure 25 shows the results of analysis of gene expression levels of different markers over time by RT-qPCR in the single-cell DAPT method (a method in which DAPT treatment is performed in step (iv)) with a DAPT concentration of 10 $\mu$M and a DAPT treatment period of 1 day, 2 days, or 3 days. In Figure 25, "day" shows the number of days of step (iv), "Cone." shows the concentration of DAPT, and "Treatment" shows the number of days of DAPT treatment.

It was found from Figure 25 that Hes1, the expression of which is activated in the downstream of Notch signaling, was downregulated immediately after DAPT treatment irrespective of the length of DAPT treatment period, but upregulated again as the culture in step (iv) proceeded for the 1-day and 2-day DAPT treatment. For the 3-day DAPT treatment, on the other hand, the expression of Hes1 was suppressed over the 10 days. At the same time, the expression of Ki67, which is a marker for proliferation ability, and the expression of Pax6, which is a marker for neural stem cells, were also suppressed to low levels. By contrast, the expression of Map2, which is a marker for neural maturation, and the expression of Ctip2, which is a marker for layers of cortical layer V/VI neural cells, were enhanced as the culture in step (iv) proceeded. Thus, it was found that the proliferation of proliferative neural stem cells can be suppressed and an increased proportion of cortical layer V/VI neurons results by performing DAPT treatment for at least 3 days.

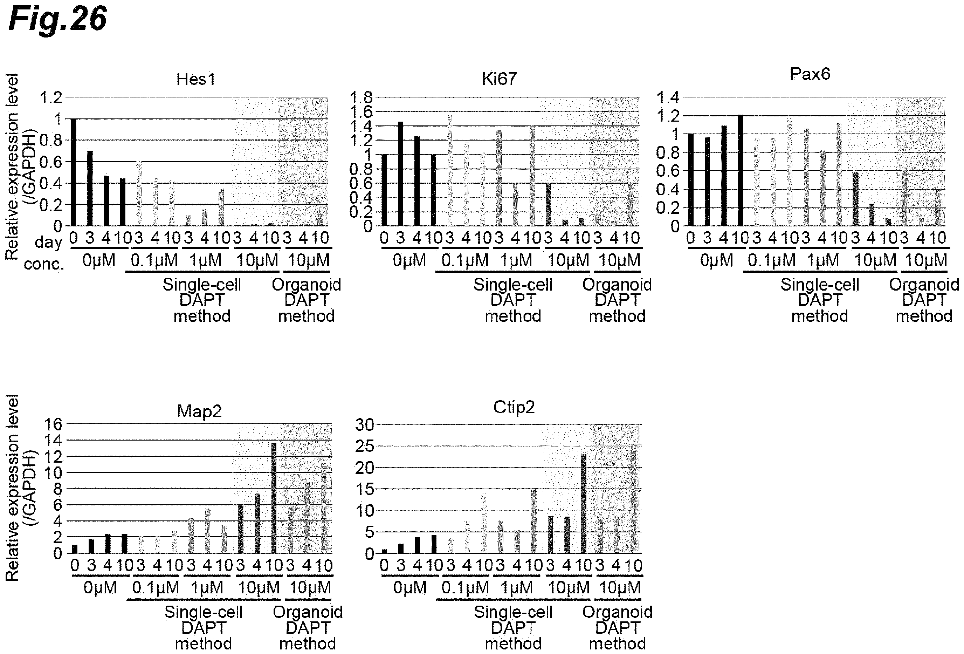

Figure 26 shows the results of analysis of gene expression levels of different markers over time by RT-qPCR in the single-cell DAPT method (a method in which DAPT treatment is performed in step (iv)) with a DAPT concentration of 0 $\mu$M, 0.1 $\mu$M, 1 $\mu$M, or 10 $\mu$M and a DAPT treatment period of 3 days. For comparison, a case that treatment was performed with a DAPT concentration of 10 $\mu$M and a DAPT treatment period of 3 days in step (ii) (organoid DAPT method) is also shown. In Figure 26, "day" shows the number of days of step (iv), and "Cone." shows the concentration of DAPT.

It was found from Figure 26 that Hes1, the expression of which is activated in the downstream of Notch signaling, was not sufficiently suppressed at DAPT treatment concentrations of 0.1 to 1 $\mu$M, but suppressed at 10 $\mu$M over the 10 days. At the same time, the expression of the proliferation marker Ki67 and the neural stem cell marker Pax6 was also suppressed over the 10 days. By contrast, the expression of Map2, which is a marker for neural maturation, and the expression of Ctip2, which is a marker for layers of cortical layer V/VI neural cells, were enhanced as the culture in step (iv) proceeded. Thus, it was found that, if DAPT treatment is performed in step (iv), by performing DAPT treatment at 10 $\mu$M for 3 days, the proliferation of proliferative neural stem cells can be suppressed and an increased proportion of cortical layer V/VI neurons results, and effects comparable to or more than those when DAPT treatment is performed in step (ii) can be achieved.

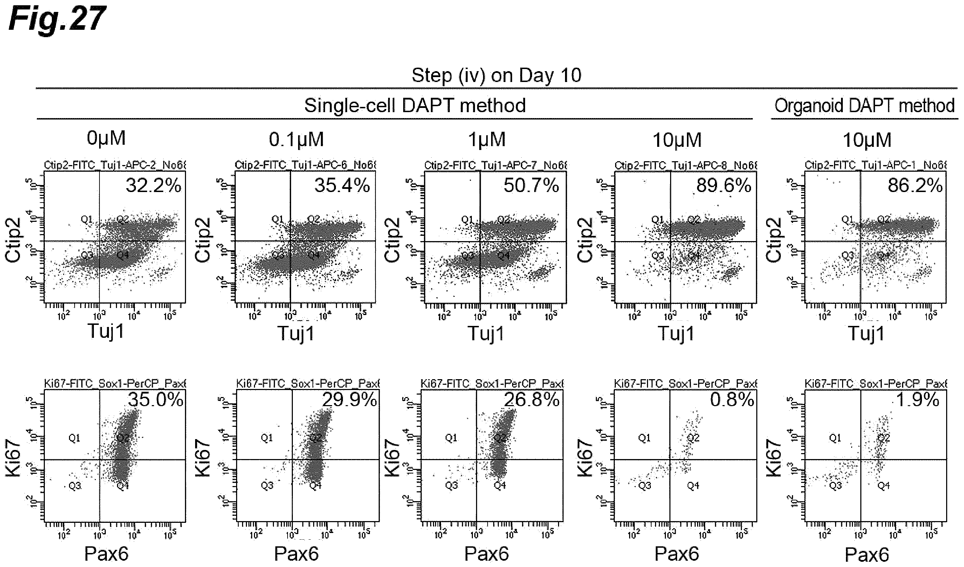

Figure 27 shows the results of analysis of gene expression levels of different markers by flow cytometry when DAPT treatment was performed in step (iv) with a DAPT concentration of 0 $\mu$M, 0.1 $\mu$M, 1 $\mu$M, or 10 $\mu$M and a DAPT treatment period of 3 days and culture was performed for 10 days in step (iv). For comparison, a case that treatment was performed with a DAPT concentration of 10 $\mu$M and a DAPT treatment period of 3 days in step (ii) (organoid DAPT method) is also shown.

It was found from Figure 27 that the proportion of Ctip2/$\beta$TubIII (Tuj1)-double-positive cells (top panels) increased from 32.2% to 89.6% as the DAPT treatment concentration increased. The proportion of Pax6-positive/Sox1-positive/Ki67-positive cells (bottom panels), which are proliferative cells, decreased from 35.0% to 0.8% through the DAPT treatment. Thus, it was revealed that if DAPT treatment is performed in step (iv), the proportion of Ctip2/$\beta$TubIII-double-positive cortical layer V/VI neural cells increases as the DAPT treatment concentration increases and the number of proliferative cells decreases, as in the case that DAPT treatment is performed in step (ii).

8-2. Single-Cell DAPT Method (2)

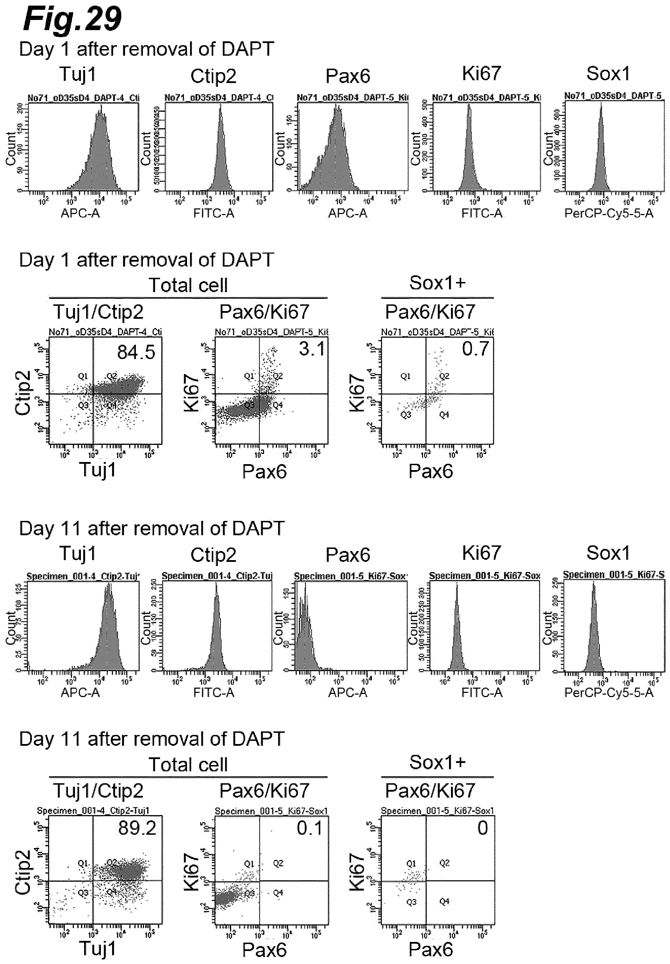

According to the method or scheme shown in Figure 28, cerebral organoids in week 5 of induction of differentiation were dispersed into single cells in step (II), and the single cells were then treated with 10 $\mu$M DAPT for 3 days in step (III). Thereafter, under conditions with a culture medium free of DAPT, culture was continued for 4 days (for 1 day after removal of DAPT) and 14 days (for 11 days after removal of DAPT), and marker expression in the resulting cell aggregates was analyzed by flow cytometry. Results of the analysis are shown in Figure 29.

Extension of the culture period after DAPT treatment resulted in the increase in the proportion of Ctip2/Tuj1-co-positive cells (the left panel in the fourth row). On the other hand, the proportion of Pax6/Ki67-double-positive cells (the middle panel in the fourth row) and the proportion of Pax6/Ki67/Sox1-triple-positive cells (the right panel in the fourth row), wherein the markers are those for proliferative neural progenitor cells, decreased as the culture period after DAPT treatment was extended. It was revealed from Figure 16(B) and Figure 29 that the neural differentiation and maturation proceeds and the proportion of proliferative cells decreases as the culture period after DAPT treatment is extended.

<Example 9> Analysis of Morphologies and Expression Profiles of Organoids

9-1. Analysis of Morphologies of Organoids

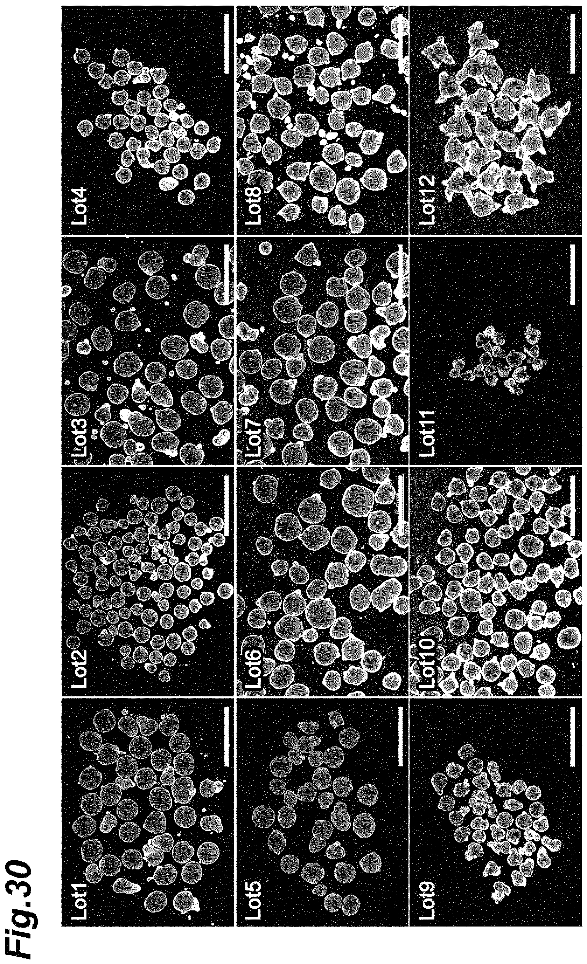

Culture was performed in the same manner as in Example 1 from -Day 7 to Day 35. Bright field images of cerebral organoids obtained in 12 induced differentiation lots (Lot 1 to Lot 12) were acquired by photographing with an inverted microscope (Leica DMS1000); Figure 30 shows the representative bright field images (scale bar: 5 mm). It was found from Figure 30 that the morphologies of organoids induced were different among lots.

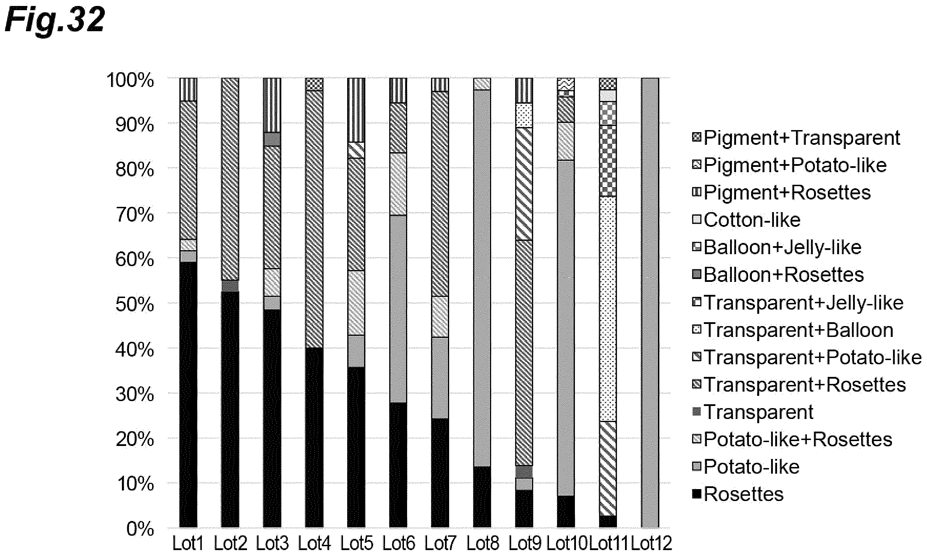

Observation of the cerebral organoids obtained from the 12 operations of induction of differentiation led to the idea that the cerebral organoids could be classified into 7 morphological groups: cerebral organoids each including rosette structure throughout the cell aggregate (rosette structure; Rosette); organoids with low transparency in each of which no clear structure was found (potato-like tissue; Potato-like); organoids each involving balloon-like structure (balloon-like tissue; Balloon); organoids each involving fibrous structure (cotton-like tissue; Cotton-like); organoids with high transparency in each of which cystoid structure was found in the inside (transparent tissue; Transparent), organoids in each of which black or brown pigmentation was found (pigments; Pigment); and organoids with high transparency in each of which no clear structure was found (jelly-like tissue; Jelly-like) (Figure 31). Figure 32 and Table 5 show statistical results according to the morphological classification.

[Table 5]

| | | | | | | | | | | | | | | |
|---|---|---|---|---|---|---|---|---|---|---|---|---|---|---|
| Rosettes | O | | O | | O | | | | O | | | O | | |
| Potato-like | | O | O | | | O | | | | | | | O | |
| Balloon | | | | | | | | O | | O | O | | | |
| Cotton-like | | | | | | | | | | | | O | | |
| Pigment | | | | | | | | | | | | O | O | O |
| Transparent | | | | O | O | O | O | O | | | | | | O |
| Jelly-like | | | | | | | | | O | | O | | | |
| Lot1 | 59 | 3 | 3 | 0 | 31 | 0 | 0 | 0 | 0 | 0 | 0 | 5 | 0 | 0 |
| Lot2 | 53 | 0 | 0 | 3 | 45 | 0 | 0 | 0 | 0 | 0 | 0 | 0 | 0 | 0 |
| Lot3 | 48 | 3 | 6 | 0 | 27 | 0 | 0 | 0 | 3 | 0 | 0 | 12 | 0 | 0 |
| Lot4 | 40 | 0 | 0 | 0 | 57 | 0 | 0 | 0 | 0 | 0 | 0 | 0 | 0 | 3 |
| Lot5 | 36 | 7 | 14 | 0 | 25 | 4 | 0 | 0 | 0 | 0 | 0 | 14 | 0 | 0 |
| Lot6 | 28 | 42 | 14 | 0 | 11 | 0 | 0 | 0 | 0 | 0 | 0 | 6 | 0 | 0 |
| Lot7 | 24 | 18 | 9 | 0 | 45 | 0 | 0 | 0 | 0 | 0 | 0 | 3 | 0 | 0 |
| Lot8 | 14 | 84 | 3 | 0 | 0 | 0 | 0 | 0 | 0 | 0 | 0 | 0 | 0 | 0 |
| Lot9 | 8 | 3 | 0 | 3 | 50 | 25 | 6 | 0 | 0 | 0 | 0 | 6 | 0 | 0 |
| Lot10 | 7 | 75 | 8 | 0 | 6 | 0 | 0 | 1 | 0 | 0 | 0 | 0 | 3 | 0 |
| Lot11 | 3 | 0 | 0 | 0 | 0 | 21 | 50 | 16 | 0 | 5 | 3 | 0 | 0 | 3 |
| Lot12 | 0 | 100 | 0 | 0 | 0 | 0 | 0 | 0 | 0 | 0 | 0 | 0 | 0 | 0 |

Table 5 shows the proportions of organoids of different morphologies in a table. The organoids were classified by the type of morphology included in one organoid. Each column in the first to seventh rows from the top shows a morphology included in individual organoids. The first to twelfth rows from the bottom show results of classification of induced organoids in the 12 lots of induction of differentiation by morphology. Each numerical value in Table 5 indicates the proportion of organoids of the corresponding morphology in percentage to the total number of organoids in the corresponding lot, and the color gradation corresponds to the numerical magnitude. Figure 32 shows the proportions of organoids of different morphologies in bar graphs.

The proportions of organoid groups generated were largely different among differentiation lots, and a lot in which "rosette structure", "balloon-like tissue or cotton-like tissue", and "transparent tissue" were included in combination, a lot mostly consisting of "balloon-like tissue or cotton-like tissue", and a lot mostly consisting of "potato-like tissue" were found (Figure 32, Table 5). Thus, it was found that the proportions of organoid types generated in an operation of induction of differentiation vary among lots. Some organoids had features attributed to multiple classes of the aforementioned seven morphological classes in combination. It should be noted that those organoids can be visually screened by observation with magnification.

9-2. Single-Cell Gene Expression Analysis for Organoids in Different Morphological Groups

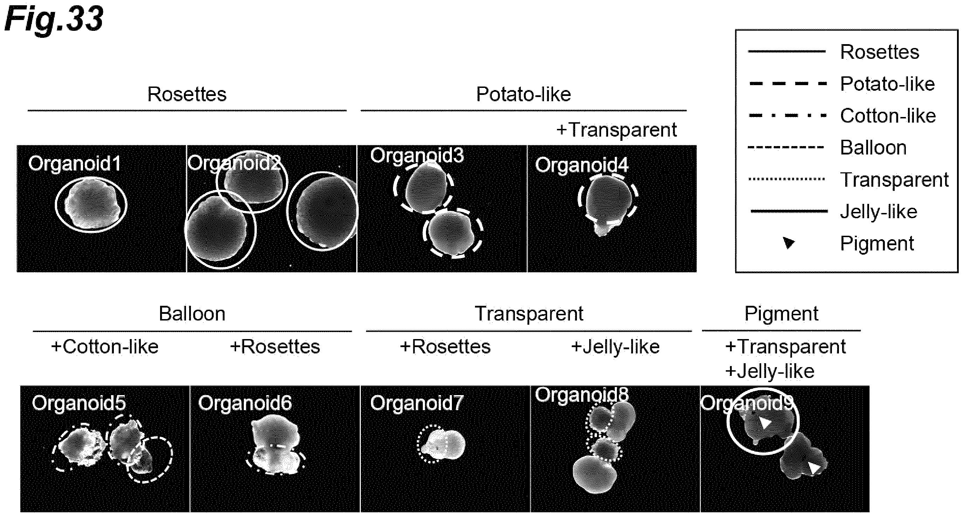

To identify cell types included in organoids in different morphological groups, single-cell gene expression analysis was performed. In the analysis, nine organoids obtained through three operations of induction of differentiation were used (Figure 33). Above each photograph, the morphology of tissue included in the corresponding organoid is shown. The single-cell gene expression analysis was performed as follows.

First, to prepare a single-cell suspension from an organoid, the organoid was separated into single cells with dispersing solution for neural cells (FUJIFILM Wako Pure Chemical Corporation) under recommended conditions. The dispersed cells were resuspended in HBSS supplemented with 10% (v/v) KSR and 10 $\mu$M Y-27632 (FUJIFILM Wako Pure Chemical Corporation) at a density of 1,000 cells/$\mu$L. Into a Chromium Next GEM Chip G (2000177 10X Genomics, Inc.), approximately 4,670 cells were loaded per channel, and treated with a Chromium controller to acquire Gel Beads-in-Emulsion (GEM). Libraries were produced by using Chromium Next GEM Single Cell 3' Reagent Kits v3.1 (1000121; 10X Genomics, Inc.) in accordance with a protocol from the manufacturer (CG000204 Rev C). The sequences of the libraries were analyzed with Novaseq 6000 (Illumina, Inc.).

Cell Ranger pipeline was used for mapping of sequences in single-cell RNA-seq. GRCh38 human genome sequences were used as reference genome sequences. UMI count values acquired through analysis with a next-generation sequencer were analyzed by using Seurat R package. First, data of organoids were normalized by a Log-Normalize method, and then all the data of organoids were integrated. Next, the top 2000 genes with large cell-to-cell variation were extracted, and principal component analysis (PCA) was carried out with the data of those genes, and the top 50 principal components (the first principal component to the 50th principal component) were acquired. To further analyze the organoids in the three lots of induction of differentiation in a simultaneous manner, batch effect correction was performed by using a Harmony method, and dimensionality reduction by a UMAP (Uniform Manifold Approximation and Projection) method was carried out to convert into two-dimensional data. Thereafter, clustering based on Shared nearest neighbor graphs was carried out to classify all the cells into clusters. Furthermore, cell types constituting each cluster were identified on the basis of a gene cluster characteristically expressed in the cluster.

Similarities and differences in gene expression among individual cells were evaluated by visualizing the two-dimensional data acquired by the UMAP method. Specifically, several tens of thousands of cells are expressed as fifty-dimensional data (principal components) based on the gene expression data, and the data are compressed into two-dimensional data by the UMAP method with the similarities and differences maintained, and converted into a plot on a plane. In the plot given by the UMAP method, each dot indicates an individual cell, and cells with similar gene expression patterns are plotted closer. If many cells with particularly high similarity are present, a massive structure appears in which the dots indicating the cells are densely positioned. A cell group constituting such a structure is interpreted as cells that exhibit very similar gene expression, that is, identical or closely relative cell types (Nature Biotechnology volume 37, pages 38-44 (2019)).

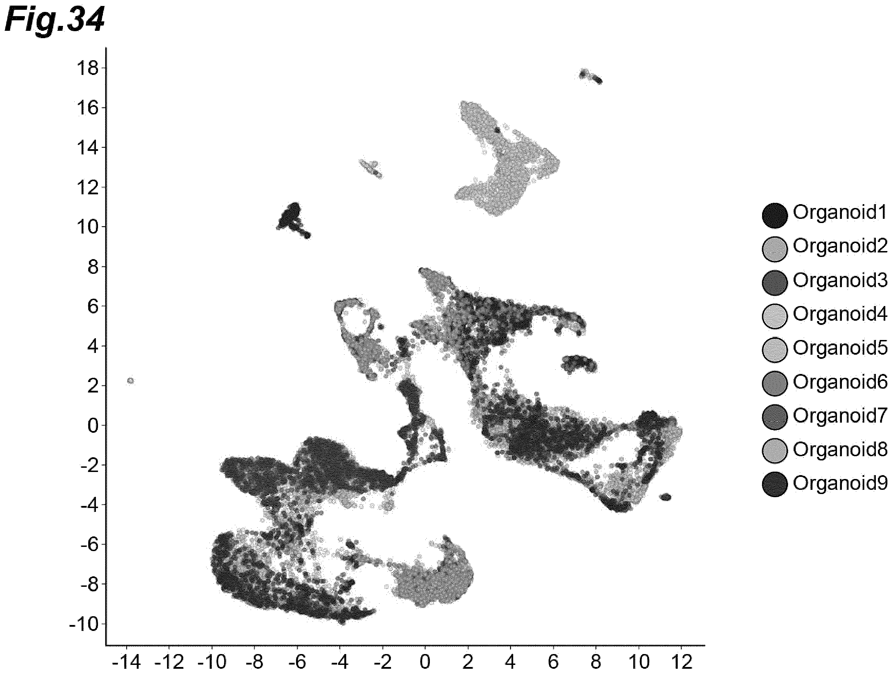

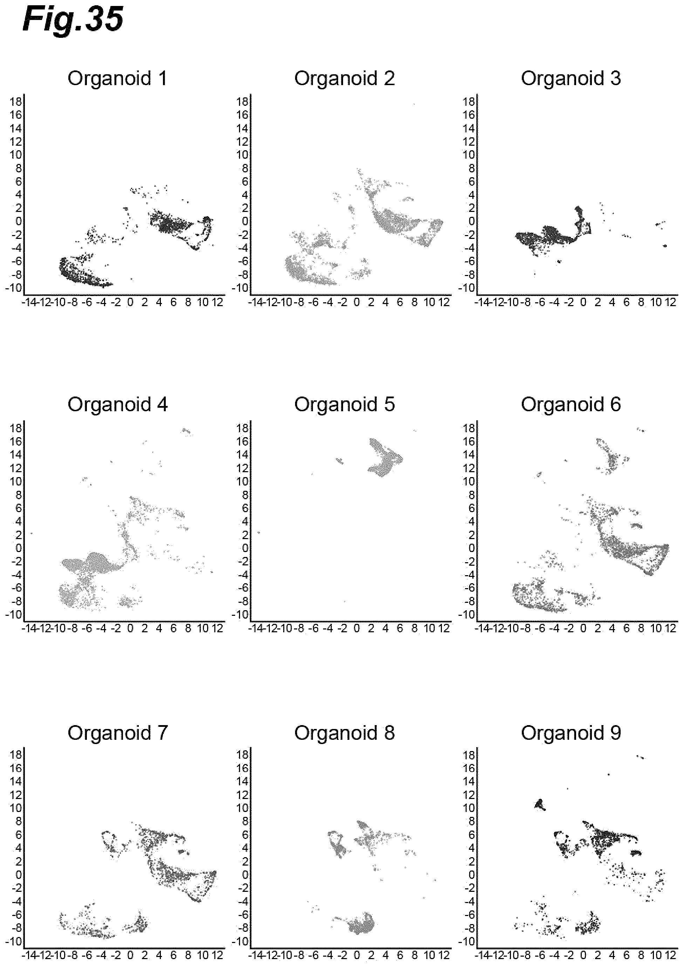

Figures 34 and 35 show results of representation of single-cell RNA-seq analysis data by the UMAP method for the cells included in the nine organoids analyzed. With reference to Figure 34, the cells included in the nine organoids were classified into 10 or more different clusters, which suggested that 10 or more cell populations each exhibiting a characteristic gene expression pattern were included. In addition, different organoids exhibited different plot patterns (Figure 35), which suggested that different cell types were included in different organoids.

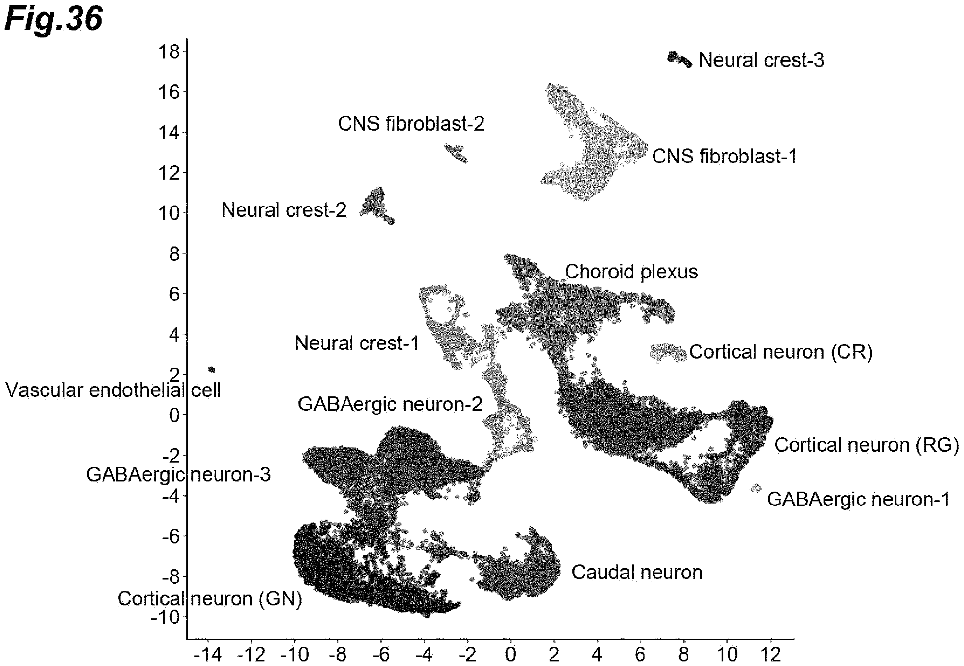

To identify the cell types included in the clusters, classification was statistically performed by a Nearest Neighbours method to give 14 clusters. Genes characteristically expressed in each cluster were extracted on the basis of proportions of expressing cells and mean expression levels; the results are shown in Table 6. Top 50 genes are shown in ascending order for each cell in Table 6. Cell type annotation was carried out with reference to published information, and the cell types of the clusters were identified based on the gene expression data (Figure 36). Each dot indicates an individual cell, and different shades of dots indicate different clusters. The cell types are expected to be Cortical neuron (radial glia (RG)): cerebral cortical neural progenitor cells (radial glia (RG)), Cortical neuron (CR): Cajal-Retzius cells (CR), Cortical neuron (glutamatergic neuron (GN)): cerebral cortical nerve cells (glutamatergic neurons (GN)), GABAergic neurons: GABAergic neural cells, Choroid plexus (ChP): choroid plexuses, CNS fibroblasts: central nervous system fibroblasts, Neural crests: neural crest cells, Vascular endothelial cell: vascular endothelial cells, and Caudal neuron: caudal nerve cells.

[Table 6]

| Cortical neuron (Radial Glia (RG)) | Cortical neuron (Glutamater gic neuron (GN)) | Cortical neuron (Cajal retzius cell (CR)) | GABAergic neuron-1 | GABAergic neuron-2 | GABAergic neuron-3 | Choroid plexus (ChP) | CNS fibroblast -1 | CNS fibroblast -2 | Caudal neuron | Neural crest-1 | Neural crest-2 | Neural crest-3 | Endothelial cells |
|---|---|---|---|---|---|---|---|---|---|---|---|---|---|
| SFRP1 | NEUROD6 | RELN | HIST1H4C | DLX2 | SIX3 | TTR | COL3A1 | GABRP | CRABP1 | HESS | LGALS 1 | S100A6 | H19 |
| ZFP36 L1 | NEUROD2 | LHX1 | TOP2A | PANTR1 | DLX6-AS 1 | TPBG | COL1A1 | S100A11 | NEFL | ZIC1 | TWIST 1 | S100B | KDR |
| ID4 | SSTR2 | NHLH2 | HIST1H3B | DLX1 | SST | TRPM3 | DCN | KRT19 | NEFM | MSX1 | PRRX1 | OLFML2A | TFPI |
| HMGB 2 | TBR1 | PCP4 | HIST1H3D | CENPF | PBX3 | FAM89 A | SPARC | KRT18 | ROBO3 | NES | SHOX2 | AL139393.3 | CARTPT |
| HES1 | ZBTB18 | RSPO3 | HIST1H2AG | TOP2A | TAC1 | RSPO3 | COL1A2 | KRT8 | CNTN2 | ATP1A2 | GPC3 | PLP1 | CAV1 |
| TTYH1 | NHLH1 | NR2F2 | NUSAP1 | DLX5 | RUNX1T1 | RSPO2 | LUM | FN1 | HOXA5 | MIR99AH G | DNM3 OS | S100A10 | CD93 |
| SOX3 | IGFBPL1 | PGF | HIST1H1B | ASCL1 | STMN2 | WLS | MGP | IGFBP3 | RGMB | CCND1 | PDGFR A | FN1 | PECAM 1 |
| SYNE2 | NRN1 | MAB21L 1 | HIST2H2AC | ZEB2 | NSG2 | IGFBP7 | DLK1 | S100A1 0 | HOXB5 | NTRK2 | ID1 | BCHE | GJA4 |
| LHX2 | RTN1 | CDKN1 A | GAD2 | GAD2 | RTN1 | SOX2-OT | FRZB | ANXA1 | LINC02381 | ID1 | CRABP 2 | S100A4 | GNG11 |
| HMGA 2 | THSD7A | LHX5-AS1 | CENPF | SLC1A3 | DLX5 | ID1 | POSTN | CRABP2 | LHX1 | ID3 | ID3 | SOX10 | CDH5 |
| SOX2 | NRXN1 | SEMA6 A | MKI67 | FAM181 B | GRIA2 | ID3 | H19 | HPGD | POU4F1 | RFX4 | KCTD1 2 | ADAM 12 | CLDN5 |
| C1orf6 1 | BHLHE22 | EBF3 | HIST1H1C | NUSAP1 | INA | NEAT1 | S100A10 | NPY | TFAP2A | CRNDE | S100A1 1 | S100A11 | ICAM2 |
| B3GAT 2 | CALB2 | LHX1-D T | SMC4 | HIST1H1B | MAPT | HTR2C | APOE | ANXA2 | HOXB3 | OLIG3 | NR2F2 | IGFBP7 | icFaP4 |
| GLI3 | KHDRBS3 | TBR1 | HMGN2 | HIST1H3B | TTC9B | ZFP36L 2 | SLC9A3R1 | CLDN6 | LINC00682 | AC092957.1 | PCDH1 8 | HIST1H1A | PRND |

(continued)

| Cortical neuron (Radial Glia (RG)) | Cortical neuron (Glutamatergic neuron (GN)) | Cortical neuron (Cajal retzius cell (CR)) | GABAergic neuron-1 | GABAergic neuron-2 | GABAergic neuron-3 | Choroid plexus (ChP) | CNS fibroblast-1 | CNS fibroblast-2 | Caudal neuron | Neural crest-1 | Neural crest-2 | Neural crest-3 | Endothelial cells |
|---|---|---|---|---|---|---|---|---|---|---|---|---|---|
| CENPF | CCSAP | PIDD1 | HMGB2 | DLX6-AS1 | AC004158.1 | SULF1 | IFITM3 | MT1E | ZIC1 | GRID2 | ID2 | LGALS1 | FLT1 |
| TOP2A | PDE1A | EMX2 | TUBA1B | MKI67 | DLX6 | PLS3 | IGFBP3 | EPCAM | PHOX2B | LINC02381 | CDC42EP5 | MMP2 | ESAM |
| UBE2C | NEUROD1 | MYT1L | DLX2 | BIRC5 | GAD2 | OTX2 | KRT19 | IGFBP7 | HOXB4 | NCKAP5 | TPM2 | ERBB3 | COL4A1 |
| CKB | NPTX1 | OLFM1 | HIST1H4D | NPY | NRGN | TRIM67 | FN1 | EMP2 | HOTAIRM1 | AL139246.5 | LRRC17 | TFAP2A | IFITM3 |
| LINC01551 | NXPH4 | THSD7A | DLX5 | GSX2 | SCG5 | PCP4 | KRT8 | ANXA3 | CACNA2D1 | TOP2A | LIMA1 | COL4A2 | RNASE1 |
| HIST1H3D | NTS | RTN1 | ASCL1 | UBE2C | MYT1L | CCK | COL5A1 | TGFBI | MAB21L2 | CDC25B | MECOM | LAMC1 | TIMP3 |
| HIST1H4C | NEUROG2 | INA | DLX6-AS1 | ASPM | SCG3 | EMX2 | TIMP1 | FOS | HOXB2 | BOC | EPB41L2 | ARPC1B | PRSS23 |
| TCF7L1 | OLFM1 | SPINK5 | PBK | LIX1 | RBFOX1 | CA2 | PLTP | AHNAK | UNCX | IRX2 | COL1A2 | CDH19 | CD34 |
| PCLAF | PRDM8 | NHLH1 | ASPM | PBK | SLC8A1 | PRTG | KRT18 | EPAS1 | NRN1 | MASP1 | TES | SLITRK6 | PLVAP |
| AMBN | CORO2B | CXCR4 | PRC1 | SCRG1 | RAB3A | CLDN5 | COL6A2 | ANKRD1 | HOXA3 | SLC1A3 | CCDC80 | VIM | RAMP2 |
| GAS1 | TP53I11 | TAGLN2 | ZEB2 | MT3 | CDK5R1 | CLU | ANXA2 | MT2A | TFAP2B | WLS | HOXA10 | HIST1H3D | CRIM1 |
| CREB5 | ZFPM2 | LHX5 | CDK1 | RORB | GRIN2B | PMCH | OLFML3 | CCN1 | EBF3 | CXCR4 | TGFB2 | CRYL1 | FN1 |
| HIST1H1B | PCDH9 | TP73 | TPX2 | CDO1 | CELF4 | CRABP2 | COL6A1 | LGALS3 | GDAP1L1 | ARL4A | MIR100HG | TAC1 | EGFL7 |
| HESS | NELL2 | WNT7B | DLX1 | ADGRV1 | FOXP2 | MAF | TMEM88 | CTHRC1 | NHLH1 | FZD10 | CXCL12 | RXRG | ACSM3 |
| EMX2 | SRRM4 | NRN1 | MAD2L1 | LINC01896 | GABRB3 | BMP7 | IL6ST | COL12A1 | ELAVL2 | ZIC4 | HMGA2 | SCAN | TIE1 |
| SOX9 | SCG3 | RGS16 | NPY | HOPX | DCLK1 | MSX1 | CXCL14 | APOE | DCC | RND2 | MIR99AHG | PRSS23 | VAMPS |
| GNAI2 | DCC | IGSF9 | HMGB1 | PRC1 | MTSS1 | FOS | S100A11 | SLC2A3 | HOXA4 | TPBG | CDH11 | DST | CALCRL |

(continued)

| Cortical neuron (Radial Glia (RG)) | Cortical neuron (Glutamatergic neuron (GN)) | Cortical neuron (Cajal retzius cell (CR)) | GABAergic neuron-1 | GABAergic neuron-2 | GABAergic neuron-3 | Choroid plexus (ChP) | CNS fibroblast-1 | CNS fibroblast-2 | Caudal neuron | Neural crest-1 | Neural crest-2 | Neural crest-3 | Endothelial cells |
|---|---|---|---|---|---|---|---|---|---|---|---|---|---|
| IFI44L | EPB41L3 | DCC | CKS1B | ETV1 | NRXN3 | CACHD1 | COL15A1 | ARHGAP29 | EBF1 | GYPC | IGF2 | ITM2C | S100A16 |
| NAP1L1 | SLC17A7 | FNDC5 | HIST1H4E | TPX2 | MEF2C | EGR1 | PDPN | PRSS23 | KLHL35 | PAX3 | NRP2 | ZEB2 | CAVIN1 |
| PTTG1 | ST18 | SVEP1 | BIRC5 | RAB31 | TMEM35A | RMST | B2M | LY6E | HOXC4 | MEIS1 | SFRP2 | KCTD12 | IGF2 |
| QKI | EOMES | SIPA1L2 | PFN2 | TMEM158 | SERTAD4 | CXCL14 | HAND1 | CCDC80 | AC008522.1 | IRX1 | MFAP2 | COL4A1 | HLA-E |
| CKS2 | NSG2 | ELAVL2 | HIST1H1E | TNC | SP9 | SLC7A8 | PODXL | SCUBE3 | LAMPS | DLL1 | SIX1 | HMG20B | HSPG2 |
| DOK5 | EMX1 | LMO2 | SHTN1 | CDK1 | ISLR2 | ATP1A2 | COL5A2 | L1TD1 | NHLH2 | ZNF503 | IL11RA | LMNA | IFITM2 |
| PAX6 | CAPS | SCG3 | DLGAP5 | SCGN | DPF1 | ANKRD37 | HAND2 | KCNMA1 | PRPH | EDNRB | ZFHX4-AS1 | CLIC1 | MECOM |
| HEY1 | SYT4 | SAMD3 | AURKB | SMOC1 | KIF21B | ZIC2 | SERPINH1 | TNC | FNDC5 | HOXB3 | PCOLCE | NR2F2 | ADGRL4 |
| NUSAP1 | SPINK5 | CACNA2D1 | UBE2C | CENPE | ATP1A3 | CDO1 | AQP1 | TSTD1 | POU3F1 | MKI67 | C1orf54 | SLC25A5 | COL4A2 |
| TMEM161B-AS1 | NSMF | CDK5R1 | KIF11 | ATAD2 | SCN3B | SPRY1 | RRBP1 | TAGLN | IRX2 | PCDH18 | HMCN1 | AHR | NRP1 |
| HIST1H3B | ANK3 | KLF7 | C11orf96 | NUF2 | SLITRK6 | PIFO | EGFL6 | FSTL1 | POU3F2 | LRIG1 | PCDH10 | RHOC | BST2 |
| SMC4 | MYT1L | DMRTA2 | UBE2S | NES | DNM3 | NR2F2 | SLC40A1 | CXCL12 | UBE2QL1 | MGST1 | TBX3 | EDNRB | LIMS1 |
| DMRTA2 | FSTL5 | SEZ6L | GTSE1 | CDC20 | CXXC4 | LGI1 | CHN1 | TPM2 | SCRT2 | EPHA3 | ITM2C | MMP17 | MYH9 |
| NASP | EBF2 | CPNE4 | CENPE | STK39 | PCDH10 | TSHZ2 | TFPI2 | VTCN1 | SCG3 | NRARP | FGFR1 | PMP22 | PLXND1 |
| LINC404fi1 | CELF4 | SNAP25 | CKS2 | MOXD1 | SYT5 | DCT | EMP3 | SPATS2L | SKAP2 | JAG1 | RA834 | MIA | IFI16 |
| ASPM | B3GAT1 | GNG3 | FBXO5 | PBX3 | ACTL6B | EMX2OS | COL21A1 | IGDCC3 | NRCAM | NAV2 | IRX3 | FAM210B | ARHGAP29 |

(continued)

| Cortical neuron (Radial Glia (RG)) | Cortical neuron (Glutamater gic neuron (GN)) | Cortical neuron (Cajal retzius cell (CR)) | GABAergic neuron-1 | GABAergic neuron-2 | GABAergic neuron-3 | Choroid plexus (ChP) | CNS fibroblast -1 | CNS fibroblast -2 | Caudal neuron | Neural crest-1 | Neural crest-2 | Neural crest-3 | Endothelial cells |
|---|---|---|---|---|---|---|---|---|---|---|---|---|---|
| HIST1H1C | EPHA 5 | CADM2 | CKAP2 | PCLAF | SCN3A | KLHDC 8B | PITX1 | CLDN4 | SNAP25 | HOXB2 | EVA1B | CST3 | ECSCR |
| CRYGD | NHLH 2 | SHLHE2 2 | H2AFX | CDKN3 | LINGO1 | GJA1 | BST2 | PERP | CBLN1 | AL35909 1.1 | ARHGAP 28 | APOD | EPAS1 |
| H2AFX | DLL3 | CABP7 | KNL1 | NTM | CELF3 | SDC2 | CDKN1C | S100A 13 | MYT1 | APCDD1 | CAST | GAS7 | CLEC14 A |

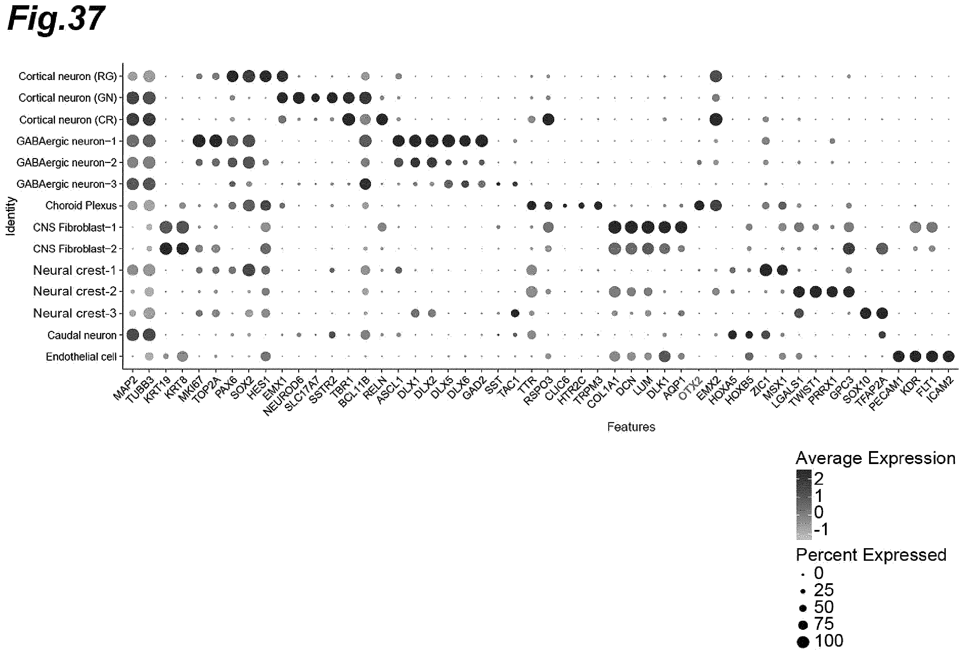

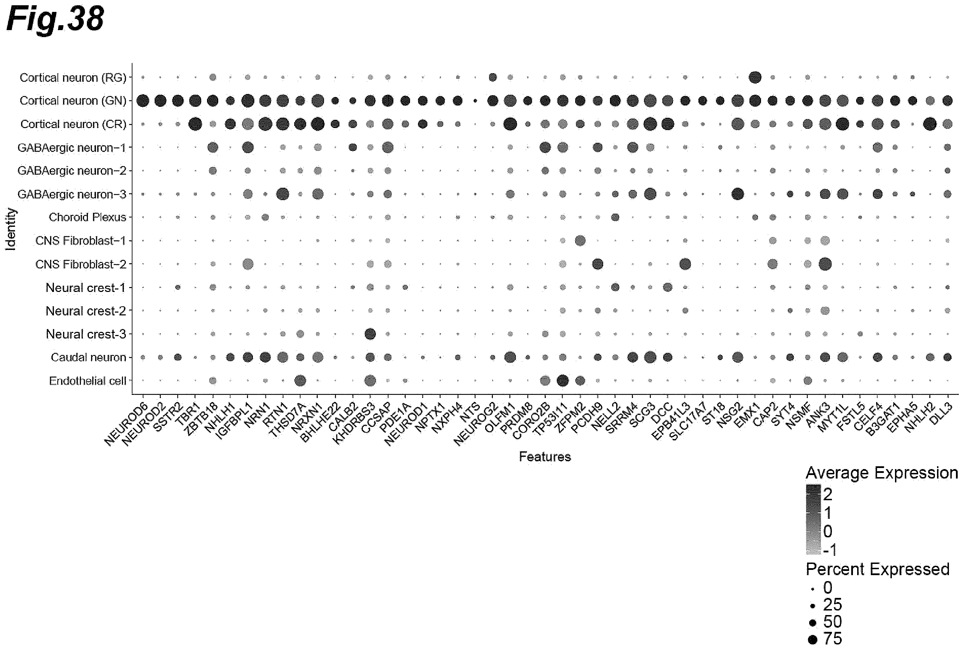

Figures 37 and 38 show dot plot representation of expression profiles of genes characteristic to different clusters and known marker genes in the single-cell gene expression data. The ordinate shows clusters and the abscissa shows genes. The size of each circle indicates the proportion of expressing cells in percentage to the total number of cells in the corresponding cluster. The color gradation represents the mean of gene expression levels in cells in a cluster.

MAP2 and TUBB3 were analyzed as markers for neural cells. KRT19 and KRT8 were analyzed as markers for epithelial cells. MKI67 and TOP2A were analyzed as markers for proliferative cells. PAX6, SOX2, and HES1 were analyzed as markers for radial glia (RG). EMX1 was analyzed as a marker for the dorsal region of the forebrain. NEUROD6 and SLC17A7 were analyzed as markers for glutamatergic neurons. SSTR3 is a somatostatin receptor, the expression of which is known to be found also in glutamatergic neurons. TBR1 and BCL11B were analyzed as markers for the deep layer, a precursor of the cerebral cortex layers V and VI. RELN was analyzed as a marker for Cajal Retzius cells. ASCL1, DLX1, DLX2, DLX5, and DLX6 were analyzed as markers the expression of which is found in the course of differentiation into GABAergic neurons. GAD2 was analyzed as a marker for GABAergic neurons. SST and TAC1 were analyzed as markers the expression of which is found in subtypes of GABAergic neurons. TTR, RSPO3, CLIC6, HTR2C, and TRPM3 were analyzed as markers the expression of which is found in the course of development of choroid plexuses. COL1A1 was analyzed as a marker for CNS fibroblasts. DCN, LUM, and DLK1 are genes that are expressed in stromal cells. AQP1 has been reported to be expressed in choroid plexuses and arachnoid granules. OTX2 and EMX2 are region-specific markers, and known to be expressed in telencephalic choroid plexuses. HOXA5 and HOXB5 were analyzed as markers for caudal neurons. ZIC1, MSX1, LGALS1, TWIST1, PRRX1, GPC3, SOX10, and TFAP2A were analyzed as markers the expression of which is found in the course of differentiation into neural crests. Of those, LGALS1, TWIST1, PRRX1, and GPC3 are genes involved in epithelial-mesenchymal transition (EMT), and SOX10 and TFAP2A are markers that are expressed in mature neural crests. PECAM1, KDR, FLT1, and ICAM2 were analyzed as markers for vascular endothelial cells.

In selection of genes to be analyzed, first, genes for each of which the proportion of expressing cells in the cluster of "Cortical neuron (GN)" was twice or more that is higher than those in other clusters were selected. Next, top 50 genes with highest fold change of the mean of expression levels in the cluster of "Cortical Neuron (GN)" to those in other clusters were selected. Finally, in single-cell gene expression analysis for 3 lots of cerebral organoids before DAPT treatment and 3 lots of cerebral cortical cell aggregates after DAPT treatment, 46 genes the expression of which was detected from all the lots were selected as those to be analyzed.

The clusters of cerebral cortical nerve cells (Cortical neurons) were expressing EMX1, which is a marker for the forebrain. Among them, "Radial glia (RG)" was expressing LHX2, and SOX2 and PAX6, which are genes essential for controlling radial glia. "Glutamatergic neuron (GN)" was expressing TBR1 (Robert F. Hevner, Tbr1 Regulates Differentiation of the Preplate and Layer 6, Neuron, 2001) and NEUROD6 (Tutukova S et al., The Role of Neurod Genes in Brain Development, Function, and Disease, Frontiers in Molecular Neuroscience, 2021), which are transcription factors characteristic to deep layer neurons and pyramidal neurons, the glutamate transporter SLC17A7 (VGluT1), and BCL11B (Ctip2). "Cajal Retzius cell (CR)" was expressing RELN, which is a marker gene for Cajal-Retzius cells, TBR1, and so on.

The clusters GABAergic neurons were expressing the glutamate decarboxylase GAD2 in common. In the clusters "GABAergic neuron-1, 2" among those, the expression of the proliferation marker MKI67 (Ki67) and ASCL1, the expression of which is found in the early stage of differentiation (VZ), was highly appreciable. In the cluster "GABAergic neuron-3", DLX5 and DLX6, the expression of which is found after the middle stage of differentiation (SVZ), were expressed.

The cluster Choroid plexus was highly expressing TTR (transthyretin), which is a marker for the choroid plexus epithelium. In view of the presence of telencephalic choroid plexuses and hindbrain-type choroid plexuses, the result that EMX2 and BMP7 were co-expressed suggested that the choroid plexuses were telencephalic ones associated with the cerebral cortex.

The clusters CNS fibroblasts were highly expressing COL1A1, which is a marker for fibroblasts associated with central nerves. At the same time, expression of AQP1 and others was also found, and hence some of those cells are inferred to be fibroblasts associated with choroid plexuses.

In the clusters Neural crests, expression of various genes the expression of which is found in the course of differentiation into neural crest cells (NCCs) (Simoes-Costa M et al., Establishing neural crest identity: a gene regulatory recipe, Development, 2015) was found. In the cluster "Neural crest-1", expression of a ZIC gene cluster and Msx1, the expression of which is found in the neural plate border as the origin of NCCs, and others was found. In the cluster "Neural crest-2", expression of an ID gene cluster in the downstream of Smad signaling, the expression of which is found in the early stage of differentiation into NCCs, and epithelial-mesenchymal transition (EMT)-related genes (LGALS1, TWIST1, PRRX1, GPC3 (Fazilaty H et al., A gene regulatory network to control EMT programs in development and disease, 2019) was found. In "Neural crest-3", expression of SOX10, TFAP2A, which is expressed in the migration stage of NCCs, and others was found.

The cluster of endothelial cells was expressing many markers for vascular endothelial cells, including KDR (VEGFR-2), PECAM1, FLT1, and ICAM2 (Gonchalov et al., Markers and Biomarkers of Endothelium: When Something

52

Is Rotten in the State, Oxidative Medicine and Cellular Longevity, 2017).

In the cluster Caudal neuron, expression of not only TUBB3, which is a marker for neurons, but also many HOX genes including HOXA5 and HOXB5, which control caudal regionalization, was found.

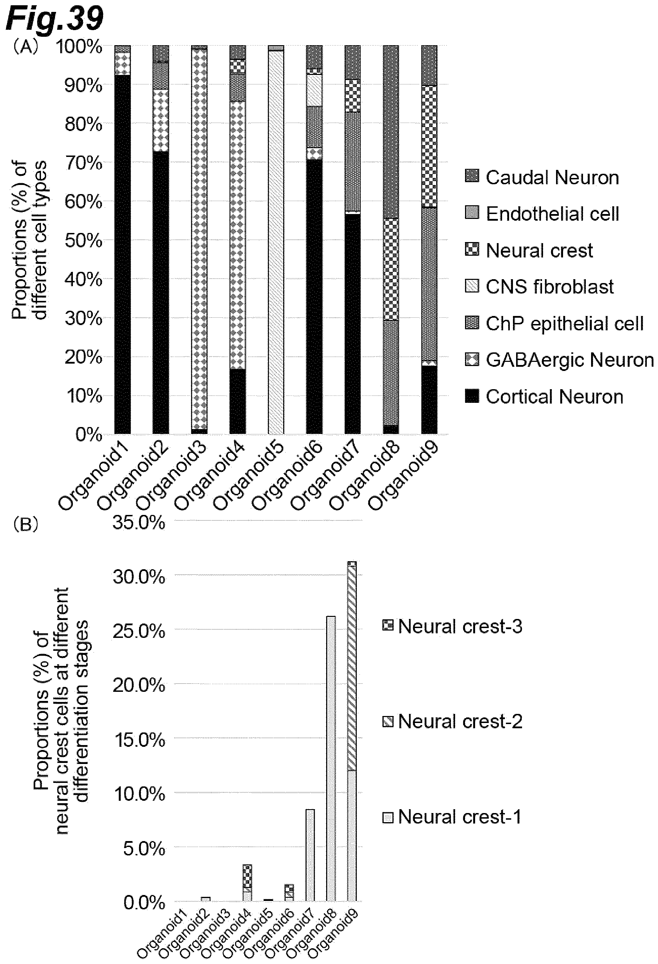

Figure 39(A) shows the proportions of different cell types in the nine organoids, and Figure 39(B) shows the proportions of neural crest cells in different differentiation stages in Neural crests. Organoid 9, which included pigment cells, had a higher proportion of Neural crests than other organoids. Especially, the proportion of "Neural crest-2", which was a cell population expressing a gene cluster involved in EMT, was high, and thus it was found that many actively migrating neural crests were included.

9-3. Analysis of Expression of Different Markers by Immunostaining

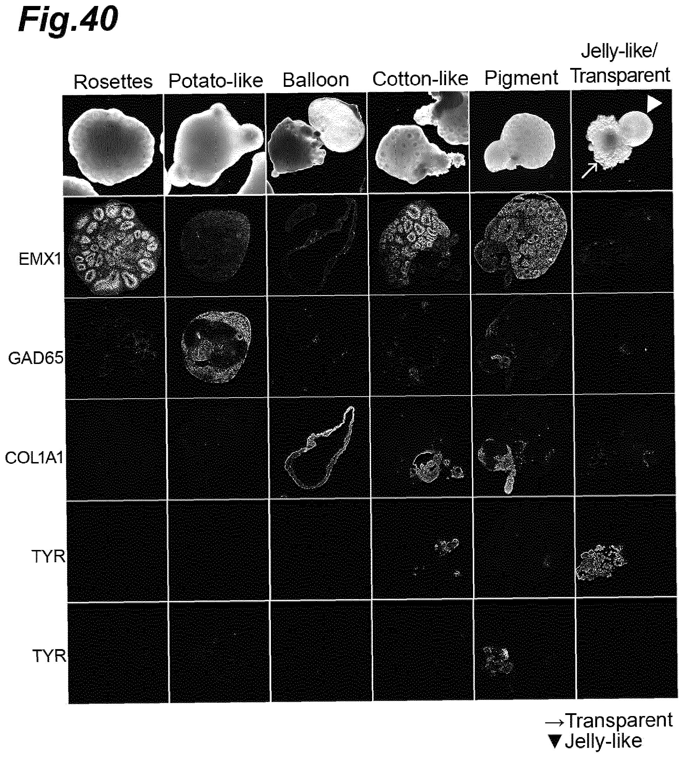

Immunostaining of representative marker proteins was performed for organoids in each group. Figure 40 shows the results.

EMX1, which is a marker for the forebrain, was found to be specifically expressed in regions in which rosette structure was found (rosettes). GAD65 (GAD2), which is a marker for GABAergic neurons, was found to be specifically expressed in a region of the "potato-like tissue". COL1A1, which is a marker for ECM fibroblasts, was found to be expressed in peripheral regions of balloon-like tissue, cotton-like tissue, and pigments. A choroid plexus specific-marker was expressed in transparent tissue. The melanocyte marker TYR was found to be expressed in the organoid "Pigment", in which pigmentation was found. It was confirmed from these results that each of the markers identified in the single-cell gene expression analysis was surely expressed in regions of the corresponding characteristic structure.

9-4. Analysis of Expression of Different Marker Genes by RT-qPCR

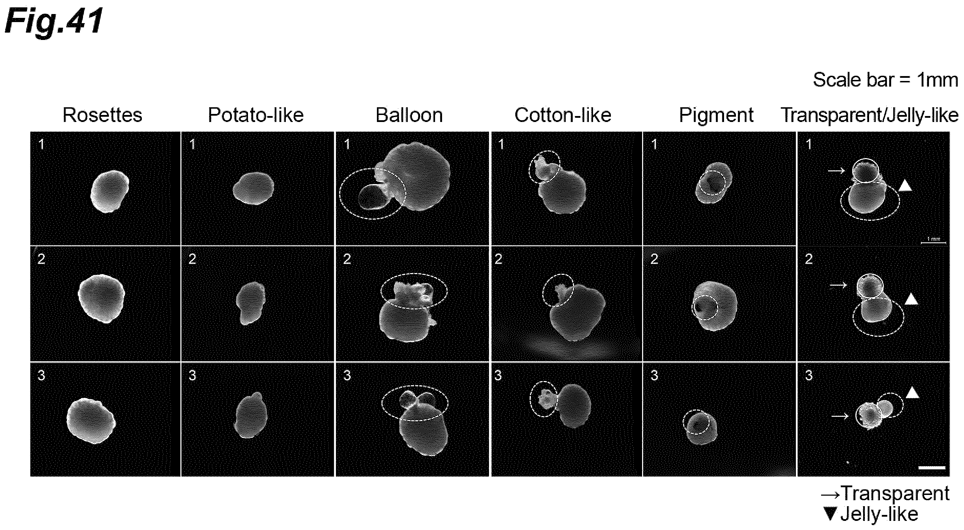

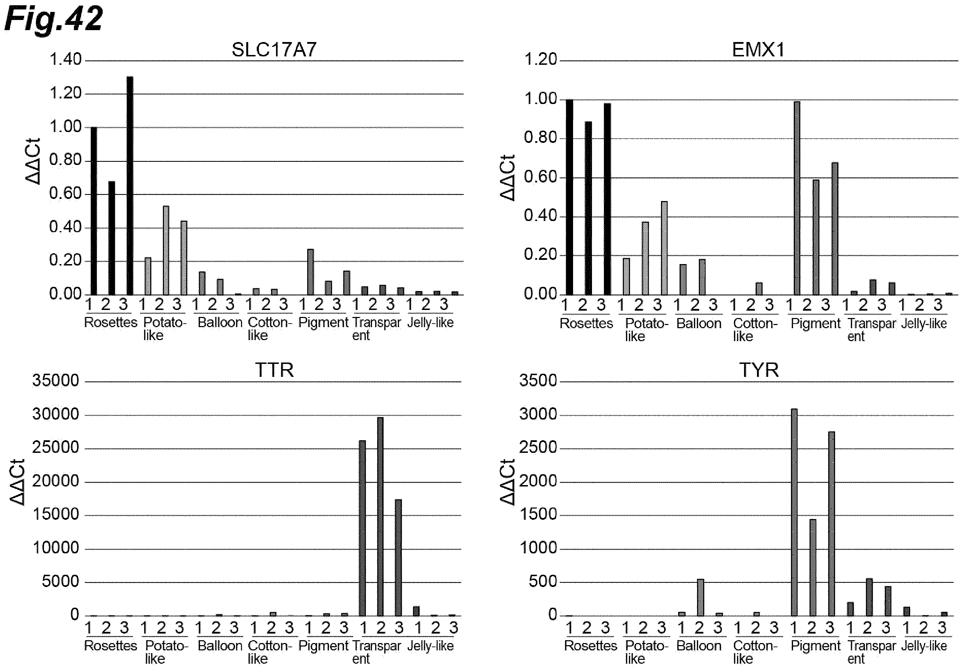

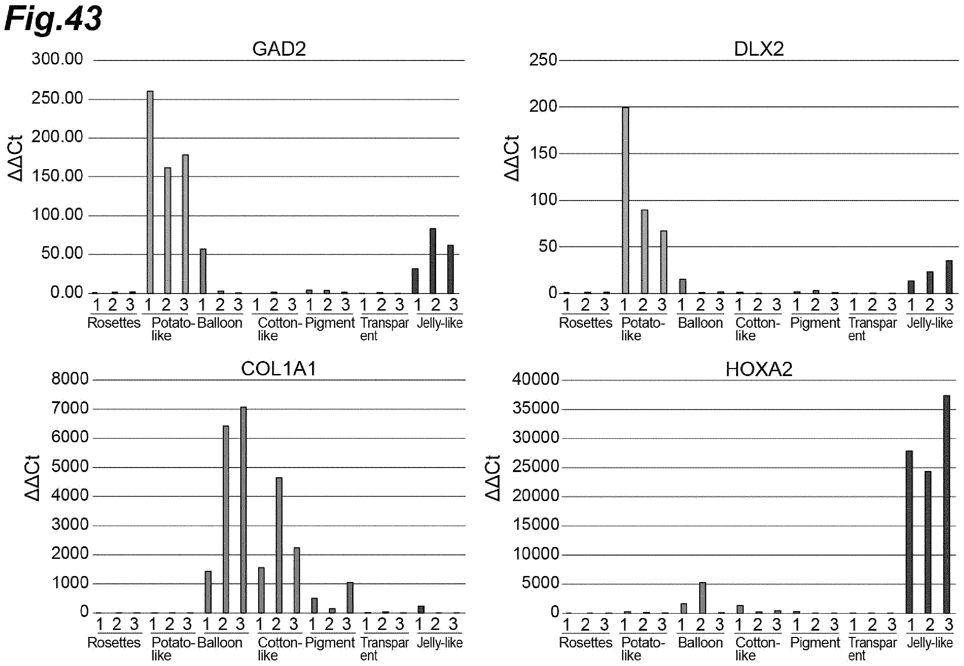

Culture was performed in the same manner as in Example 1 from -Day 7 to Day 35. Three organoids were obtained per group, and bright field images were acquired by photographing with an inverted microscope (Leica DMS1000) (Figure 41). For the organoids, expression of marker genes was analyzed by an RT-qPCR method. The analysis results are shown in Figures 42 and 43.

VGluT1 and EMX1, which were identified as markers for cerebral cortical nerve cells (cortical neurons), were found to be expressed in the organoid of Rosettes. DLX2 and GAD2, which were identified as markers for GABAergic neurons, were found to be highly expressed in the organoids of Potato-like. TYR, which is a marker for melanocytes, was found to be highly expressed in the organoids of Pigment. COL1A1, which is a marker for CNS fibroblasts, was found to be expressed in balloon-like tissue and cotton-like tissue. HOXA2, which is a marker for caudal neurons, was found to be expressed in jelly-like tissue.

9-5. Analysis of Cerebral Organoids and Cerebral Cortical Cell aggregates by Single-Cell Gene Expression Analysis

Culture was performed in the same manner as in Example 1 from -Day 7 to Day 35. From organoids obtained through three operations of induction of differentiation, organoids of Rosettes were visually selected. Thereafter, the cerebral organoids were treated with DAPT for 3 days by the method described for step (ii) in Example 4 to induce cerebral cortical cell aggregates ("DAPT+"). As a control group, a group without addition of DAPT in step (ii) was established (cerebral organoids: "DAPT-"). With those cells, single-cell gene expression analysis was carried out by the same method as in 9-2.

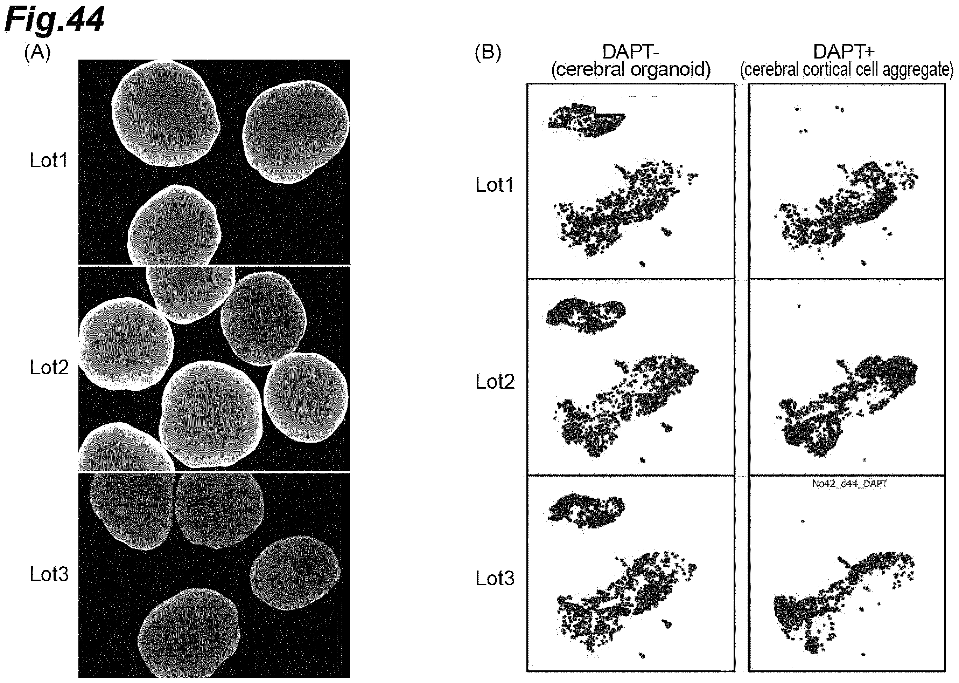

The morphologies of the organoids of Rosettes from each operation of induction of differentiation (Lot 1, Lot 2, Lot 3) were observed with an inverted microscope; Figure 44(A) shows the representative bright field images. Figure 44(B) shows UMAP plots of single-cell gene expression analysis. (Rosettes Lot 1, Rosettes Lot 2, Rosettes Lot 3)

It was found that the organoids (cerebral organoids) obtained under the DAPT- conditions were classified into two clusters and two cell populations were present therein (Figure 44(B)). It was found that the organoids (cerebral organoids) obtained under the DAPT+ conditions were collectively forming one cluster, constituting one cell population. The distributions of the cells in the three production lots were overlapping, from which it was found that the difference among production lots was small and equivalent cell populations were successfully obtained with good reproducibility.

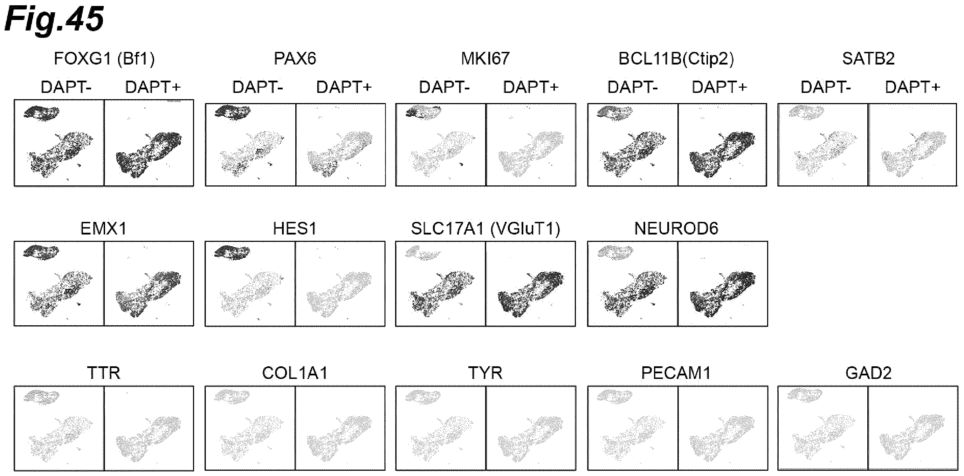

For the above organoids, expression of the marker genes identified in the above was further analyzed (Figure 45). The cerebral organoids (DAPT-) were classified into two clusters, and the right cluster was highly expressing SLC17A7 and NEUROD6, which are markers for glutamatergic neurons, and Ctip2, which is a marker for deep layer neurons, suggesting that glutamatergic neurons and progenitor cells thereof were include therein. The left cluster was highly expressing PAX6 and MKI67, which are markers for radial glia, suggesting the presence of highly proliferative radial glia. Expression of Satb2, which is a marker for upper neurons, was scarcely found, suggesting that most cells were cerebral cortex layer V, VI progenitor cells. TTR, COL1A1, TYR, and PECAM1, which are markers identified herein for non-target cells, were hardly detected. It was found from those results that cells of the cerebral cortex can be obtained and contamination with non-target cells can be reduced by selecting organoids entirely having rosette structure on the basis of morphology or eliminating organoids of other morphologies.

The cerebral cortical cell aggregates (DAPT+) were collectively forming one cluster, and expressing SLC17A7 (VGluT1), NEUROD6, and Ctip2, but the cell population with expression of Pax6 and MKI67, which are markers for non-target cells, largely shrunk. TTR, COL1A1, TYR, and PECAM1 were hardly detected. It was found from these results that cerebral cortical cell aggregates including glutaminergic neurons with high purity can be obtained by performing DAPT treatment.

Each of the genes shown in Table 6 can also be used as a marker for any of cerebral cortical neural progenitor cells (included in cerebral cortical nerve cells in a broad sense, and corresponding to radial glia (RG)), cerebral cortical nerve cells (including glutamatergic neurons (GN) and Cajal-Retzius cells (CR)), GABAergic neural cells, choroid plexuses (ChP), central nervous system fibroblasts, neural crest cells, vascular endothelial cells, and caudal neural cells.

Markers for cerebral cortical neural progenitor cells and cerebral cortical nerve cells (including glutamatergic neurons and Cajal-Retzius cells) can be each used as a marker for cerebral organoids because those cells are included in cerebral organoids.

In particular, genes expressed in cerebral cortical nerve cells, specifically, genes expressed in glutamatergic neurons or Cajal-Retzius cells can be each used as a marker for the cerebral cortical cell aggregate of the present invention or the high-purity cerebral cortical cell aggregate of the present invention.

Among the 50 genes belonging to clusters of cerebral cortical nerve cells (except radial glia) (Table 6), three genes (EOMES, SPINK5, EBF2) gave low expression levels in organoids after DAPT treatment, and hence it was found that the residual 47 genes are suitable as markers for the cerebral cortical cell aggregate of the present invention or the high-purity cerebral cortical cell aggregate of the present invention. In that gene list, NEUROD6, NEUROD2, SSTR2, TBR1, NRXN1, BHLHE22, NEUROD1, NEUROG2, SLC17A7, and EMX1 are known to be expressed in the forebrain dorsal region, which is a region in which the cerebral cortex develops, the presumptive area of the cerebral cortex layer II to layer VI (cortical plate), and glutamatergic neural cells, and the other 37 genes are those newly identified as markers for cerebral cortical nerve cells.

That is, whether the cerebral cortical cell aggregate or high-purity cerebral cortical cell aggregate specified in the present application includes cerebral cortical nerve cells, which are target cells, can be evaluated by examining the presence or absence of expression of at least one, at least two, at least three, or at least five genes selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3, or the expression levels thereof. More preferably, evaluation can be performed by examining the presence or absence of expression of SLC17A7, NEUROD6, and EMX1, or the expression levels thereof.

In addition, it was found that the gene clusters that are expressed in cerebral cortical neural progenitor cells (radial glia (RG)), GABAergic neural cells (GABAergic neurons), choroid plexuses (ChP), central nervous system fibroblasts (CNS fibroblasts), neural crest cells (neural crests), vascular endothelial cells, or caudal neural cells (caudal neurons) shown in Table 6 (hereinafter, referred to as non-target-cell gene clusters) can be used as markers for non-target cells in the cerebral cortical cell aggregate or high-purity cerebral cortical cell aggregate of the present invention to examine whether the amount of non-target cells is equal to or less than a reference value.

That is, whether the cerebral cortical cell aggregate or high-purity cerebral cortical cell aggregate specified in the present application includes none of the non-target cells or includes non-target cells in an amount equal to or less than a reference value can be evaluated on the basis of, as a criterion, whether the expression levels of at least one, at least two, or at least three genes selected from the non-target-cell gene clusters in Table 6 indicate substantially no expression or are equal to or less than a reference value.

Specifically, evaluation can be performed on the basis of, as a criterion, whether the expression levels of at least one, at least two, or at least three genes indicate substantially no expression or are equal to or less than a reference value, the genes selected from LHX2, SOX2, and PAX6, which have been identified as markers for radial glia, GAD2, DLX1, DLX2, DLX5, and DLX6, which have been identified as markers for GABAergic neural cells, TTR and TRPM3, which have been identified as markers for choroid plexuses, COL1A1, which has been identified as a marker for central nervous system fibroblasts, the ZIC gene cluster, Msx1, the ID gene cluster in the downstream of Smad signaling, and the epithelial-mesenchymal transition (EMT)-related genes (TWIST1, PRRX1, GPC3, Sox 10, and TFAP2), which were identified as markers for neural crest cells, many markers for vascular endothelial cells including KDR (VEGFR-2), PECAM1, and CDH5 (VE-cadherin), which have been identified as markers for vascular endothelial cells, and TUBB3, which has been identified as a marker for caudal neural cells, and also from many HOX genes that control caudal regionalization. The usage of the markers shown in Table 6 is not limited to them, and the markers can be used for various purposes through selection of appropriate markers according to the application and measurement of the expression.

More preferably, one or more genes selected from the group consisting of GAD2, COL1A1, TYR, TTR, and HOXA2 in Table 6 are substantially unexpressed, or the expression level is equal to or less than a reference value.

Cerebral organoids produced by the production method specified in the present invention are not necessarily limited in applications, and may be used for purposes as shown in the following.

<Application for Cerebrum (Substance Screening)>

The influence of a low-molecular-weight compound, an antibody, a nucleic acid, or a substance of another type may be examined by adding it to a cerebral organoid produced by the production method of the present invention. That is, the cerebral organoid can be used for the purpose of clarifying the influence, toxicity, and usefulness of a low-molecular-weight compound, an antibody, or a substance of another type. In this case, examination can be performed on the influence on organized neural cells or organized neural networks formed in the cerebral organoid. In particular, the production method and screening method specified in the present invention for a cerebral organoid enable mass production of cerebral organoids relatively homogenous in terms of expression profile or shape or the like, or tissue configuration. In doing so, the quality assessment method described in section 13 for a cerebral organoid may be used to objectively evaluate the quality of cerebral organoids. Use of such cerebral organoids relatively homogenous in terms of expression profile or shape or the like, or tissue configuration allows replicated test to be carried out with ease, and reproducibility, which was previously difficult to achieve, can be achieved with ease.

<Application for Cerebrum (Cerebral Organoid with Disease)>

The above substance screening may be applied to cerebral organoids for a specific disease, the cerebral organoids induced from pluripotent stem cells derived from cells of specific genotype. In this case, an optimum step can be appropriately employed according to the characteristics of the original pluripotent stem cells in producing cerebral organoids by using the method described in the above section "2" for producing a cerebral organoid. In particular, cerebral organoids induced from pluripotent stem cells derived from cells collected from a patient, for example, affected by schizophrenia, bipolar disorder, autism spectrum disorder, Alzheimer's disease, dementia, or microcephaly (cerebral organoids with mental disorder) may be produced for use not only in examining the effect of a drug but also in elucidating the mechanism of the disease. More specifically, comparison of glutamatergic neurons or the like as excitatory neural cells, GABAergic neurons as inhibitory neural cells, or glial cells with those of healthy individuals, and analysis of cell properties including gene expression, protein expression, metabolic condition, and electrophysiological characteristics are included.

## Claims

1. A method for producing a cerebral organoid from a pluripotent stem cell in the absence of a sustentacular cell, comprising:

   (1) a step of culturing the pluripotent stem cell in a culture solution, wherein the culture solution is substantially free of bFGF and provokes substantially no TGFβ signal; and
   (2) a step of inducing the cell obtained in step (1) to differentiate into a neural cell.

2. The method according to claim 1, wherein step (2) comprises:

   (2a) a step of subjecting the cell obtained in step (1) to suspension culture in a culture solution containing a TGFβ signaling inhibitor and a Wnt signaling inhibitor to obtain a cell aggregate; and
   (2b) a step of subjecting the cell aggregate obtained in step (2a) to suspension culture in a culture solution substantially free of a TGFβ signaling inhibitor and a Wnt signaling inhibitor to obtain a cerebral organoid.

3. The method according to claim 2, wherein the suspension culture in step (2a) is static culture.

4. The method according to claim 2 or 3, wherein the suspension culture in step (2b) is shaking culture.

5. The method according to any one of claims 1 to 4, wherein culture period in step (1) is less than 3 days.

6. The method according to any one of claims 1 to 5, wherein culture period in step (1) is 12 hours or more and 2 days or less.

7. The method according to any one of claims 1 to 6, wherein the culture solution in step (1) contains a TGFβ signaling inhibitor selected from the group consisting of SB431542, A-83-01, and XAV-939.

8. The method according to any one of claims 2 to 7, wherein the culture solutions in step (1), step (2a), and step (2b) are each a serum-free culture solution.

9. The method according to any one of claims 1 to 8, wherein the pluripotent stem cell is a human induced pluripotent stem cell or a human embryonic stem cell.

10. The method according to any one of claims 1 to 9, further comprising:
(3) a step of screening a cerebral organoid from a plurality of cell aggregates obtained in step (2) on the basis of, as indices, one or more selected from the group consisting of shape, internal structure, size, surface coloring or patterning, and gene expression of a cell aggregate.

11. A cell culture produced by using the method according to any one of claims 1 to 9, wherein

the cell culture comprises a plurality of spherical cell aggregates, and
a proportion of cerebral organoids in the plurality of spherical cell aggregates is 40% or more.

12. The cell culture according to claim 11, wherein a proportion of a cerebral cortex-like structural body occupying each of the cerebral organoids is 40% or more.

13. The cell culture according to claim 12, wherein each of the cerebral organoids is a cell aggregate further having one or more characteristics selected from the following (1) to (5):

(1) being a spherical cell aggregate;
(2) having a cerebral cortex-like structural body inside of the cell aggregate;
(3) having no pigmentation in a surface;
(4) having none of cystoid shape, protruding shape, and balloon-like shape in a part of the cell aggregate; and
(5) expressing at least one marker selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

14. A cerebral cortical cell aggregate, wherein

(a) number of cells positive for a proliferation marker is 10% or less of total number of cells,
(b) number of cells positive for one or more markers selected from the group consisting of a neuronal marker, a cortical layer V/VI marker, and a forebrain marker is 70% or more of total number of cells, and
(c) the cerebral cortical cell aggregate includes substantially no neuroepithelium or cerebral cortex-like structure.

15. The cerebral cortical cell aggregate according to claim 14, wherein the proliferation marker in (a) is Ki67, and the neuronal marker, the cortical layer V/VI marker, and the forebrain marker in (b) are βIII-tubulin, Ctip2, and FOXG1, respectively.

16. The cerebral cortical cell aggregate according to claim 14 or 15, further expressing at least one marker selected from the group consisting of:
(d) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

17. The cerebral cortical cell aggregate according to claim 16, expressing SLC17A7.

18. The cerebral cortical cell aggregate according to any one of claims 15 to 17, substantially unexpressing one or more genes selected from the group consisting of GAD2, COL1A1, TYR, TTR, and HOXA2.

19. A method for producing a cerebral cortical cell aggregate from a pluripotent stem cell in the absence of a sustentacular cell, comprising:

(i) a step of obtaining a cerebral organoid from the pluripotent stem cell; and
(ii) a step of culturing the cerebral organoid obtained in step (i) in a culture solution containing a Notch signaling inhibitor to obtain a cerebral cortical cell aggregate.

20. The method according to claim 19, wherein, in step (i), a cerebral organoid is obtained from a pluripotent stem cell by the method according to any one of claims 1 to 10.

21. A high-purity cerebral cortical cell aggregate, wherein

(A) number of cells positive for a proliferation marker is 5% or less of total number of cells,
(B) number of cells positive for one or more markers selected from a neuronal marker, a cortical layer V/VI marker, and a forebrain marker is 70% or more of total number of cells, and
(C) the high-purity cerebral cortical cell aggregate includes substantially no neuroepithelium or cerebral cortex-like structure.

22. The high-purity cerebral cortical cell aggregate according to claim 21, wherein the proliferation marker in (A) is Ki67, and
the neuronal marker, the cortical layer V/VI marker, and the forebrain marker in (B) are βIII-tubulin, Ctip2, and FOXG1, respectively.

23. The high-purity cerebral cortical cell aggregate according to claim 21 or 22, expressing at least one gene selected from the group consisting of:
(D) NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3.

24. The high-purity cerebral cortical cell aggregate according to claim 23, expressing one or more genes selected from the group consisting of SLC17A7, NEUROD6, and EMX1.

25. The high-purity cerebral cortical cell aggregate according to any one of 21 to 24, substantially unexpressing one or more genes selected from the group consisting of GAD2, COL1A1, TYR, TTR, and HOXA2.

26. A method for producing a high-purity cerebral cortical cell aggregate from a pluripotent stem cell in the absence of a sustentacular cell, comprising:

(i) a step of obtaining a cerebral organoid from the pluripotent stem cell;
(ii) a step of culturing the cerebral organoid obtained in step (i) in a culture solution;
(iii) a step of dispersing the cell culture obtained in step (ii) into single cells or two- to five-membered cell clumps; and
(iv) a step of culturing the cell culture obtained in step (ii) or the cell population obtained in step (iii) in a culture solution containing one or more neurotrophic factors, ascorbic acid, and a cAMP activator to obtain a cell aggregate, wherein

the culture solution in step (ii) and/or the culture solution in step (iv) contain or contains a Notch signaling inhibitor.

27. The method according to claim 26, wherein, in step (i), a cerebral organoid is obtained from the pluripotent stem cell by the method according to any one of claims 1 to 10.

28. The method according to any one of claims 19, 20, 26, and 27, wherein the cerebral organoid to be subjected to step (ii) is a cerebral organoid 28 to 44 days after initiation of induction of differentiation into a neural cell.

29. The method according to any one of claims 19, 20, and 26 to 28, wherein culture period in step (ii) is 2 to 6 days.

30. The method according to any one of claims 19, 20, and 26 to 29, wherein culture period in step (iv) is 2 to 14 days.

31. The method according to any one of claims 19, 20, and 26 to 30, wherein the Notch signaling inhibitor is a γ-secretase inhibitor.

32. The method according to claim 31, wherein the γ-secretase inhibitor is N-[N-(3,5-difluorophenacetyl)-L-alanyl]-S-phenylglycine t-butyl ester (DAPT) or Compound E.

33. A cell population comprising the high-purity cerebral cortical cell aggregate according to any one of claims 21 to 25, wherein size, shape, or constituent cell composition of the high-purity cerebral cortical cell aggregate is homogeneous.

34. A pharmaceutical composition comprising the cerebral cortical cell aggregate according to any one of claims 14 to 18, the high-purity cerebral cortical cell aggregate according to any one of claims 21 to 25, or the cell population according to claim 33, or a cell population obtained by dispersing any of them into a constituent cell, as an active ingredient.

35. A tissue for transplantation, the tissue comprising the cerebral cortical cell aggregate according to any one of claims 14 to 18, the high-purity cerebral cortical cell aggregate according to any one of claims 21 to 25, or the cell population according to claim 33, or a cell population obtained by dispersing any of them into a constituent cell.

36. A therapeutic drug for cerebrovascular disorder, the therapeutic drug comprising the cerebral cortical cell aggregate according to any one of claims 14 to 18, the high-purity cerebral cortical cell aggregate according to any one of claims 21 to 25, or the cell population according to claim 33, or a cell population obtained by dispersing any of them into a constituent cell, as an active ingredient.

37. A therapeutic method for cerebrovascular disorder, comprising administering or transplanting the cerebral cortical cell aggregate according to any one of claims 14 to 18, the high-purity cerebral cortical cell aggregate according to any one of claims 21 to 25, or the cell population according to claim 33, or a cell population obtained by dispersing any of them into a constituent cell to a cerebral cortex or basal ganglion of a subject in need thereof.

38. A quality assessment method for a cerebral organoid or a cerebral cortical cell aggregate, comprising:

(aa) a step of measuring an expression level of at least one gene selected from the group consisting of GAD2, COL1A1, TYR, TTR, and HOXA2, or a protein encoded by the gene or a fragment thereof in a cerebral organoid or a cerebral cortical cell aggregate; and
(bb) a step of determining with reference to a measurement result in step (aa) that an amount of non-target cells included in the cerebral organoid or the cerebral cortical cell aggregate is equal to or less than a reference value if the expression level of the gene is equal to or less than a reference value.

39. A quality assessment method for a cerebral organoid or a cerebral cortical cell aggregate, comprising:

(AA) a step of measuring an expression level of at least one gene selected from the group consisting of NEUROD6, NEUROD2, SSTR2, TBR1, ZBTB18, NHLH1, IGFBPL1, NRN1, RTN1, THSD7A, NRXN1, BHLHE22, CALB2, KHDRBS3, CCSAP, PDE1A, NEUROD1, NPTX1, NXPH4, NTS, NEUROG2, OLFM1, PRDM8, CORO2B, TP53I11, ZFPM2, PCDH9, NELL2, SRRM4, SCG3, DCC, EPB41L3, SLC17A7, ST18, NSG2, EMX1, CAP2, SYT4, NSMF, ANK3, MYT1L, FSTL5, CELF4, B3GAT1, EPHA5, NHLH2, and DLL3 in a cerebral organoid or a cerebral cortical cell aggregate; and
(BB) a step of determining with reference to a measurement result in step (AA) that an amount of target cells included in the cerebral organoid or the cerebral cortical cell aggregate is equal to or more than a reference value if the expression level of the gene is equal to or more than a reference value.

## *Fig.1*

(A)
201B7

(B)
Ff-I01s04

## Fig.2

(A)

●TGFβ pathway-related gene

(B)

●TGFβ pathway-related gene

EP 4 357 449 A1

## Fig.3

Passage · Induction of differentiation · Immunostaining

| Step (1) | Step (2) |

d-7  -5  -1  d0  d18  d35

| iPSC maintenance culture | SFEBq: 96 well plate | 90mm dish |
| StemFit, LM511-E8 | 20% KSR, suspension culture | N2, Shaker |
| Y | w/o C +SB | SB + IWR1e |

Y

Abbreviations in figure
w/o C: StemFit culture medium without solution C
Y : Y-27632
SB: SB431542
N2: N2 supplement

EP 4 357 449 A1

## Fig.4

Efficiency of cerebral organoid formation

Scale bar = 100μm

## Fig.5

Abbreviations in figure
w/o C: StemFit culture medium without solution C
Y: Y-27632
SB: SB431542
N2: N2 supplement

EP 4 357 449 A1

## Fig.6

# Fig.7

EP 4 357 449 A1

Fig.8

## *Fig.9*

pre 1d-5d

*Fig.10*

● Control(StemFit)
○ StemFit-bFGF, +TGFβi

qPCR, ddCt
n=3 independent experiments

**Fig.11**

(A)

Dispersion & reaggregation

| Step (1) | | Step (ii)-(iv) |

d-1    d0         d18    d33    d36    d40

| iPSC maintenance culture | Induction of differentiation (SFEBq) |
| 6-well plate | 96-well (v) | 90-mm, orbital shaker (50 rpm) |
| StemFit, LM511-E8 | GMEM/20%KSR | DMEM/F12 + N2 |
| -bFGF, +SB | SB + IWR1e | DAPT | G/A/B/A |

Y

Y

G/A/B/A: collective term for four compounds of GDNF, ascorbic acid, BDNF, and dibutyryl-cAMP

(B)

Day36 | Day40

DAPT- | DAPT+ | Spherical cell aggregate

1mm | 1mm

Scale bar =500μm

EP 4 357 449 A1

# Fig.12

(A)

Day36

| | Ctip2 | Foxg1 | Pax6 | Ki67 | DAPI |

DAPT-

DAPT+

(B)

Day40

| | Ctip2 | Foxg1 | Pax6 | DAPI |

Reaggregated
spherical cell aggregate
after DAPT treatment

## Fig.13

| Step (1) | | Step (ii) |

| | d-1 | d0 | | d18 | d40 | d43 |

| iPSC maintenance culture | Induction of differentiation (SFEBq) |
| 6-well plate | 96-well (v) | 90-mm (orbital shaker) |
| StemFit, LM511-E8 | GMEM/20%KSR | DMEM/F12 + N2 |
| -bFGF, +SB | SB + IWR1e | DAPT |

Y

EP 4 357 449 A1

Fig.14

Fig.15

# Fig.16

**(A)**

**(B)**

DAPT treatment (Day)

## Fig.17

(A)

(B)

Scale bar: 4wk 100µm, 6wk & 10wk 200µm

# Fig.18

# Fig.19

Staining antibody: Ku80

EP 4 357 449 A1

## Fig.20

# Fig.21

Fig.22

Fig.23

# Fig.24

**(A) Organoid DAPT method** (for performing DAPT treatment in step (ii))

| | Step (i) | | Step (ii)-(iv) |

Passage

| | Step (1) | Step (2) | Dispersion Sampling | Sampling |

d-7    d-1    d0    d6    d18    d35    d38    d42    d48

| iPSC maintenance culture | Induction of differentiation (SFEBq) |

| 6-well plate/LM511-E8 | 96-well (v) | 90-mm, orbital shaker (50 rpm) | 96-wp (v) |

| StemFit | E6 | DMEM/F12/20%KSR | DMEM/F12 + N2 |
| | or | | |
| | w/o C +SB | SB + IWR1e | DAPT | G/A/B/A |
| | | Y | | Y |

**(B) Single-cell DAPT method** (for performing DAPT treatment in step (iv))

| | Step (i) | | Step (ii)-(iv) |

Passage

| | Step (1) | Step (2) | Dispersion Sampling | Sampling |

d-7    d-1    d0    d6    d18    d35    d38    d41 d42    d48

| iPSC maintenance culture | Induction of differentiation (SFEBq) |

| 6-well plate/LM511-E8 | 96-well (v) | 90-mm, orbital shaker (50 rpm) | 96-wp (v) |

| StemFit | E6 | DMEM/F12/20%KSR | DMEM/F12 + N2 |
| | or | | |
| | w/o C +SB | SB + IWR1e | DAPT |
| | | Y | G/A/B/A |
| | | | Y |

Fig.25

Fig.26

## Fig.27

Step (iv) on Day 10

Single-cell DAPT method | Organoid DAPT method

0μM | 0.1μM | 1μM | 10μM | 10μM

## Fig.28

EP 4 357 449 A1

# Fig.29

Day 1 after removal of DAPT

Tuj1   Ctip2   Pax6   Ki67   Sox1

Day 1 after removal of DAPT

Total cell — Tuj1/Ctip2 (84.5), Pax6/Ki67 (3.1); Sox1+ — Pax6/Ki67 (0.7)

Day 11 after removal of DAPT

Tuj1   Ctip2   Pax6   Ki67   Sox1

Day 11 after removal of DAPT

Total cell — Tuj1/Ctip2 (89.2), Pax6/Ki67 (0.1); Sox1+ — Pax6/Ki67 (0)

Fig.30

Fig.31

Fig.32

Fig.33

*Figure showing organoid classifications: Rosettes (Organoid1, Organoid2), Potato-like +Transparent (Organoid3, Organoid4), Balloon +Cotton-like / +Rosettes (Organoid5, Organoid6), Transparent +Rosettes / +Jelly-like (Organoid7, Organoid8), Pigment +Transparent +Jelly-like (Organoid9). Legend: Rosettes, Potato-like, Cotton-like, Balloon, Transparent, Jelly-like, Pigment.*

EP 4 357 449 A1

EP 4 357 449 A1

## Fig.34

Organoid1
Organoid2
Organoid3
Organoid4
Organoid5
Organoid6
Organoid7
Organoid8
Organoid9

# Fig.35

Fig.36

# Fig.37

Fig.38

# Fig.39

(A)

(B)

# Fig.40

Fig.41

# Fig.42

EP 4 357 449 A1

Fig.43

# Fig.44

(A)

Lot1

Lot2

Lot3

(B)

| | DAPT-<br>(cerebral organoid) | DAPT+<br>(cerebral cortical cell aggregate) |
|---|---|---|
| Lot1 | | |
| Lot2 | | |
| Lot3 | | No42_d44_DAPT |

EP 4 357 449 A1

*Fig.45*

FOXG1 (Bf1)  PAX6  MKI67  BCL11B(Ctip2)  SATB2

EMX1  HES1  SLC17A1 (VGluT1)  NEUROD6

TTR  COL1A1  TYR  PECAM1  GAD2

DAPT-  DAPT+

## INTERNATIONAL SEARCH REPORT

| International application No. |
| --- |
| **PCT/JP2022/024226** |

**A. CLASSIFICATION OF SUBJECT MATTER**

*C12N 5/0735*(2010.01)i; *C12N 5/074*(2010.01)i
FI: C12N5/074; C12N5/0735

According to International Patent Classification (IPC) or to both national classification and IPC

**B. FIELDS SEARCHED**

Minimum documentation searched (classification system followed by classification symbols)

C12N5/0735; C12N5/074

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | US 2020/0208090 A1 (THE JOHNS HOPKINS UNIVERSITY) 02 July 2020 (2020-07-02) paragraph [0146] | 1, 3-4, 7-18, 21-25, 33-37, 39 |
| Y | paragraph [0146] | 2-13, 38 |
| A | paragraph [0146] | 19-20, 26-32 |
| X | ALBANESE, Alexandre et al., Multiscale 3D phenotyping of human cerebral organoids, Scientific Reports, 2020, vol. 10, 21487 (pp.1-17), https://doi.org/10.1038/s41598-020-78130-7 In particular, "Material and methods", "Cerebral organoid culture" | 1, 3-18, 21-25, 33-37, 39 |
| Y | In particular, "Material and methods", "Cerebral organoid culture" | 2-13, 38 |
| A | In particular, "Material and methods", "Cerebral organoid culture" | 19-20, 26-32 |

☑ Further documents are listed in the continuation of Box C.    ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **19 August 2022** | **30 August 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

International application No.

**PCT/JP2022/024226**

### C.    DOCUMENTS CONSIDERED TO BE RELEVANT

| Category* | Citation of document, with indication, where appropriate, of the relevant passages | Relevant to claim No. |
|---|---|---|
| X | SONG, Liqing et al., Assembly of Human Stem Cell-Derived Cortical Spheroids and Vascular Spheroids to Model 3-D Brain-like Tissues, Scientific Reports, 12 April 2019, vol. 9, 5977 (pp.1-16), https://doi.org/10.1038/s41598-019-42439-9 <br> In particular, "Methods", "Generation of cortical iNPC spheroids.", fig. 1 | 1, 3-18, 21-25, 33-37, 39 |
| Y | In particular, "Methods", "Generation of cortical iNPC spheroids.", fig. 1 | 2-13, 38 |
| A | In particular, "Methods", "Generation of cortical iNPC spheroids.", fig. 1 | 19-20, 26-32 |
| X | SAKAGUCHI, Hideya et al., Self-Organized Synchronous Calcium Transients in a Cultured Human Neural Network Derived from Cerebral Organoids, Stem Cell Reports, 10 September 2019, vol. 13, pp.458-473, https://doi.org/10.1016/j.stemcr.2019.05.029 <br> In particular, "EXPERIMENTAL PROCEDURES", "Differentiation Culture of hPSCs", fig. 3 | 1-6, 8-18, 21-37, 39 |
| Y | In particular, "EXPERIMENTAL PROCEDURES", "Differentiation Culture of hPSCs", fig. 3 | 2-13, 38 |
| A | In particular, "EXPERIMENTAL PROCEDURES", "Differentiation Culture of hPSCs", fig. 3 | 19-20 |
| X | OYAMA, Hiroshi et al., Long-term Culture of Human iPS Cell-derived Telencephalic Neuron Aggregates on Collagen Gel, https://doi.org/10.1016/j.stemcr.2019.05.029, 2018, vol. 43, pp. 85-94 <br> In particular, "Materials and Methods", "HUman iPs cell culture", fig. 1 | 1-18, 21-25, 33-37, 39 |
| Y | In particular, "Materials and Methods", "HUman iPs cell culture", fig. 1 | 2-13, 38 |
| A | In particular, "Materials and Methods", "HUman iPs cell culture", fig. 1 | 19-20, 26-32 |
| X | HASAN, Md Fayad and BERDICHEVSKY, Yevgeny , Neuron and astrocyte aggregation and sorting in three-dimensional neuronal constructs, Commun. Biol., 17 May 2021, vol. 4, no. 1, 587 (pp.1-16), DOI:10.1038/s42003-021-02104-2 <br> In particular, summary, fig. 3 | 14-18, 21-25, 33-37, 39 |
| Y | In particular, summary, fig. 3 | 38 |
| A | In particular, summary, fig. 3 | 1-13, 19-20, 26-32 |
| X | IZSAK, Julia et al., TGF-β1 Suppresses Proliferation and Induces Differentiation in Human iPSC Neural in vitro Models, Frontiers in Cell and Developmental Biology, 28 October 2020, vol. 8, article 571332 (pp.1-15), doi: 10.3389/fcell.2020.571332 <br> In particular, abstract, fig. 4 | 14-25, 28-37, 39 |
| Y | In particular, abstract, fig. 4 | 38 |
| A | In particular, abstract, fig. 4 | 1-13, 26-27, |
| X | BEJOY, Julie et al., Wnt-Notch Signaling Interactions During Neural and Astroglial Patterning of Human Stem Cells, TISSUE ENGINEERING: part A, 2020, vol. 26, no. 7 and 8, pp.419-431, DOI: 10.1089/ten.tea.2019.0202 <br> In particular, abstract, p. 424, right column, first paragraph, fig. 4, supplementary fig. S5 | 14-25, 28-37, 39 |
| Y | In particular, abstract, p. 424, right column, first paragraph, fig. 4, supplementary fig. S5 | 38 |
| A | In particular, abstract, p. 424, right column, first paragraph, fig. 4, supplementary fig. S5 | 1-13, 26-27 |
| Y | LOPEZ-BENDITO, Guillermina et al., Preferential Origin and Layer Destination of GAD65-GFP Cortical Interneurons, Cerebral Cortex, 2004, vol. 14, no. 10, pp. 1122-1133, doi:10.1093/cercor/bhh072 <br> particularly, fig. 2 | 38 |
| A |  | 1-37, 39 |

<div align="center">

**INTERNATIONAL SEARCH REPORT**

</div>

| International application No. |
|---|
| **PCT/JP2022/024226** |

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

  a.  ☑ forming part of the international application as filed:

  ☑ in the form of an Annex C/ST.25 text file.

  ☐ on paper or in the form of an image file.

  b.  ☐ furnished together with the international application under PCT Rule 13*ter*.1(a) for the purposes of international search only in the form of an Annex C/ST.25 text file.

  c.  ☐ furnished subsequent to the international filing date for the purposes of international search only:

  ☐ in the form of an Annex C/ST.25 text file (Rule 13*ter*.1(a)).

  ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/JP2022/024226**

| Box No. III    Observations where unity of invention is lacking (Continuation of item 3 of first sheet) |
|---|

This International Searching Authority found multiple inventions in this international application, as follows:

The claims are classified into the following 46 inventions.

<Invention 1>

The inventions in claims 1-13 relate to a method for producing cerebral organoids from pluripotent stem cells, and specify a culture condition where bFGF is not included and TGFβ signaling is not induced.

<Invention 2>

The inventions in claims 14-18 and 21-25 relate to a cerebral cortex cell mass, but share the feature of a mass containing nerve cells with the inventions in claims 1-13 classified as invention 1. However, based on the common technical knowledge, this feature cannot be said to be a special technical feature that makes a contribution over the prior art, and there is no other identical or corresponding special technical feature. Furthermore, claims 14-18 and 21-25 are not dependent claims citing claims 1-13. Accordingly, claims 14-18 and 21-25 cannot be classified as invention 1, and are classified as invention 2.

<Invention 3>

The inventions in claims 19-20 relate to a method for obtaining a cerebral cortex cell mass by obtaining cerebral organoids from pluripotent stem cells, and culturing this in a culture solution containing a Notch signaling inhibitor. However, the inventions do not specify a culture condition where bFGF is not included and TGFβ signaling is not induced. Furthermore, in view of common technical knowledge, it is clear that the inventions in claims 19-20 do not share special technical features with invention 1 or invention 2. Thus, claims 19-20 cannot be classified as invention 1 or 2, and are classified as invention 3.

<Invention 4>

The inventions in claims 26-27 relate to a method for obtaining cerebral organoids from pluripotent stem cells, after which the cerebral organoids are dispersed in cell aggregates, and then a cell mass is caused to form. However, the inventions do not specify a culture condition, etc., where bFGF is not included and TGFβ signaling is not induced. Furthermore, in view of common technical knowledge, it is clear that the invention does not share special technical feature with inventions 1-3. Thus, claims 26-27 cannot be classified as inventions 1-3, and are classified as invention 4.

<Inventions 5-9>

The invention as in claim 38 relates to a quality evaluation method for cerebral organoids or a cerebral cortex cell mass, and specifies that determination is made on the basis of the expression amount of five genes. However, the invention does not specify, among other things, a culture condition where bFGF is not included and TGFβ is not induced. Furthermore, in view of common technical knowledge, it is clear that the invention in claim 38 does not share special technical features with inventions 1-4. Thus, claim 38 cannot be classified as inventions 1-4. Additionally, evaluating tissue by the gene expression amount belongs to common technical knowledge, and thus it is also clear that claim 38 is classified into five inventions for each of the five genes.

<Inventions 10-46>

The invention in claim 39 relates to 47 genes, and for the same reason as <Inventions 5-9> above, it is classified into 47 inventions for each of the 47 genes.

The inventions in claims 28-37 cite any of the claims classified as inventions 2-4, and thus the cited portions are classified as respective inventions 2-4.

## INTERNATIONAL SEARCH REPORT

| International application No. |
|---|
| **PCT/JP2022/024226** |

**Box No. III    Observations where unity of invention is lacking (Continuation of item 3 of first sheet)**

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/024226**

| Patent document cited in search report | Publication date (day/month/year) | Patent family member(s) | Publication date (day/month/year) |
|---|---|---|---|
| US 2020/0208090 A1 | 02 July 2020 | WO 2017/083705 A1 paragraph [0168] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015076388 A
- WO 2016167372 A
- WO 2016063985 A
- WO 9622362 A
- WO 02101057 A
- US 5843780 A

- US 6200806 B
- US 6280718 B
- WO 9830679 A
- WO 2009146408 A
- WO 2008035110 A

**Non-patent literature cited in the description**

- **KITAHARA et al.** *Stem Cell Reports,* 2020, vol. 15, 467-481
- **KUWAHARA et al.** *Scientific Reports,* 2019, vol. 9, 18936
- **EIRAKU, M et al.** *Cell Stem Cell,* 2008, vol. 3, 519-532
- **YAMANAKA et al.** *Cell,* 2006, vol. 126 (4), 663-676
- *Cell,* 2007, vol. 131 (5), 861-872
- *Science,* 2007, vol. 318 (5858), 1917-1920
- *Nat. Biotechnol.,* 2008, vol. 26 (1), 101-106
- *Stem Cells,* 2013, vol. 31, 458-466
- *Science,* 2013, vol. 341, 651-654
- *Nature,* 2022, vol. 605, 325-331
- **R. K. LINDEMANN et al.** *Mol. Cancer,* 2003, vol. 2, 20
- **MINAMI I. et al.** *Cell Rep.,* 2012, vol. 2, 1448-1460
- *Proc Natl Acad Sci USA.,* 09 September 2008, vol. 105 (36), 13409-14
- **SAKAGUCHI et al.** *Stem Cell Reports,* 2019, vol. 13, 458-473

- **KADOSHIMA et al.** *PNAS,* 2013, vol. 110 (50), 20284-20289
- **WATANABE, K. et al.** *Nature Biotechnology,* 2007, vol. 25 (6), 681-686
- *Nature Biotechnology,* 2019, vol. 37, 38-44
- **ROBERT F. HEVNER.** Tbr1 Regulates Differentiation of the Preplate and Layer. *Neuron,* 2001, vol. 6
- **TUTUKOVA S et al.** The Role of Neurod Genes in Brain Development, Function, and Disease. *Frontiers in Molecular Neuroscience,* 2021
- **SIMOES-COSTA M et al.** Establishing neural crest identity: a gene regulatory recipe. *Development,* 2015
- **FAZILATY H et al.** *A gene regulatory network to control EMT programs in development and disease,* 2019
- **GONCHALOV et al.** Markers and Biomarkers of Endothelium: When Something Is Rotten in the State. *Oxidative Medicine and Cellular Longevity,* 2017